# EUROPEAN PATENT APPLICATION

(11) **EP 4 276 094 A1**
(43) Date of publication of application: **15.11.2023**
(21) Application number: 23172294.3
(22) Date of filing: 09.05.2023
(51) Int. Cl.: C07D 209/86, C07D 405/04, H10K 85/00

(54) **LIGHT EMITTING ELEMENT AND AMINE COMPOUND FOR THE SAME**

(30) Priority: 11.05.2022 KR 20220057855; 09.01.2023 KR 20230002931
(71) Applicant: Samsung Display Co., Ltd., Yongin-si, Gyeonggi-do 17113 (KR)
(72) Inventor: Sakamoto, Naoya, Tsurumi-ku, Yokohama (JP)
(74) Representative: Gulde & Partner

(57) **Abstract**

A light emitting element and an amine compound for a light emitting element are provided. The light emitting element includes a first electrode, a second electrode disposed on the first electrode, and at least one functional layer disposed between the first electrode and the second electrode and including an amine compound represented by Formula 1.

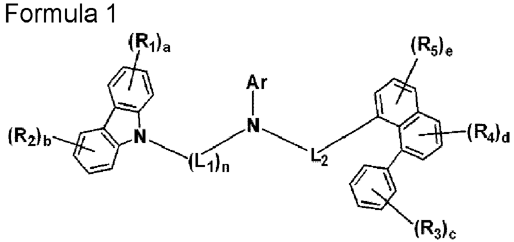

## Description

### BACKGROUND

### 1. Field

One or more aspects of embodiments of the present disclosure relate to a light emitting element and an amine compound for a light emitting element, and particularly, to a light emitting element including an amine compound in a functional layer of the light emitting element.

### 2. Description of the Related Art

Recently, the utilization of an organic electroluminescence display device as an image display device is being actively conducted. The organic electroluminescence display device is a type of kind of a display device that is self-luminescent in its light emitting element in which holes and electrons injected from a first electrode and a second electrode of the light emitting element recombine in an emission layer of the light emitting element so that a light emitting material in the emission layer emits light to achieve display.

In order to increase an emission efficiency of the light emitting element and improve a lifetime (lifespan) of the light emitting element, materials suitable for a hole transport region of the light emitting element and having excellent or suitable hole transport properties and stability are being intensively researched.

### SUMMARY

The invention is defined by the appended claims. More specifically, according to one aspect of the present invention there is provided an amine compound represented by Formula 1 is defined by the appended claims. According to another aspect of the present invention there is provided a light emitting element as defined in claim 13 and including the amine compound represented by Formula 1.

The light emitting element shows high emission efficiency and long-life characteristics.

The amine compound is a material for a light emitting element for improving emission efficiency and element lifetime.

Additional aspects will be set forth in part in the description which follows and, in part, will be apparent from the description, or may be learned by practice of the presented embodiments.

### BRIEF DESCRIPTION OF THE DRAWINGS

The accompanying drawings are included to provide a further understanding of the present disclosure and are incorporated in and constitute a part of this specification. The drawings illustrate embodiments of the present disclosure and, together with the description, serve to explain principles of the present disclosure. Above and/or other aspects of the present disclosure will become apparent and appreciated from the following description of embodiments taken in conjunction with the accompanying drawings. In the drawings:
FIG. 1 is a plan view showing a display device according to one or more embodiments of the present disclosure;
FIG. 2 is a cross-sectional view of a display device according to one or more embodiments of the present disclosure;
FIG. 3 is a cross-sectional view schematically showing a light emitting element according to one or more embodiments of the present disclosure;
FIG. 4 is a cross-sectional view schematically showing a light emitting element according to one or more embodiments of the present disclosure;
FIG. 5 is a cross-sectional view schematically showing a light emitting element according to one or more embodiments of the present disclosure;
FIG. 6 is a cross-sectional view schematically showing a light emitting element according to one or more embodiments of the present disclosure;
FIG. 7 is a cross-sectional view of a display device according to one or more embodiments of the present disclosure;
FIG. 8 is a cross-sectional view of a display device according to one or more embodiments of the present disclosure;
FIG. 9 is a cross-sectional view showing a display device according to one or more embodiments of the present disclosure; and
FIG. 10 is a cross-sectional view showing a display device according to one or more embodiments of the present disclosure.

### DETAILED DESCRIPTION

The present disclosure may have one or more suitable modifications and may be embodied in different forms, and example embodiments will be explained in more detail with reference to the accompany drawings. The present disclosure may, however, be embodied in different forms and should not be construed as limited to the embodiments set forth herein.

Like reference numerals refer to like elements throughout the present disclosure, and duplicative descriptions thereof may not be provided for conciseness. In the drawings, the dimensions of structures may be exaggerated for clarity of illustration. It will be understood that, although the terms first, second, etc. may be utilized herein to describe one or more suitable elements, these elements should not be limited by these terms. These terms are only utilized to distinguish one element from another element. Thus, a first element could be termed a second element without departing from the teachings of the present disclosure. Similarly, a second element could be termed a first element. As utilized herein, the singular forms are intended to include the plural forms as well, unless the context clearly indicates otherwise.

In the present disclosure, it will be further understood that the terms "comprise(s)/include(s)" and/or "comprising/including," when utilized in this specification, specify the presence of stated features, numerals, steps, operations, elements, parts, or the combination thereof, but do not preclude the presence or addition of one or more other features, numerals, steps, operations, elements, parts, or the combination thereof. As used herein, the terms "and", "or", and "and/or" include any and all combinations of one or more of the associated listed items. Expressions such as "at least one of," "one of," and "selected from," when preceding a list of elements, modify the entire list of elements and do not modify the individual elements of the list. For example, "at least one of a, b or c", "at least one selected from a, b, and c", "at least one selected from among a to c", etc., may indicate only a, only b, only c, both (e.g., simultaneously) a and b, both (e.g., simultaneously) a and c, both (e.g., simultaneously) b and c, all of a, b, and c, or variations thereof. Further, the use of "may" when describing embodiments of the present disclosure refers to "one or more embodiments of the present disclosure."

In the present disclosure, when a layer, a film, a region, a plate, etc. is referred to as being "on" or "above" another part, it can be "directly on" the other part, or intervening layers may also be present. In contrast, when a layer, a film, a region, a plate, etc. is referred to as being "under" or "below" another part, it can be "directly under" the other part, or intervening layers may also be present. Also, when an element is referred to as being disposed "on" another element, it can be disposed under the other element.

In the present disclosure, the term "substituted or unsubstituted" corresponds to substituted or unsubstituted with at least one substituent selected from the group consisting of a deuterium atom, a halogen atom, a cyano group, a nitro group, an amino group (an amine group), a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, a hydrocarbon ring group, an aryl group, and a heterocyclic group. In some embodiments, each of the exemplified substituents may be substituted or unsubstituted. For example, a biphenyl group may be interpreted as an aryl group, or a phenyl group substituted with a phenyl group.

In the present disclosure, the terms such as "forming a ring via the combination with an adjacent group" and "combined with an adjacent group to form a ring" may refer to forming a substituted or unsubstituted hydrocarbon ring, or a substituted or unsubstituted heterocycle via the combination with an adjacent group. The hydrocarbon ring may include an aliphatic hydrocarbon ring and an aromatic hydrocarbon ring. The heterocycle may include an aliphatic heterocycle and an aromatic heterocycle. The hydrocarbon ring and the heterocycle may be monocycles or polycycles. In some embodiments, the ring formed via the combination with an adjacent group may be combined with another ring to form a spiro structure.

In the present disclosure, the term "adjacent group" may refer to a substituent substituted for an atom which is directly combined with an atom substituted with a corresponding substituent, another substituent substituted for an atom which is substituted with a corresponding substituent, or a substituent sterically positioned at the nearest position to a corresponding substituent. For example, in 1,2-dimethylbenzene, two methyl groups may be interpreted as "adjacent groups" to each other, and in 1,1-diethylcyclopentene, two ethyl groups may be interpreted as "adjacent groups" to each other. For another example, in 4,5-dimethylphenanthrene, two methyl groups may be interpreted as "adjacent groups" to each other.

In the present disclosure, a halogen atom may be a fluorine atom, a chlorine atom, a bromine atom, or an iodine atom.

In the present disclosure, an alkyl group may be a linear, branched, or cyclic type or kind. The carbon number of the alkyl group may be 1 to 50, 1 to 30, 1 to 20, 1 to 10, or 1 to 6. Examples of the alkyl group may include methyl, ethyl, n-propyl, isopropyl, n-butyl, s-butyl, t-butyl, i-butyl, 2-ethylbutyl, 3,3-dimethylbutyl, n-pentyl, i-pentyl, neopentyl, t-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-t-butylcyclohexyl, n-heptyl, 1-methylheptyl, 2,2-dimethylheptyl, 2-ethylheptyl, 2-butylheptyl, n-octyl, t-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldocecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-hexyleicosyl, 2-octyleicosyl, n-henicosyl, n-docosyl, n-tricosyl, n-tetracosyl, n-pentacosyl, n-hexacosyl, n-heptacosyl, n-octacosyl, n-nonacosyl, n-triacontyl, etc., without limitation.

In the present disclosure, an alkenyl group may refer to a hydrocarbon group including one or more carbon-carbon double bonds in the middle or at the terminal of an alkyl group having a carbon number of 2 or more. The alkenyl group may be a linear chain or a branched chain. The carbon number of the alkenyl group is not specifically limited, but may be 2 to 30, 2 to 20, or 2 to 10. Examples of the alkenyl group may include a vinyl group, an allyl group, a 1-butenyl group, a 1-pentenyl group, a 1 ,3-butadienyl group, a styryl group, a styrylvinyl group, etc., without limitation.

In the present disclosure, an alkynyl group may refer to a hydrocarbon group including one or more carbon-carbon triple bonds in the middle or at the terminal of an alkyl group having a carbon number of 2 or more. The alkynyl group may be a linear chain or a branched chain. The carbon number of the alkynyl group is not specifically limited, but may be 2 to 30, 2 to 20, or 2 to 10. Examples of the alkynyl group may include an ethynyl group, a propionyl group, etc., without limitation.

In the present disclosure, a hydrocarbon ring group may refer to an optional functional group or substituent derived from an aliphatic hydrocarbon ring. The hydrocarbon ring group may be a saturated hydrocarbon ring group of 5 to 20 ring-forming carbon atoms.

In the present disclosure, an aryl group may refer to an optional functional group or substituent derived from an aromatic hydrocarbon ring. The aryl group may be a monocyclic aryl group or a polycyclic aryl group. The carbon number for forming rings in the aryl group may be 6 to 50, 6 to 30, 6 to 20, or 6 to 15. Examples of the aryl group may include phenyl, naphthyl, fluorenyl, anthracenyl, phenanthryl, biphenyl, terphenyl, quaterphenyl, quinquephenyl, sexiphenyl, triphenylenyl, pyrenyl, benzofluoranthenyl, chrysenyl, etc., without limitation.

In the present disclosure, a fluorenyl group may be substituted, and two substituents may be combined with each other to form a spiro structure. Examples of a substituted fluorenyl group are as follows, but embodiments of the present disclosure are not limited thereto.

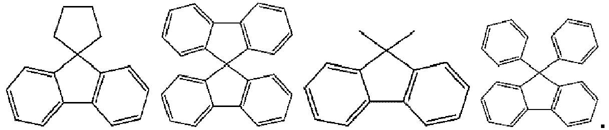

In the present disclosure, a heterocyclic group may refer to an optional functional group or substituent derived from a ring including one or more selected from among B, O, N, P, Si, and S as heteroatoms. The heterocyclic group may include an aliphatic heterocyclic group and an aromatic heterocyclic group. The aromatic heterocyclic group may be a heteroaryl group. The aliphatic heterocyclic group and the aromatic heterocyclic group may be a monocycle or a polycycle.

In the present disclosure, a heterocyclic group may include one or more selected from among B, O, N, P, Si, and S as heteroatoms. When the heterocyclic group includes two or more heteroatoms, the two or more heteroatoms may be the same or different. In the present disclosure, the heterocyclic group may be a monocyclic heterocyclic group or polycyclic heterocyclic group and has concept including a heteroaryl group. The carbon number for forming rings of the heterocyclic group may be 2 to 50, 2 to 30, 2 to 20, or 2 to 10.

In the present disclosure, an aliphatic heterocyclic group may include one or more selected from among B, O, N, P, Si, and S as heteroatoms. The number of ring-forming carbon atoms of the aliphatic heterocyclic group may be 2 to 30, 2 to 20, or 2 to 10. Examples of the aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, etc., without limitation.

In the present disclosure, a heteroaryl group may include one or more selected from among B, O, N, P, Si, and S as heteroatoms. When the heteroaryl group includes two or more heteroatoms, the two or more heteroatoms may be the same or different. The heteroaryl group may be a monocyclic heterocyclic group or polycyclic heterocyclic group. The carbon number for forming rings of the heteroaryl group may be 2 to 50, 2 to 30, 2 to 20, or 2 to 10. Examples of the heteroaryl group may include thiophene, furan, pyrrole, imidazole, triazole, tetrazole, pyridine, bipyridine, pyrimidine, triazine, acridyl, pyridazine, pyrazinyl, quinoline, quinazoline, quinoxaline, phenoxazine, phthalazine, pyrido pyrimidine, pyrido pyrazine, pyrazino pyrazine, isoquinoline, indole, carbazole, N-arylcarbazole, N-heteroarylcarbazole, N-alkylcarbazole, benzoxazole, benzimidazole, benzothiazole, benzocarbazole, benzothiophene, dibenzothiophene, thienothiophene, benzofuran, phenanthroline, thiazole, isooxazole, oxazole, oxadiazole, thiadiazole, phenothiazine, dibenzosilole, dibenzofuran, etc., without limitation.

In the present disclosure, the same explanation on the above-described aryl group may be applied to an arylene group except that the arylene group is a divalent group. The same explanation on the above-described heteroaryl group may be applied to a heteroarylene group except that the heteroarylene group is a divalent group.

In the present disclosure, a boron group may refer to the above-defined alkyl group or aryl group combined with a boron atom. The boron group may include an alkyl boron group and an aryl boron group. Examples of the boron group may include a dimethylboron group, a diethylboron group, a t-butylboron group, a diphenylboron group, a phenylboron group, and/or the like, without limitation.

In the present disclosure, a silyl group may include an alkyl silyl group and an aryl silyl group. Examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc., without limitation.

In the present disclosure, the carbon number of a carbonyl group is not specifically limited, but the carbon number may be 1 to 40, 1 to 30, or 1 to 20. For example, the carbonyl group may have the follow structures, but is not limited thereto.

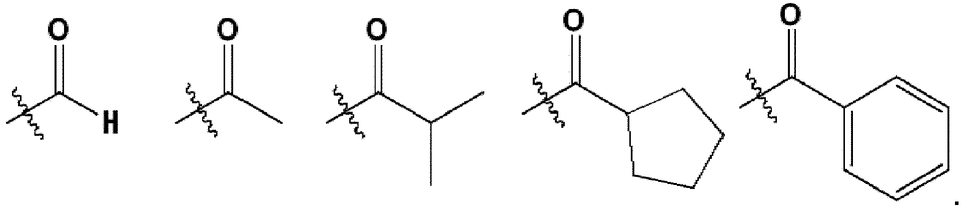

In the present disclosure, the carbon number of a sulfinyl group and sulfonyl group is not specifically limited, but may be 1 to 30. The sulfinyl group may include an alkyl sulfinyl group and an aryl sulfinyl group. The sulfonyl group may include an alkyl sulfonyl group and an aryl sulfonyl group.

In the present disclosure, a thio group may include an alkyl thio group and an aryl thio group. The thio group may refer to the above-defined alkyl group or aryl group combined with a sulfur atom. Examples of the thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, etc., without limitation.

In the present disclosure, an oxy group may refer to the above-defined alkyl group or aryl group which is combined with an oxygen atom. The oxy group may include an alkoxy group and an aryl oxy group. The alkoxy group may be a linear, branched, or cyclic chain. The carbon number of the alkoxy group is not specifically limited but may be, for example, 1 to 20 or 1 to 10. Examples of the oxy group may include methoxy, ethoxy, n-propoxy, isopropoxy, butoxy, pentyloxy, hexyloxy, octyloxy, nonyloxy, decyloxy, benzyloxy, etc. However, embodiments of the present disclosure are not limited thereto.

In the present disclosure, the carbon number of an amine group is not specifically limited, but may be 1 to 30. The amine group may include an alkyl amine group and an aryl amine group. Examples of the amine group may include a methylamine group, a dimethylamine group, a phenylamine group, a diphenylamine group, a naphthylamine group, a 9-methyl-anthracenylamine group, etc., without limitation.

In the present disclosure, alkyl groups in an alkyloxy group, alkylthio group, alkylsulfinyl group, alkylsulfoxy group, alkylaryl group, alkylamino group, alkylboron group, alkyl silyl group, and alkyl amine group may be the same as the examples of the above-described alkyl group.

In the present disclosure, aryl groups in an aryloxy group, arylthio group, arylsulfinyl group, arylsulfoxy group, aryl amino group, arylboron group, aryl silyl group, and aryl amine group may be the same as the examples of the above-described aryl group.

In the present disclosure, a direct linkage may refer to a single bond.

In the present disclosure, 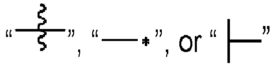 may refer to a position to be connected.

Hereinafter, a light emitting element according to one or more embodiments will be explained in more detail referring to the drawings.

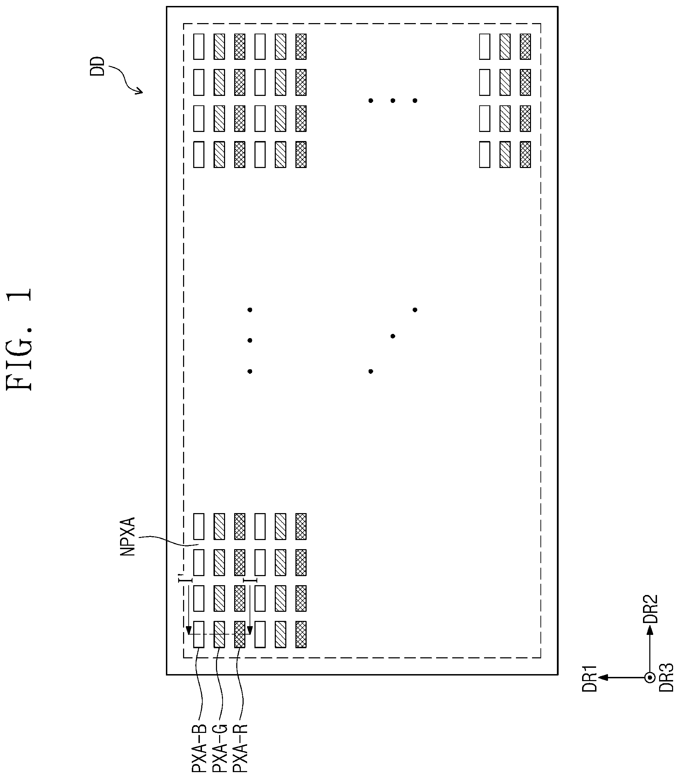

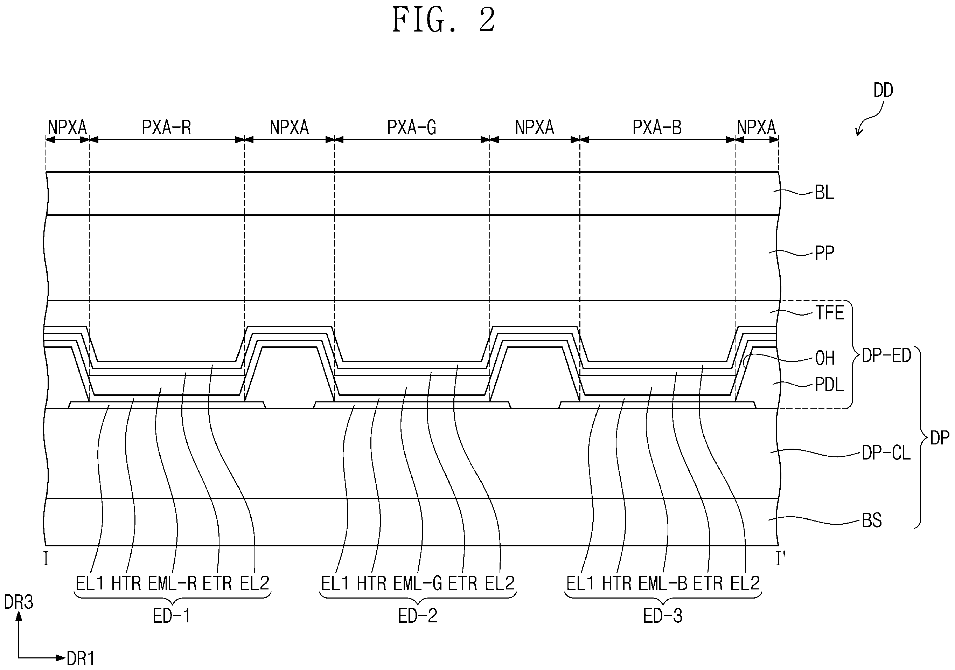

FIG. 1 is a plan view showing a display device DD according to one or more embodiments of the present disclosure. FIG. 2 is a cross-sectional view of a display device DD according to one or more embodiments of the present disclosure. FIG. 2 is a cross-sectional view showing a part corresponding to line I-I' of FIG. 1.

The display device DD may include a display panel DP and an optical layer PP disposed on the display panel DP. The display panel DP may include light emitting elements ED-1, ED-2, and ED-3. The display device DD may include a plurality of light emitting elements ED-1, ED-2, and ED-3. The optical layer PP may be disposed on the display panel DP and control reflected light by external light at the display panel DP. The optical layer PP may include, for example, a polarization layer or a color filter layer. In some embodiments, different from the drawings, the optical layer PP may not be provided in the display device DD.

On the optical layer PP, a base substrate BL may be disposed. The base substrate BL may be a member providing a base surface where the optical layer PP is disposed. The base substrate BL may be a glass substrate, a metal substrate, a plastic substrate, etc. However, embodiments of the present disclosure are not limited thereto. The base substrate BL may be an inorganic layer, an organic layer, or a composite material layer. In some embodiments, different from the drawings, the base substrate BL may not be provided.

The display device DD according to one or more embodiments may further include a plugging layer. The plugging layer may be disposed between a display element layer DP-ED and a base substrate BL. In some embodiments, the plugging layer may be an organic layer. The plugging layer may include at least one selected from among an acrylic resin, a silicon-based resin, and an epoxy-based resin.

The display panel DP may include a base layer BS, a circuit layer DP-CL provided on the base layer BS, and a display element layer DP-ED. The display element layer DP-ED may include a pixel definition film PDL, light emitting elements ED-1, ED-2, and ED-3 disposed in the pixel definition film PDL, and an encapsulating layer TFE disposed on the light emitting elements ED-1, ED-2, and ED-3.

The base layer BS may be a member providing a base surface where the display element layer DP-ED is disposed. The base layer BS may be a glass substrate, a metal substrate, a plastic substrate, etc. However, embodiments of the present disclosure are not limited thereto. The base layer BS may be an inorganic layer, an organic layer, or a composite material layer.

In one or more embodiments, the circuit layer DP-CL may be disposed on the base layer BS, and the circuit layer DP-CL may include a plurality of transistors. Each of the transistors may include a control electrode, an input electrode, and an output electrode. For example, the circuit layer DP-CL may include switching transistors and driving transistors for driving the light emitting elements ED-1, ED-2, and ED-3 of the display element layer DP-ED.

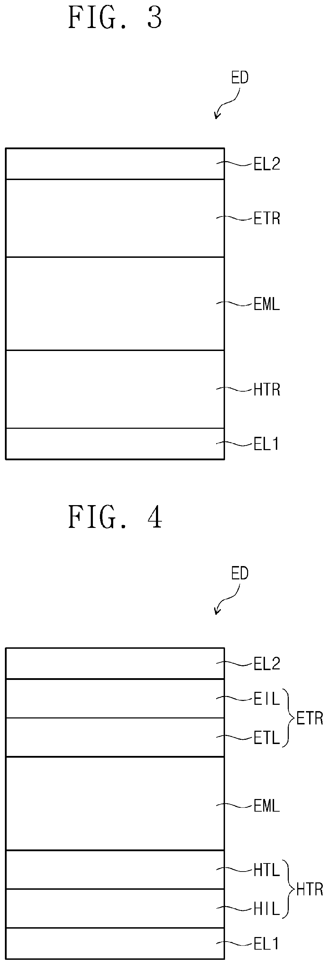

In one or more embodiments, the light emitting elements ED-1, ED-2, and ED-3 may have the structures of the light emitting elements ED according to FIG. 3 to FIG. 6, which will be explained later. Each of the light emitting elements ED-1, ED-2, and ED-3 may include a first electrode EL1, a hole transport region HTR, emission layers EML-R, EML-G, and EML-B, an electron transport region ETR, and a second electrode EL2.

In FIG. 2, shown is an embodiment where the emission layers EML-R, EML-G, and EML-B of light emitting elements ED-1, ED-2, and ED-3 are disposed in opening portions OH defined in a pixel definition film PDL, and a hole transport region HTR, an electron transport region ETR, and a second electrode EL2 are provided as common layers in all light emitting elements ED-1, ED-2, and ED-3. However, embodiments of the present disclosure are not limited thereto. Different from FIG. 2, in one or more embodiments, the hole transport region HTR and the electron transport region ETR may be patterned and provided in the opening portions OH defined in the pixel definition film PDL. For example, in one or more embodiments, the hole transport region HTR, the emission layers EML-R, EML-G, and EML-B, and the electron transport region ETR of the light emitting elements ED-1, ED-2, and ED-3 may be patterned by an inkjet printing method and provided.

An encapsulating layer TFE may cover the light emitting elements ED-1, ED-2, and ED-3. The encapsulating layer TFE may encapsulate the display element layer DP-ED. The encapsulating layer TFE may be a thin film encapsulating layer. The encapsulating layer TFE may be one layer or a stacked layer of a plurality of layers. The encapsulating layer TFE may include at least one insulating layer. The encapsulating layer TFE according to one or more embodiments may include at least one inorganic layer (hereinafter, encapsulating inorganic layer). In some embodiments, the encapsulating layer TFE according to one or more embodiments may include at least one organic layer (hereinafter, encapsulating organic layer) and at least one encapsulating inorganic layer.

The encapsulating inorganic layer protects the display element layer DP-ED from moisture/oxygen, and the encapsulating organic layer protects the display element layer DP-ED from foreign materials such as dust particles. The encapsulating inorganic layer may include silicon nitride, silicon oxy nitride, silicon oxide, titanium oxide, aluminum oxide, or one or more combinations thereof, without specific limitation. The encapsulating organic layer may include an acrylic compound, an epoxy-based compound, etc. The encapsulating organic layer may include a photopolymerizable organic material, without specific limitation.

The encapsulating layer TFE may be disposed on the second electrode EL2 and may be disposed while filling the opening portion OH.

Referring to FIG. 1 and FIG. 2, the display device DD may include a non-luminous area NPXA and luminous areas PXA-R, PXA-G, and PXA-B. The luminous areas PXA-R, PXA-G, and PXA-B may be areas emitting light produced from the light emitting elements ED-1, ED-2, and ED-3, respectively. The luminous areas PXA-R, PXA-G, and PXA-B may be separated from each other on a plane.

The luminous areas PXA-R, PXA-G, and PXA-B may be areas separated by the pixel definition film PDL. The non-luminous areas NPXA may be areas between neighboring luminous areas PXA-R, PXA-G, and PXA-B and may be areas corresponding to the pixel definition film PDL. In some embodiments of the present disclosure, each of the luminous areas PXA-R, PXA-G, and PXA-B may correspond to each pixel. The pixel definition film PDL may divide the light emitting elements ED-1, ED-2, and ED-3. The emission layers EML-R, EML-G, and EML-B of the light emitting elements ED-1, ED-2, and ED-3 may be disposed and divided in the opening portions OH defined in the pixel definition film PDL.

The luminous areas PXA-R, PXA-G, and PXA-B may be divided into a plurality of groups according to the color of light produced from the light emitting elements ED-1, ED-2, and ED-3. In the display device DD, shown in FIG. 1 and FIG. 2, three luminous areas PXA-R, PXA-G, and PXA-B emitting red light, green light, and blue light, respectively, are illustrated as an example. For example, the display device DD of one or more embodiments may include a red luminous area PXA-R, a green luminous area PXA-G, and a blue luminous area PXA-B, which are separated from each other.

In the display device DD according to one or more embodiments, a plurality of light emitting elements ED-1, ED-2, and ED-3 may be to emit light having different wavelength regions. For example, in one or more embodiments, the display device DD may include a first light emitting element ED-1 emitting red light, a second light emitting element ED-2 emitting green light, and a third light emitting element ED-3 emitting blue light. For example, the red luminous area PXA-R, the green luminous area PXA-G, and the blue luminous area PXA-B of the display device DD may correspond to the first light emitting element ED-1, the second light emitting element ED-2, and the third light emitting element ED-3, respectively.

However, embodiments of the present disclosure are not limited thereto, for example, the first to third light emitting elements ED-1, ED-2, and ED-3 may be to emit light in substantially the same wavelength region, or at least one thereof may be to emit light in a different wavelength region. For example, in one embodiment, all the first to third light emitting elements ED-1, ED-2, and ED-3 may be to emit blue light.

The luminous areas PXA-R, PXA-G, and PXA-B in the display device DD according to one or more embodiments may be arranged in a stripe shape. Referring to FIG. 1, in some embodiments, a plurality of red luminous areas PXA-R may be arranged with each other along a second direction axis DR2, a plurality of green luminous areas PXA-G may be arranged with each other along the second direction axis DR2, and a plurality of blue luminous areas PXA-B may be arranged with each other along the second direction axis DR2. In some embodiments, the red luminous area PXA-R, the green luminous area PXA-G, and the blue luminous area PXA-B may be arranged by turns along a first direction axis DR1.

In FIG. 1 and FIG. 2, the areas of the luminous areas PXA-R, PXA-G, and PXA-B are shown similar, but embodiments of the present disclosure are not limited thereto. The areas of the luminous areas PXA-R, PXA-G, and PXA-B may be different from each other according to the wavelength region of light emitted. In some embodiments, the areas of the luminous areas PXA-R, PXA-G, and PXA-B may refer to areas on a plane defined by the first direction axis DR1 and the second direction axis DR2.

In some embodiments, the arrangement type or kind of the luminous areas PXA-R, PXA-G, and PXA-B is not limited to the configuration shown in FIG. 1, and the arrangement order of the red luminous areas PXA-R, the green luminous areas PXA-G, and the blue luminous areas PXA-B may be provided in one or more suitable combinations according to the properties of display quality required for the display device DD. For example, the arrangement type or kind of the luminous areas PXA-R, PXA-G, and PXA-B may be a pentile (PENTILE^{®}) arrangement type or kind (for example, an RGBG matrix, an RGBG structure, or an RGBG matrix structure), or a diamond (Diamond Pixel ^{™}) arrangement type or kind (e.g., a display (e.g., an OLED display) containing red, blue, and green (RGB) luminous areas arranged in the shape of diamonds). PENTILE^{®} is a duly registered trademark of Samsung Display Co., Ltd. Diamond Pixel^{™} is a trademark of Samsung Display Co., Ltd.

In some embodiments, the areas of the luminous areas PXA-R, PXA-G, and PXA-B may be different from each other. For example, in one or more embodiments, the area of the green luminous area PXA-G may be smaller than the area of the blue luminous area PXA-B, but embodiments of the present disclosure are not limited thereto.

Hereinafter, FIG. 3 to FIG. 6 are cross-sectional views schematically showing light emitting elements ED according to embodiments of the present disclosure. The light emitting element ED according to one or more embodiments may include a first electrode EL1, a second electrode EL2 oppositely disposed to the first electrode EL1, and at least one functional layer disposed between the first electrode EL1 and the second electrode EL2. In one or more embodiments, the light emitting element ED may include an amine compound of the present disclosure, which will be explained later, in the at least one functional layer.

The light emitting element ED may include a hole transport region HTR, an emission layer EML, an electron transport region ETR, and/or the like, stacked in order (in the stated order), as the at least one functional layer. Referring to FIG. 3, in one or more embodiments, the light emitting element ED may include a first electrode EL1, a hole transport region HTR, an emission layer EML, an electron transport region ETR, and a second electrode EL2, stacked in order (in the stated order).

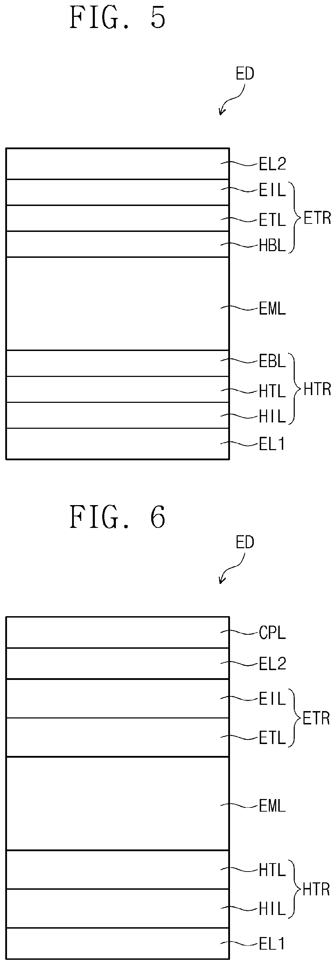

When compared with FIG. 3, FIG. 4 shows the cross-sectional view of a light emitting element ED according to one or more embodiments, wherein a hole transport region HTR may include a hole injection layer HIL and a hole transport layer HTL, and an electron transport region ETR may include an electron injection layer EIL and an electron transport layer ETL. In addition, when compared with FIG. 3, FIG. 5 shows the cross-sectional view of a light emitting element ED according to one or more embodiments, wherein a hole transport region HTR may include a hole injection layer HIL, a hole transport layer HTL, and an electron blocking layer EBL, and an electron transport region ETR may include an electron injection layer EIL, an electron transport layer ETL, and a hole blocking layer HBL. When compared with FIG. 4, FIG. 6 shows the cross-sectional view of a light emitting element ED according to one or more embodiments, including a capping layer CPL disposed on the second electrode EL2.

In one or more embodiments, the light emitting element ED may include an amine compound of the present disclosure, which will be explained later, in a hole transport region HTR. In one or more embodiments, the light emitting element ED may include an amine compound of the present disclosure in at least one selected from among the hole injection layer HIL, hole transport layer HTL, and electron blocking layer EBL of the hole transport region HTR. For example, in some embodiments, in the light emitting element ED, the hole transport layer HTL may include an amine compound according to one or more embodiments of the present disclosure.

In the light emitting element ED according to one or more embodiments of the present disclosure, the first electrode EL1 has conductivity (e.g., is a conductor). The first electrode EL1 may be formed utilizing a metal material, a metal alloy, or a conductive compound. The first electrode EL1 may be an anode or a cathode. However, embodiments of the present disclosure are not limited thereto. In some embodiments, the first electrode EL1 may be a pixel electrode. The first electrode EL1 may be a transmissive electrode, a transflective electrode, or a reflective electrode. The first electrode EL1 may include at least one selected from among silver (Ag), magnesium (Mg), copper (Cu), aluminum (Al), platinum (Pt), palladium (Pd), gold (Au), nickel (Ni), neodymium (Nd), iridium (Ir), chromium (Cr), lithium (Li), calcium (Ca), lithium fluoride (LiF), molybdenum (Mo), titanium (Ti), tungsten (W), indium (In), tin (Sn), and zinc (Zn), one or more compounds of two or more selected therefrom, one or more mixtures of two or more selected therefrom, and/or one or more oxides thereof.

When the first electrode EL1 is a transmissive electrode, the first electrode EL1 may include a transparent metal oxide such as indium tin oxide (ITO), indium zinc oxide (IZO), zinc oxide (ZnO), and/or indium tin zinc oxide (ITZO). When the first electrode EL1 is a transflective electrode or a reflective electrode, the first electrode EL1 may include Ag, Mg, Cu, Al, Pt, Pd, Au, Ni, Nd, Ir, Cr, Li, Ca, LiF/Ca (a stacked structure of LiF and Ca), LiF/Al (a stacked structure of LiF and Al), Mo, Ti, W, one or more compounds thereof, or one or more mixtures thereof (for example, a mixture of Ag and Mg). Also, the first electrode EL1 may have a structure including a plurality of layers including a reflective layer or a transflective layer formed utilizing the above materials, and a transmissive conductive layer formed utilizing ITO, IZO, ZnO, or ITZO. For example, the first electrode EL1 may have a three-layer structure of ITO/Ag/ITO. However, embodiments of the present disclosure are not limited thereto. The first electrode EL1 may include one or more of the above-described metal materials, combinations of two or more metal materials selected from the above-described metal materials, and/or one or more oxides of one or more the above-described metal materials. The thickness of the first electrode EL1 may be from about 700 Å to about 10,000 Å. For example, in one or more embodiments, the thickness of the first electrode EL1 may be from about 1,000 Å to about 3,000 Å.

The hole transport region HTR may be provided on the first electrode EL1. The hole transport region HTR may have a single layer formed utilizing a single material, a single layer formed utilizing a plurality of different materials, or a multilayer structure including a plurality of layers formed utilizing a plurality of different materials.

The hole transport region HTR may include at least one selected from among a hole injection layer HIL, a hole transport layer HTL, and an electron blocking layer EBL. In some embodiments, though not shown, the hole transport region HTR may include a plurality of stacked hole transport layers.

In some embodiments, differently, the hole transport region HTR may have the structure of a single layer of a hole injection layer HIL or a hole transport layer HTL, and may have a structure of a single layer formed utilizing a hole injection material and a hole transport material. In some embodiments, the hole transport region HTR may have a structure of a single layer formed utilizing a plurality of different materials, or a structure stacked from the first electrode EL1 of hole injection layer HIL/hole transport layer HTL, hole injection layer HIL/hole transport layer HTL/buffer layer, hole injection layer HIL/buffer layer, or hole transport layer HTL/buffer layer, without limitation.

The thickness of the hole transport region HTR may be, for example, about 50 Å to about 15,000 Å. The hole transport region HTR may be formed utilizing one or more suitable methods such as a vacuum deposition method, a spin coating method, a casting method, a Langmuir-Blodgett (LB) method, an inkjet printing method, a laser printing method, and/or a laser induced thermal imaging (LITI) method.

In one or more embodiments, the light emitting element ED may include the amine compound of the present disclosure in a hole transport region HTR. In one or more embodiments, in the light emitting element ED, the hole transport region HTR may include a hole injection layer HIL, a hole transport layer HTL, and an electron blocking layer EBL. For example, the hole transport layer HTL may include an amine compound of the present disclosure.

The amine compound is represented by Formula 1.

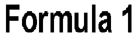

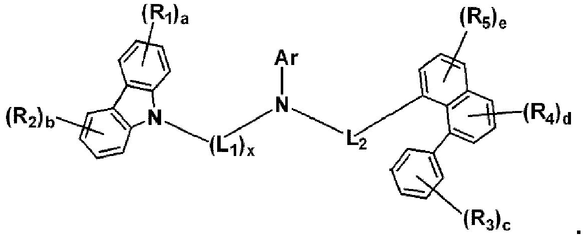

In Formula 1, L₁ and L₂ are each independently be selected from a substituted or unsubstituted arylene group and a substituted or unsubstituted heteroarylene group. For example, L₁ may be selected from a substituted or unsubstituted phenylene group, a substituted or unsubstituted divalent biphenyl group, a substituted or unsubstituted divalent terphenyl group, a substituted or unsubstituted divalent quaterphenyl group, a substituted or unsubstituted divalent fluorene group, a substituted or unsubstituted divalent dibenzofuran group, a substituted or unsubstituted divalent dibenzothiophene group, a substituted or unsubstituted divalent dibenzosilole group, and a substituted or unsubstituted divalent naphthyl group.

In one or more embodiments, L₁ may be represented by at least one selected from among the substituents in Substituent Group S.

### Substituent Group S

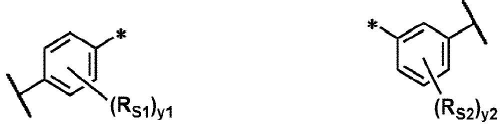

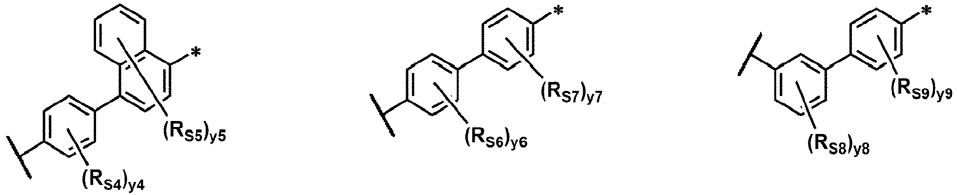

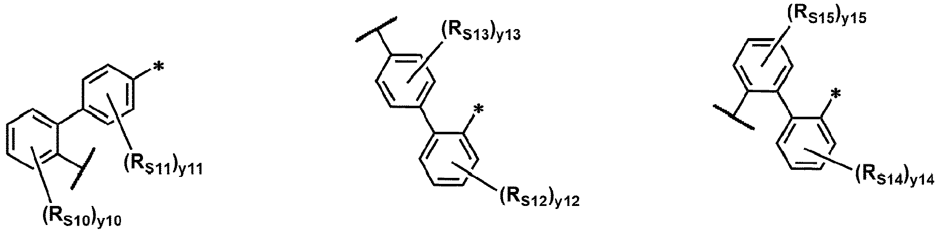

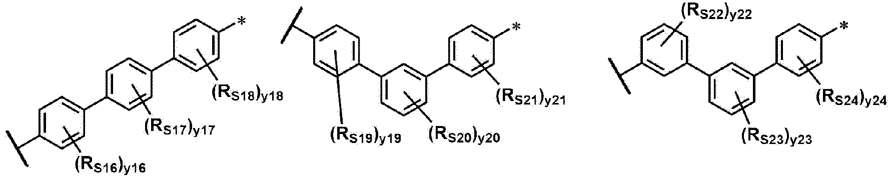

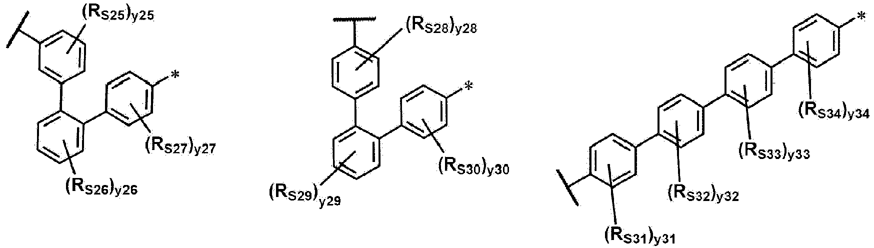

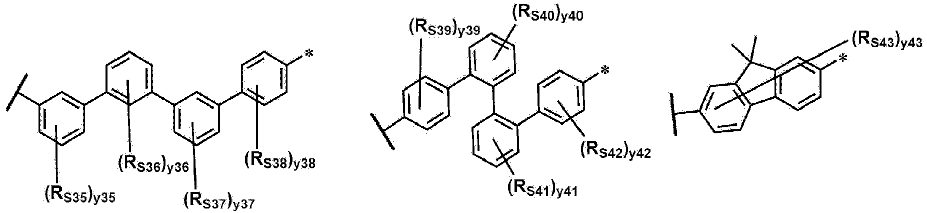

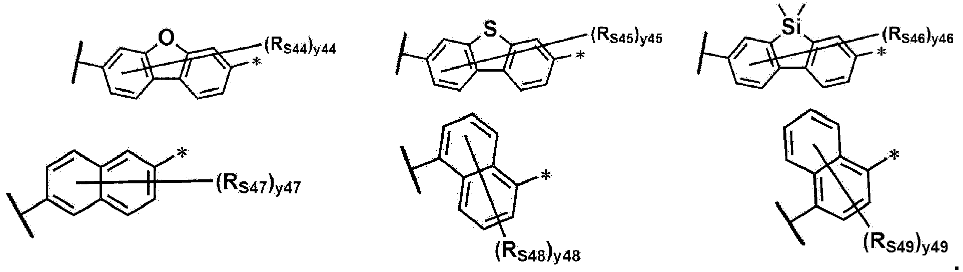

In Substituent Group S, "-∗ "refers to a position connected with the amine nitrogen atom of Formula 1, and 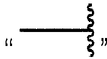 refers to a position connected with the nitrogen atom of carbazole in Formula 1.

In Substituent Group S, R_{S1}, R_{S2}, and R_{S4} to R_{S49} are each independently be selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms. For example, in some embodiments, R_{S1}, R_{S2}, and R_{S4} to R_{S49} may each independently be selected from a hydrogen atom and a deuterium atom.

y1, y2, y4, and y6 to y42 are each independently be an integer of 0 to 4, and y5 and y43 to y49 are each independently be an integer of 0 to 6. For example, y1, y2 and y4 to y49 may each be 0.

In some embodiments, a case where y1 is 0, may be the same as a case where y1 is 4, and R_{S1} is a hydrogen atom. The same may be applied for cases where y2, y4, and y6 to y42 are 0.

In some embodiments, a case where y5 is 0, may be the same as a case where y5 is 6, and R_{S5} is a hydrogen atom. The same may be applied for cases where y43 to y49 are 0.

In one or more embodiments, L₂ is selected from a substituted or unsubstituted phenylene group, a substituted or unsubstituted divalent biphenyl group, a substituted or unsubstituted divalent naphthyl group, a substituted or unsubstituted divalent phenanthryl group, and a substituted or unsubstituted divalent triphenylenyl group.

For example, in some embodiments, L₂ may be selected from an unsubstituted phenylene group, an unsubstituted divalent biphenyl group, an unsubstituted divalent naphthyl group, an unsubstituted divalent phenanthryl group, and an unsubstituted divalent triphenylenyl group.

"x" is 1 or 2. When "x" is 1, the carbazole group in Formula 1 is connected with the amine nitrogen atom of Formula 1 via one L₁ linker. When "x" is 2, the carbazole group in Formula 1 is connected with the amine nitrogen atom of Formula 1 via two L₁ linkers.

Ar is selected from a substituted or unsubstituted aryl group and a substituted or unsubstituted heteroaryl group. For example, in some embodiments, Ar may be selected from a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted phenanthryl group, a substituted or unsubstituted fluorene group, a substituted or unsubstituted carbazole group, a substituted or unsubstituted dibenzofuran group, and a substituted or unsubstituted dibenzothiophene group.

R₁ and R₂ are each independently be selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms, and/or combined with an adjacent group to form a ring. For example, in some embodiments, R₁ and R₂ may each independently be selected from a hydrogen atom, a deuterium atom, a substituted or unsubstituted methyl group, a substituted or unsubstituted phenyl group, a substituted or unsubstituted dibenzofuran group, and a substituted or unsubstituted dibenzothiophene group, and/or combined with an adjacent group to form a ring.

R₃ is selected from a hydrogen atom, a deuterium atom, a halogen atom, a substituted or unsubstituted silyl group, a substituted or unsubstituted alkenyl group of 2 to 30 carbon atoms, a substituted or unsubstituted aryl group of 6 to 30 ring-forming carbon atoms, and a substituted or unsubstituted heteroaryl group of 2 to 30 ring-forming carbon atoms, and/or combined with an adjacent group to form a ring, where a case where R₃ is an amine group-substituted aryl group is excluded. For example, in one or more embodiments, R₃ may be selected from a hydrogen atom, a deuterium atom, a substituted or unsubstituted pyridine group, a substituted or unsubstituted allyl group, a substituted or unsubstituted tetrazole group, a substituted or unsubstituted dibenzofuran group, and a substituted or unsubstituted triazine group, and/or combined with an adjacent group to form a ring, however, a case where R₃ is an amine group-substituted aryl group is excluded.

R₄ and R₅ are each independently be selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms. For example, R₄ and R₅ may not be combined with an adjacent group to form a ring. For example, in some embodiments, R₄ and R₅ may each independently be selected from a hydrogen atom and a deuterium atom. In some embodiments, at least one R₄ is a hydrogen atom.

"a" and "b" are each independently be an integer of 0 to 4. For example, "a" and "b" may each independently be 0 or 1. A case where "a" is 0, may be the same as a case where "a" is 4, and R₁ is a hydrogen atom. A case where "b" is 0, may be the same as a case where "b" is 4, and R₂ is a hydrogen atom.

"c" is an integer of 0 to 5. For example, "c" may be 0 to 2. A case where "c" is 0, may be the same as a case where "c" is 5, and R₃ is a hydrogen atom. When "c" is 2, two R₃ may be combined with each other to form a ring.

"d" is an integer of 0 to 3. For example, "d" may be 0. A case where "d" is 0, may be the same as a case where "d" is 3, and R₄ is a hydrogen atom.

"e" is an integer of 0 to 3. For example, "e" may be 0. A case where "e" is 0, may be the same as a case where "e" is 3, and R₅ is a hydrogen atom.

In some embodiments, when "a" is an integer of 2 or more, each of a plurality of R₁s may be the same, or at least one R₁ may be different from the remaining R₁s. The same may be applied for cases where "b" to "e" are integers of 2 or more.

The amine compound includes a substituent with a carbazole skeleton bonded to the amine nitrogen atom of the amine compound, a substituent with a phenyl group-substituted naphthalene skeleton, and a substituent of an aryl group or a heteroaryl group. Accordingly, the amine compound shows high molecular stability and excellent or suitable hole transport properties, and when utilized as the hole transport material of a light emitting element, may contribute to the improvement of the lifetime of the light emitting element.

In one or more embodiments, the amine compound may be a monoamine compound. In one or more embodiments, the amine compound may include a chemical structure in which optional hydrogen atom is substituted with deuterium atom.

In one or more embodiments, the amine compound represented by Formula 1 may be represented by any one selected from among Formula 1-1 to Formula 1-3.

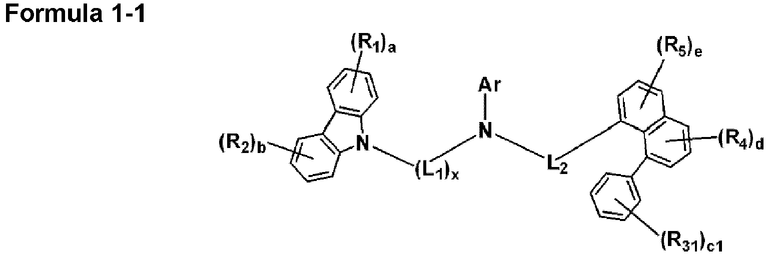

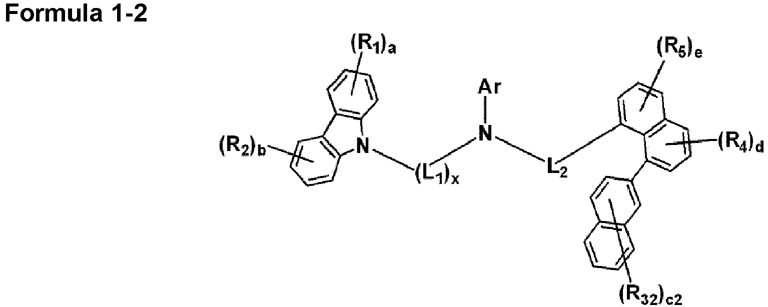

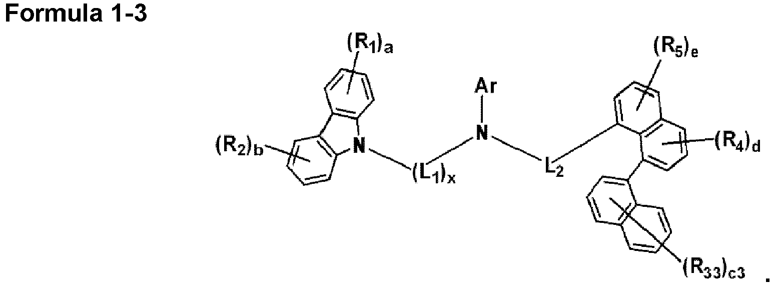

Formula 1-1 to Formula 1-3 correspond to cases of Formula 1 where R₃ is embodied.

In Formula 1-1 to Formula 1-3, R₃₁, R₃₂, and R₃₃ are each independently be selected from a hydrogen atom, a deuterium atom, a halogen atom, a substituted or unsubstituted silyl group, a substituted or unsubstituted alkenyl group of 2 to 30 carbon atoms, a substituted or unsubstituted aryl group of 6 to 30 ring-forming carbon atoms, and a substituted or unsubstituted heteroaryl group of 2 to 30 ring-forming carbon atoms. However, cases where R₃₁, R₃₂, and R₃₃ are aryl groups substituted with an amine group are excluded. For example, in some embodiments, R₃₁ may be selected from a hydrogen atom, a deuterium atom, a substituted or unsubstituted allyl group, a substituted or unsubstituted pyridine group, a substituted or unsubstituted dibenzofuran group, a substituted or unsubstituted tetrazole group, and a substituted or unsubstituted triazine group.

For example, in some embodiments, R₃₂ and R₃₃ may each independently be selected from a hydrogen atom, a deuterium atom, and a substituted or unsubstituted allyl group.

c1 is an integer of 0 to 5. For example, c1 may be 0 or 1. A case where c1 is 0, may be the same as a case where c1 is 5, and R₃₁ is a hydrogen atom.

c2 and c3 are each independently be an integer of 0 to 7. For example, c2 and c3 may each be 0. A case where c2 is 0, may be the same as a case where c2 is 7, and R₃₂ is a hydrogen atom. A case where c3 is 0, may be the same as a case where c3 is 7, and R₃₃ is a hydrogen atom.

In Formula 1-1 to Formula 1-3, Ar, R₁, R₂, R₄, R₅, L₁, L₂, "a", "b", "d", "e", and "x" are each independently be the same as defined in Formula 1.

Referring to Formula 1-1 to Formula 1-3, in one or more embodiments, the amine compound may be characterized in that a phenyl group-substituted naphthyl group or a naphthyl group-substituted naphthyl group is connected with the amine nitrogen atom of the amine compound via a linker. Accordingly, the amine nitrogen atom of the amine compound may be three-dimensionally protected, and molecular stability of the amine compound during charge transport may be improved. In some embodiments, the phenyl group-substituted naphthyl group or the naphthyl group-substituted naphthyl group may interact with the linker to improve the charge transport capacity of the amine compound even further. Therefore, a hole transport layer including the amine compound of the present disclosure may efficiently transport holes to an emission layer.

In one or more embodiments, the amine compound represented by Formula 1 may be represented by any one selected from among Formula 2-1 to Formula 2-6.

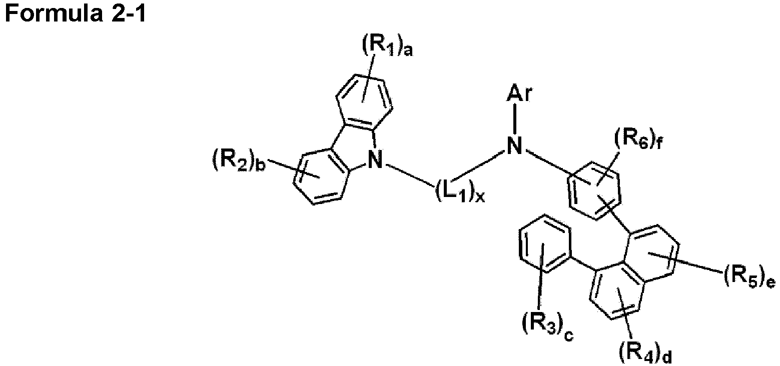

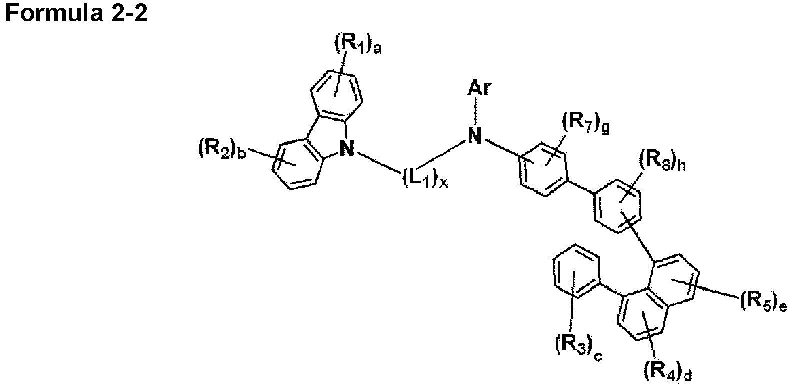

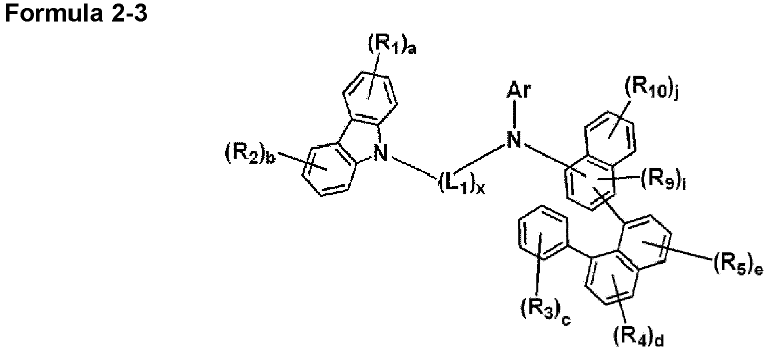

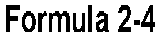

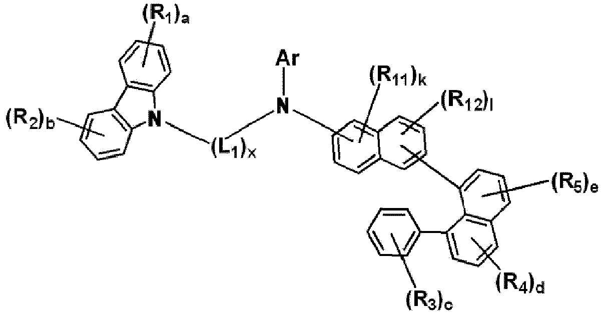

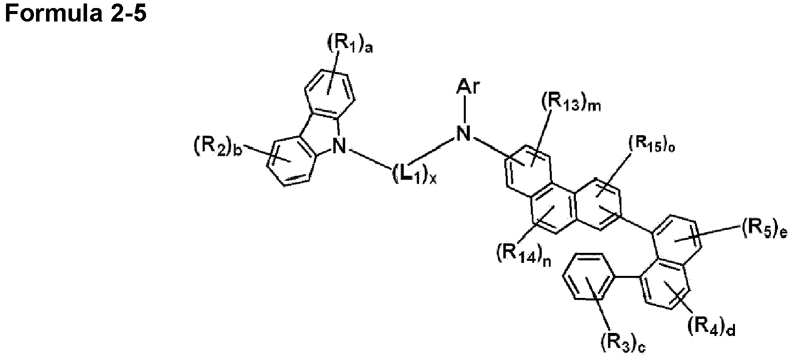

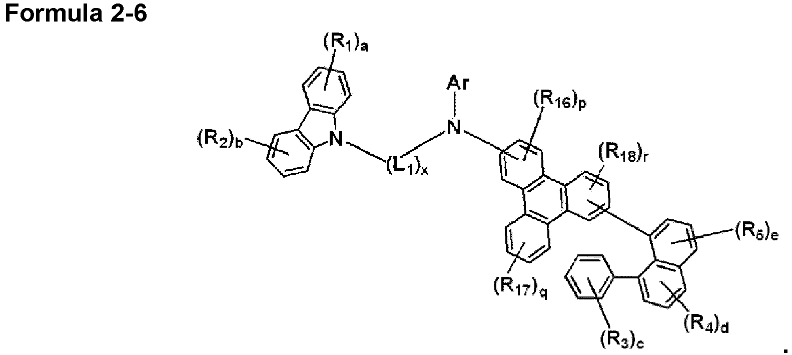

Formula 2-1 to Formula 2-6 correspond to cases of Formula 1 where L₂ is embodied.

In Formula 2-1 to Formula 2-6, R₆ to R₁₈ are each independently be selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms. For example, in some embodiments, R₆ to R₁₈ may each independently be selected from a hydrogen atom and a deuterium atom.

"f", "g", "h", "j", and "q" are each independently be an integer of 0 to 4. For example, "f", "g", "h", "j", and "q" may each be 0. A case where "f" is 0, may be the same as a case where "f" is 4, and R₆ is a hydrogen atom. The same may be applied for cases where "g", "h", "j", and "q" are 0.

"i" and "n" are each independently be an integer of 0 to 2. For example, "i" and "n" may each be 0. A case where "i" is 0, may be the same as a case where "i" is 2, and R₉ is a hydrogen atom. The same may be applied for a case where "n" is 0.

"k", "l", "m", "o", "p", and "r" are each independently be an integer of 0 to 3. For example, "k", "l", "m", "o", "p", and "r" may each be 0. A case where "k" is 0, may be the same as a case where "k" is 3, and R₁₁ is a hydrogen atom. The same may be applied for cases where "l", "m", "o", "p", and "r" are 0.

In Formula 2-1 to Formula 2-6, Ar, R₁ to R₅, L₁, "a" to "e", and "x" are each independently be the same as defined in Formula 1.

In one or more embodiments, the amine compound represented by Formula 1 may be represented by Formula 3-1 or Formula 3-2.

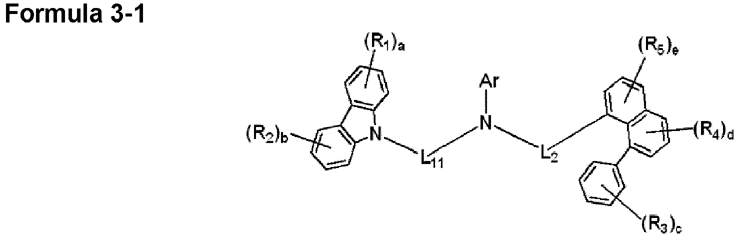

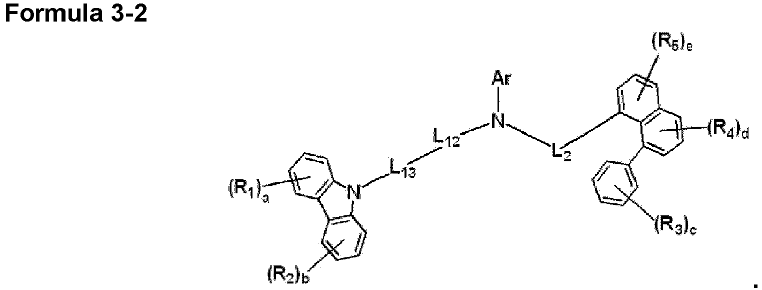

Formula 3-1 and Formula 3-2 correspond to cases of Formula 1 where (L₁)ₓ is embodied. Formula 3-1 illustrates a case of Formula 1 where "x" is 1, and L₁ is L₁₁. Formula 3-2 illustrates a case of Formula 1 where "x" is 2, and two L₁ are L₁₂ and L₁₃.

In Formula 3-1 and Formula 3-2, L₁₁ to L₁₃ are each independently be selected from a substituted or unsubstituted arylene group of 6 to 30 ring-forming carbon atoms and a substituted or unsubstituted heteroarylene group of 2 to 30 ring-forming carbon atoms. For example, in one or more embodiments, L₁₁ may be selected from a substituted or unsubstituted phenylene group, a substituted or unsubstituted divalent biphenyl group, a substituted or unsubstituted divalent terphenyl group, a substituted or unsubstituted divalent quaterphenyl group, a substituted or unsubstituted divalent fluorene group, a substituted or unsubstituted divalent dibenzofuran group, a substituted or unsubstituted divalent dibenzothiophene group, a substituted or unsubstituted divalent dibenzosilole group, and a substituted or unsubstituted divalent naphthyl group. For example, in one or more embodiments, L₁₂ may be a substituted or unsubstituted phenylene group, and L₁₃ may be a substituted or unsubstituted divalent naphthyl group.

In one or more embodiments, the amine compound represented by Formula 1 may be represented by any one selected from among the compounds in Compound Group 1. In one or more embodiments, the hole transport region HTR of the light emitting element ED may include at least one selected from among the amine compounds disclosed in Compound Group 1. For example, in some embodiments, the hole transport layer HTL of the light emitting element ED may include at least one selected from among the amine compounds disclosed in Compound Group 1. In Compound Group 1, D is a deuterium atom.

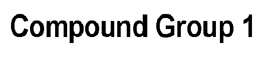

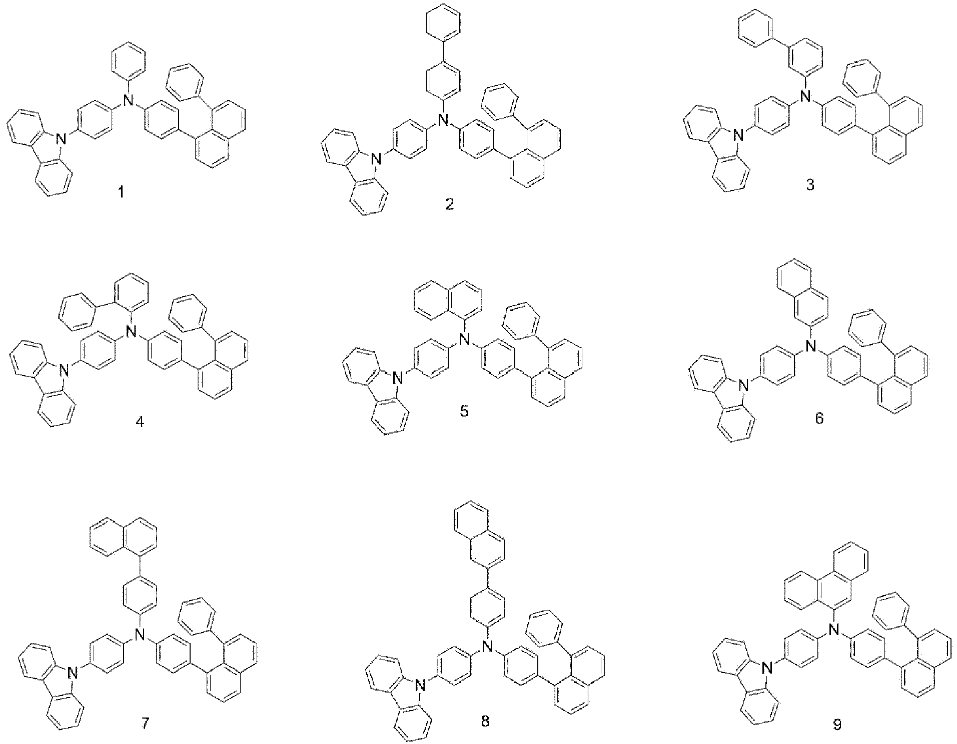

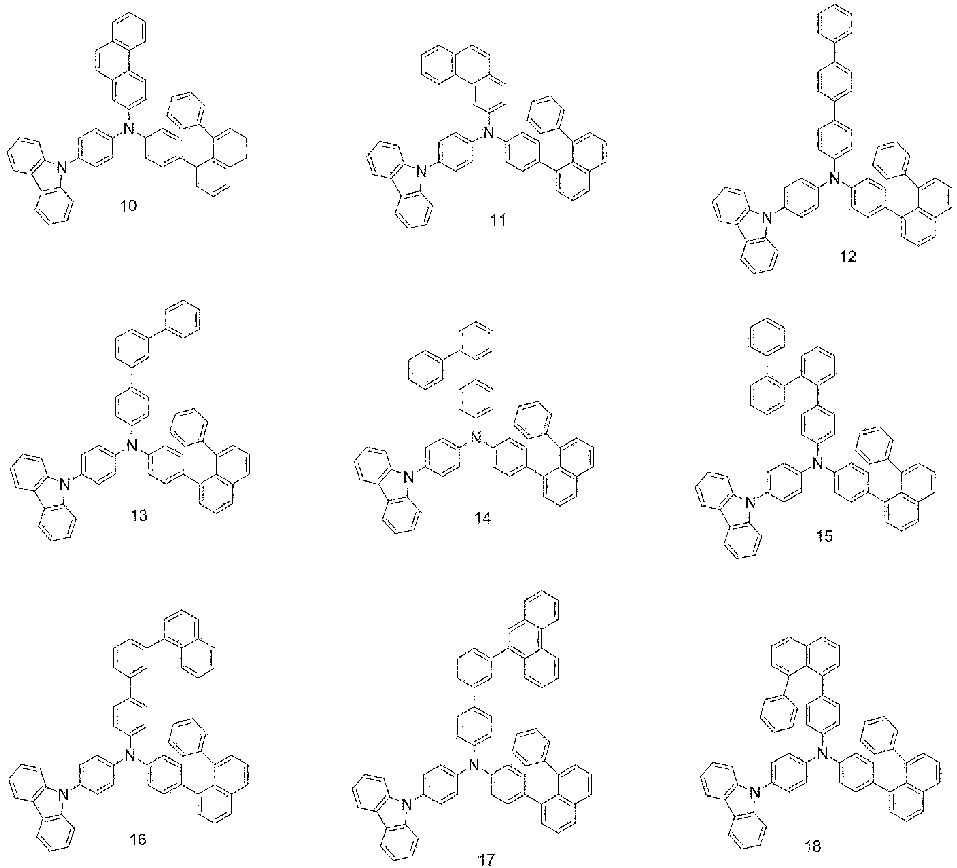

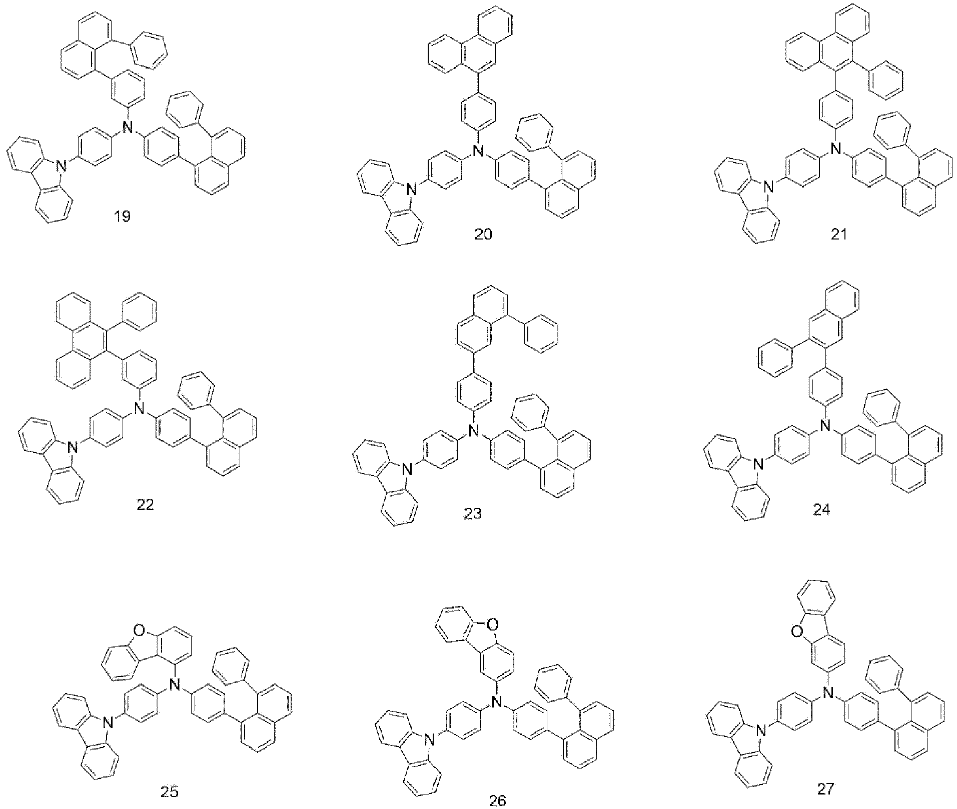

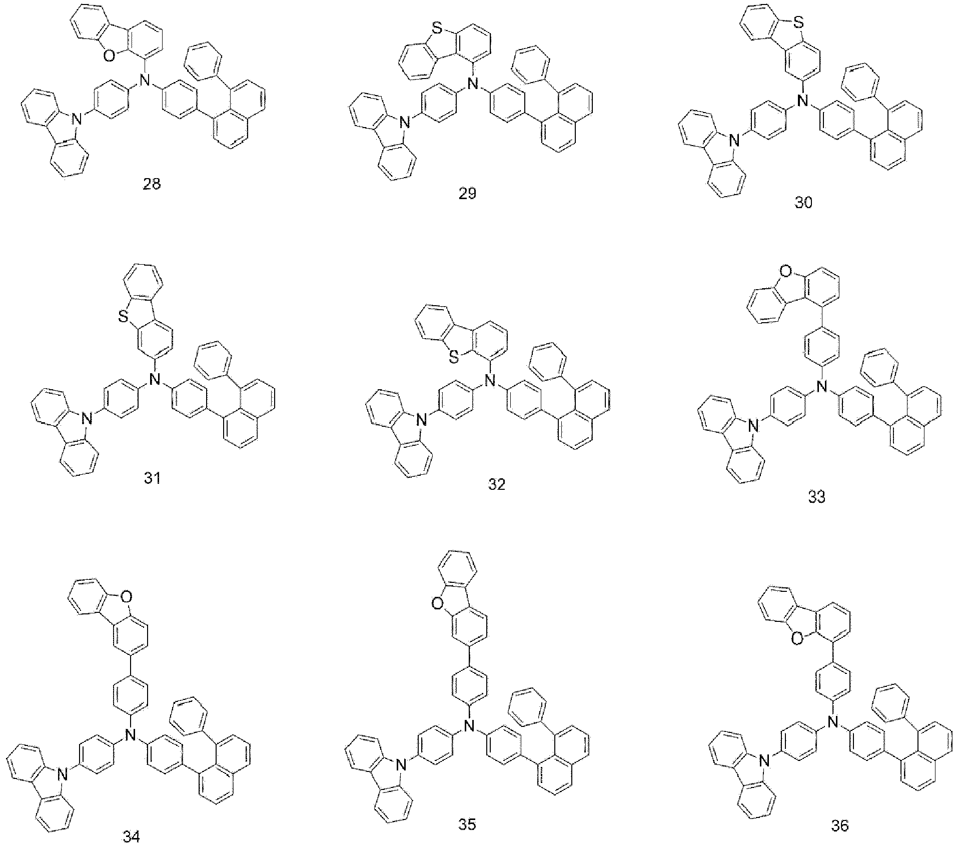

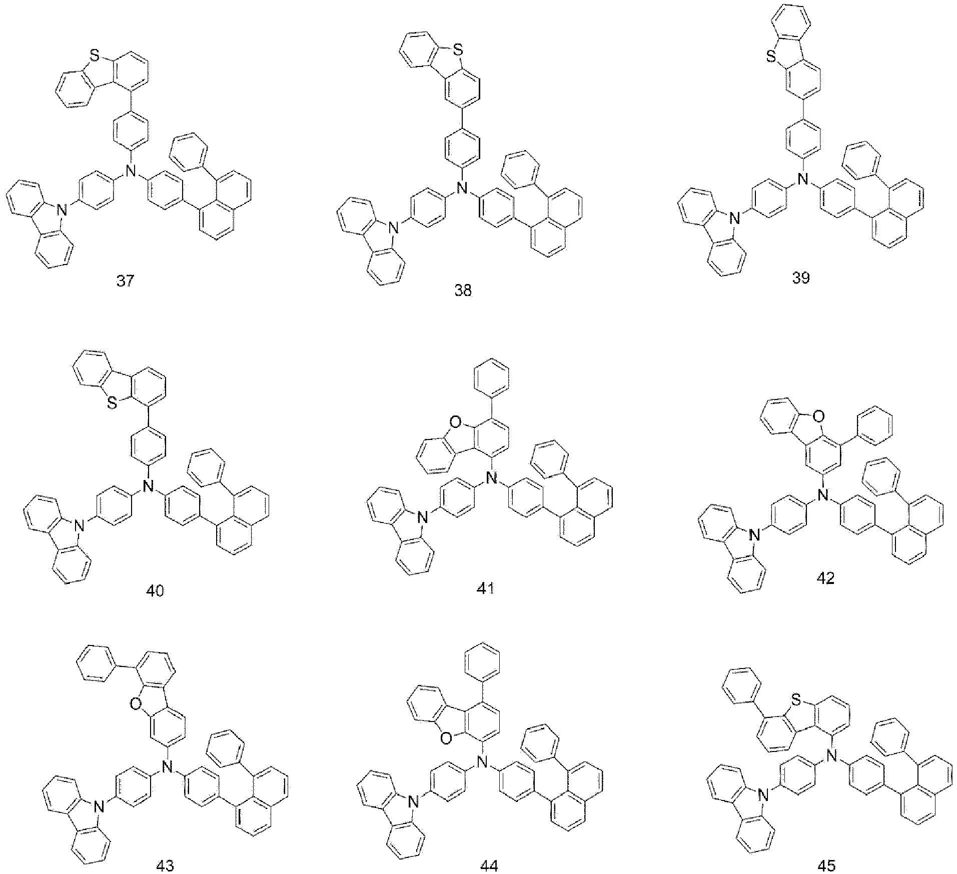

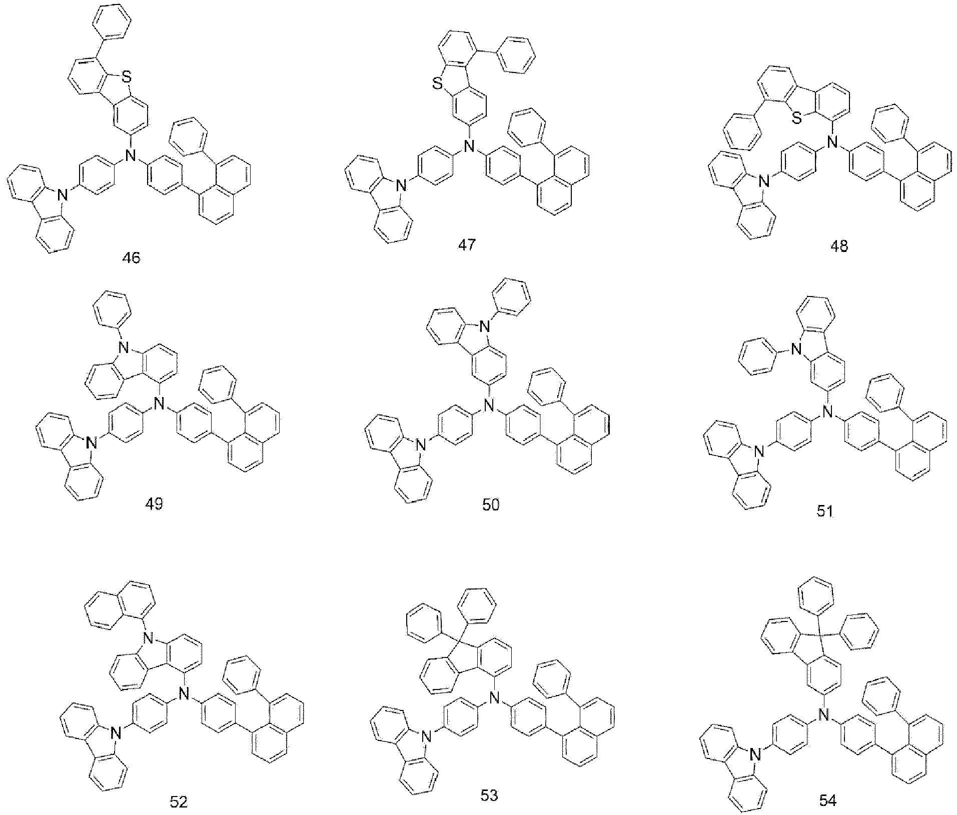

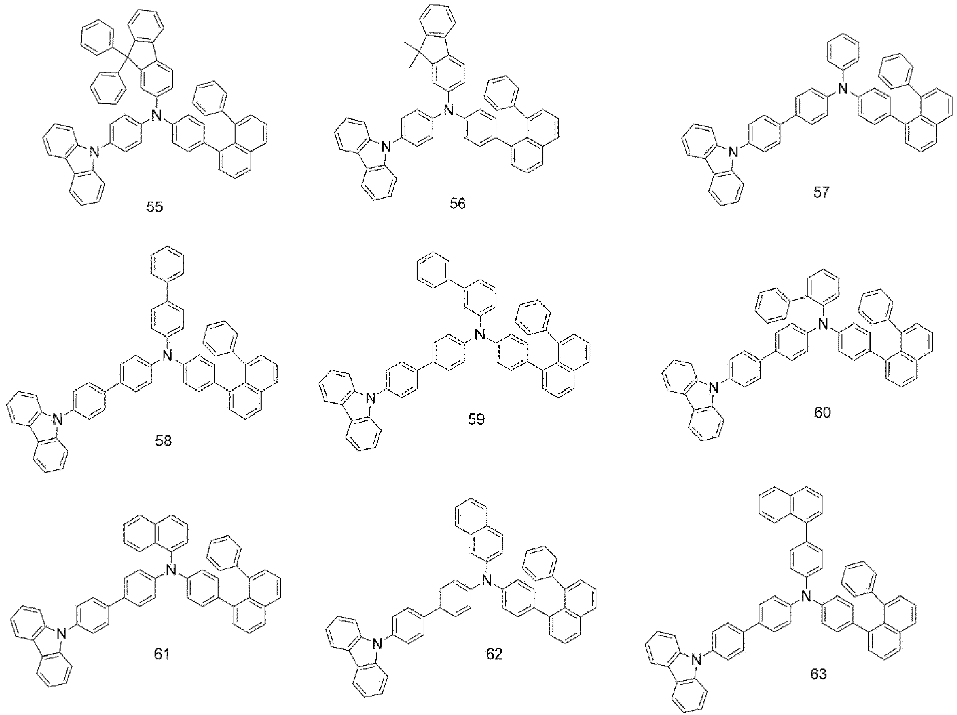

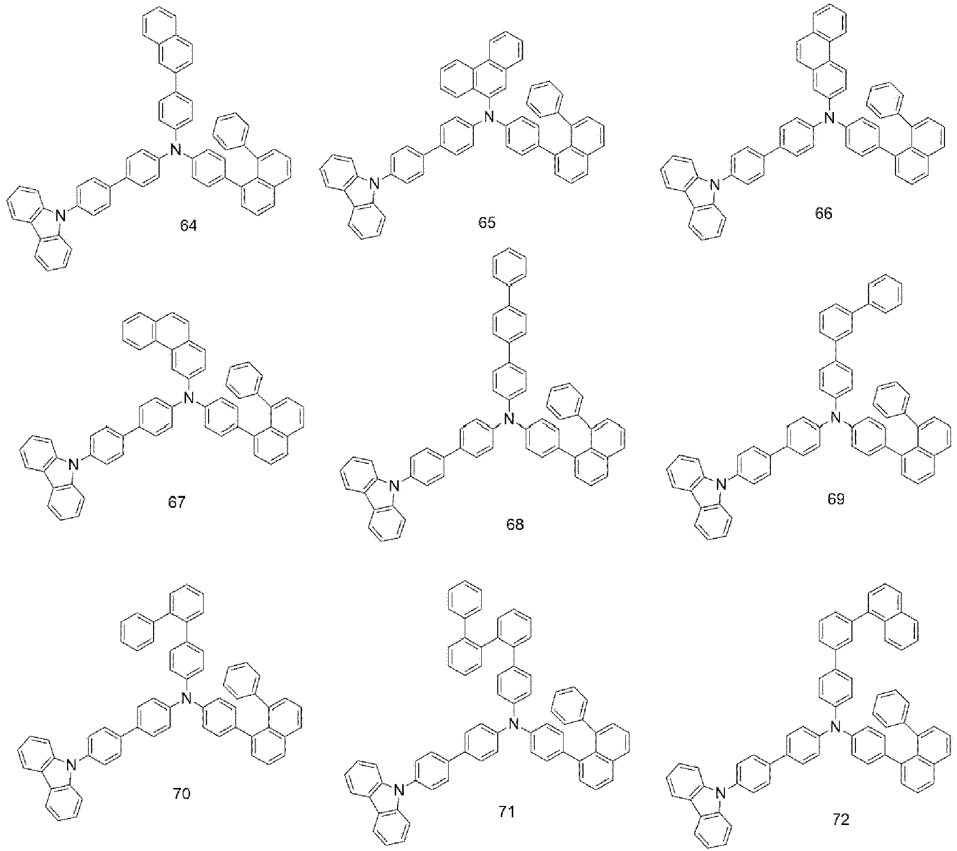

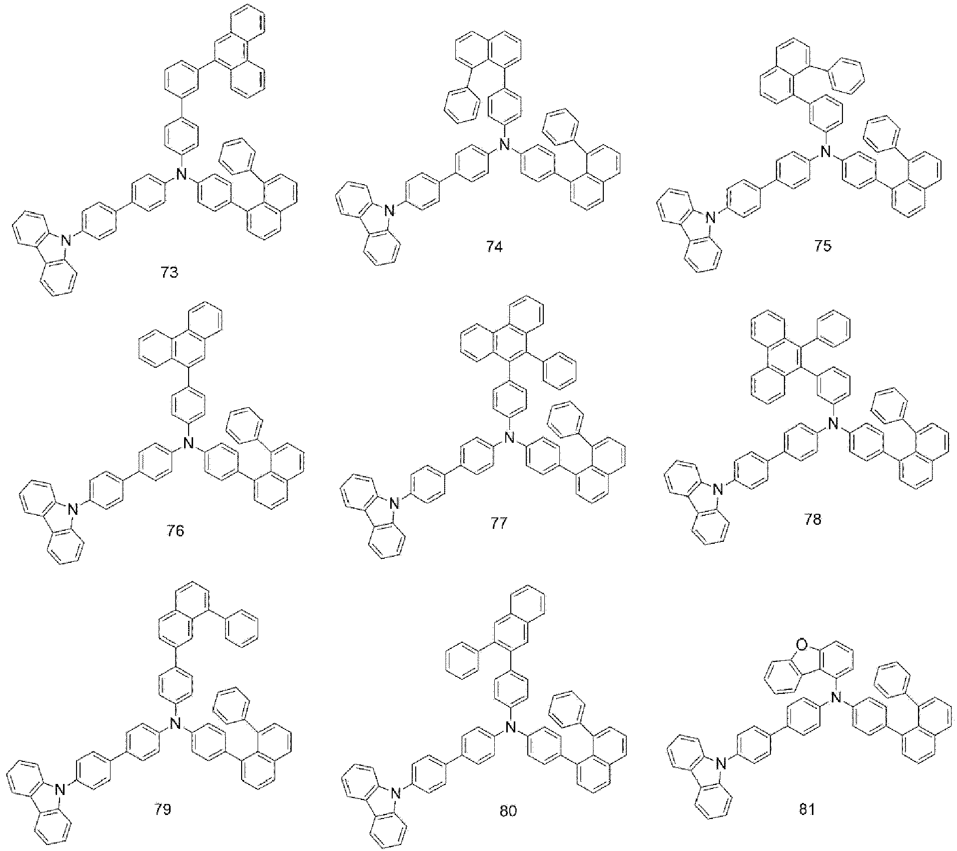

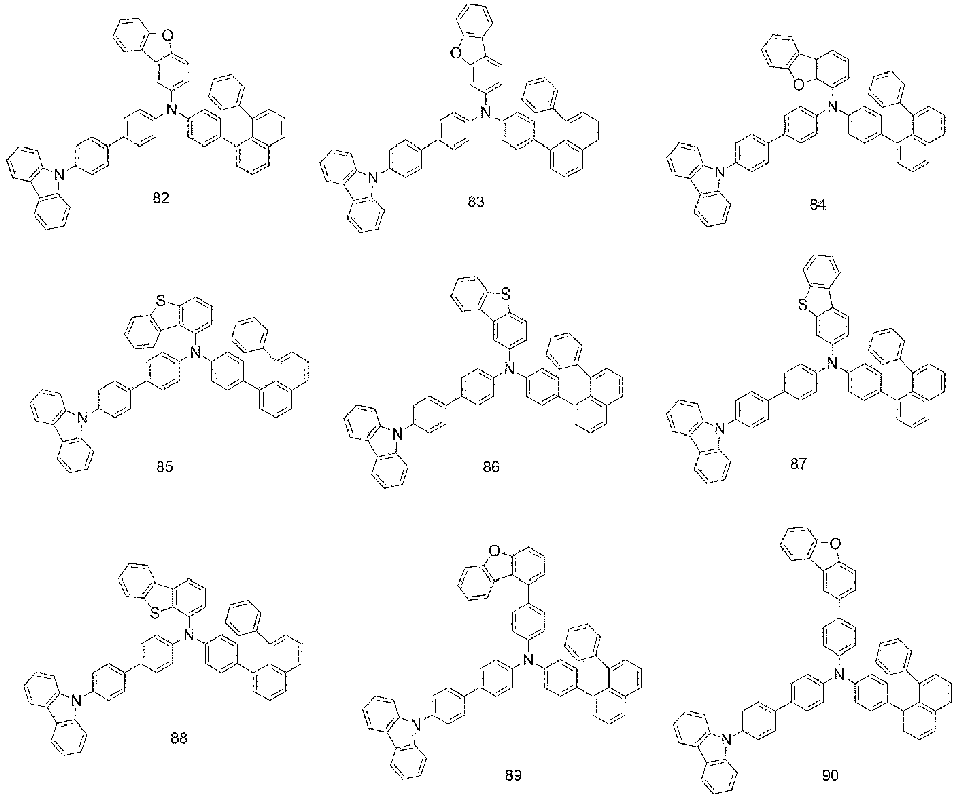

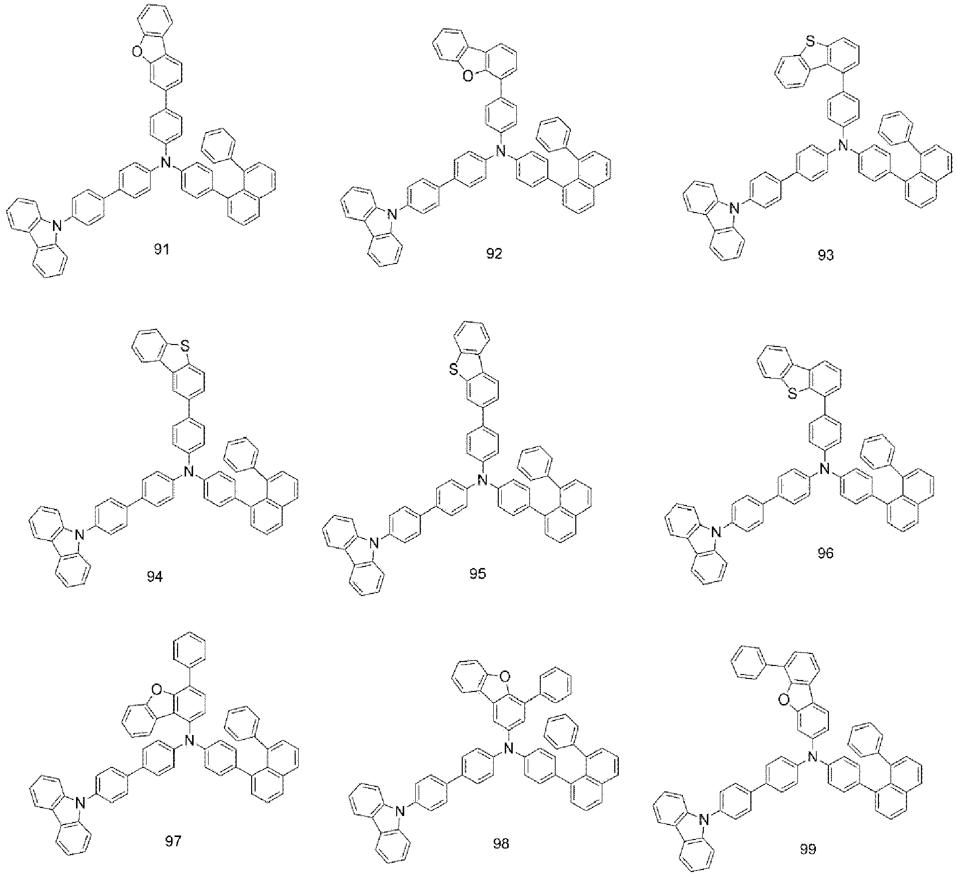

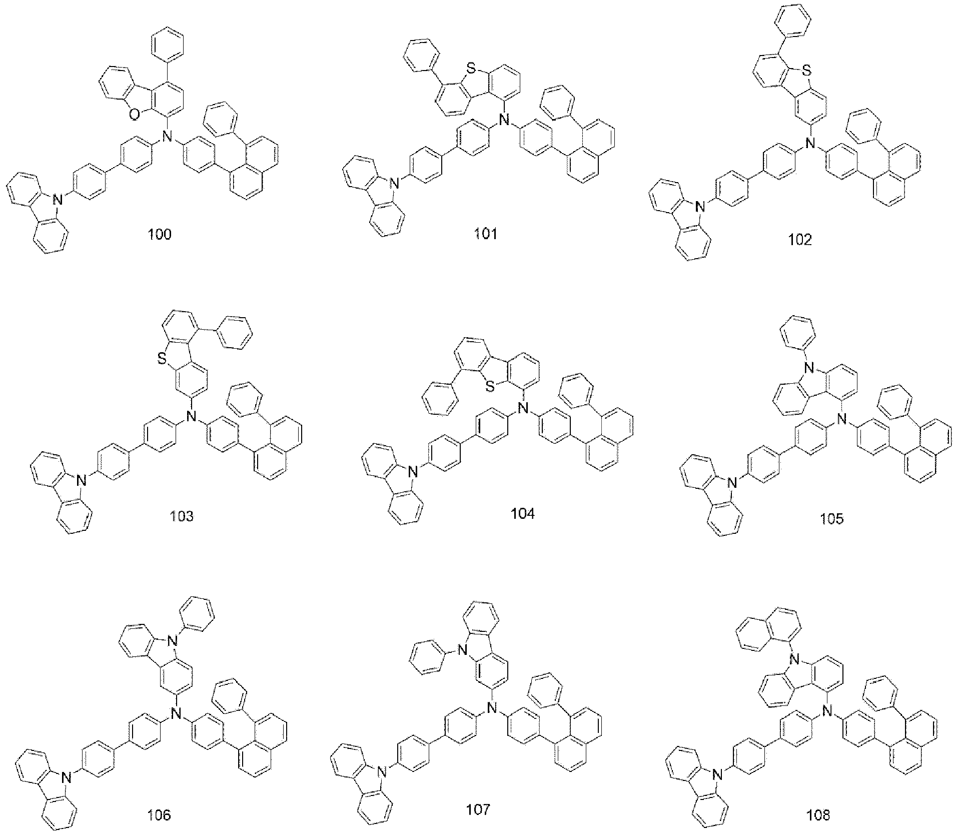

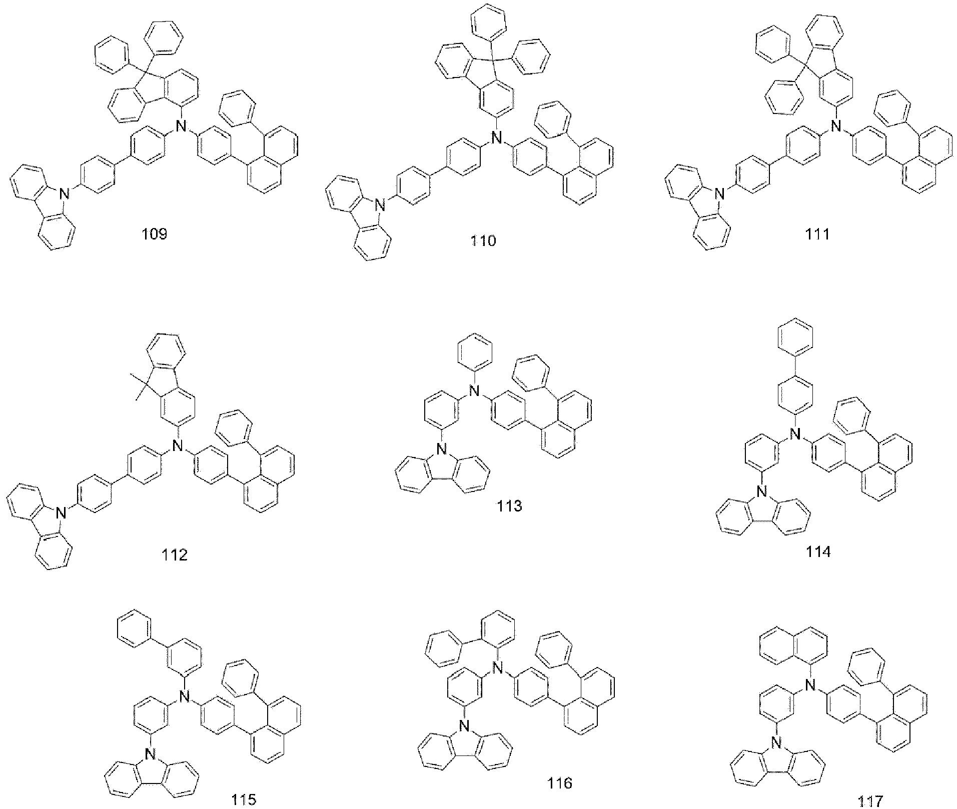

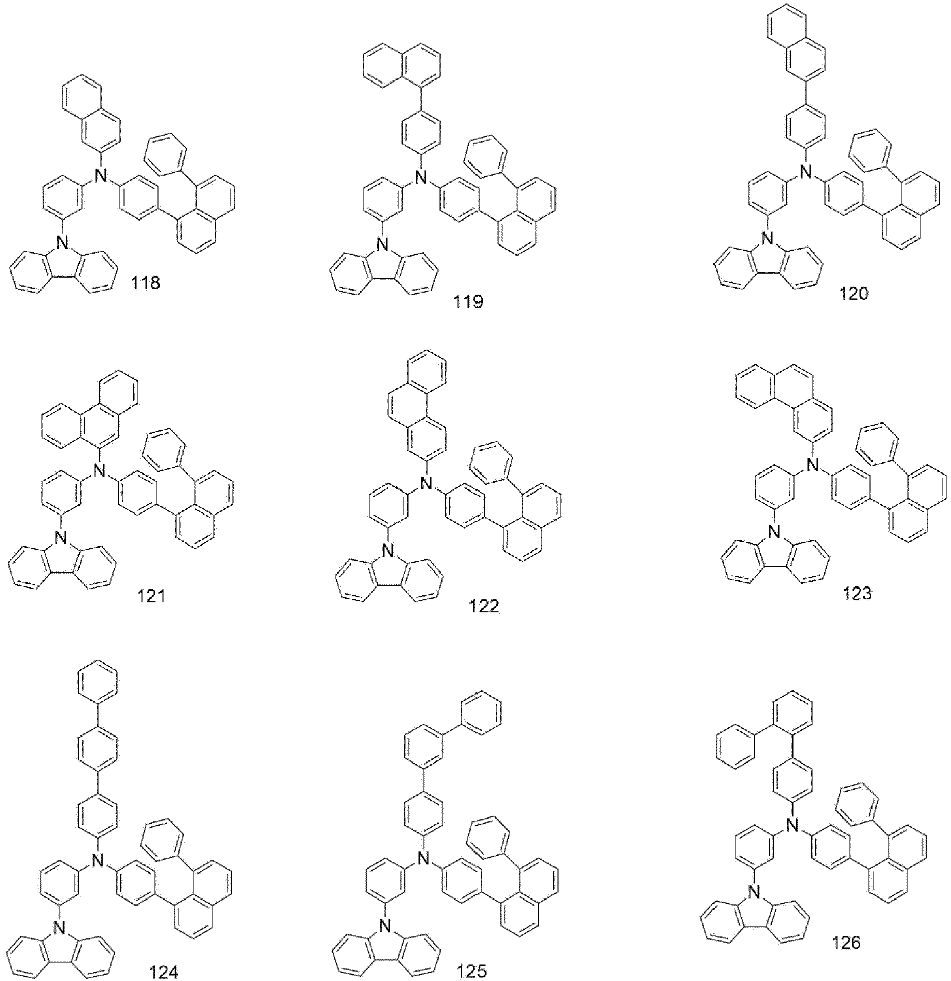

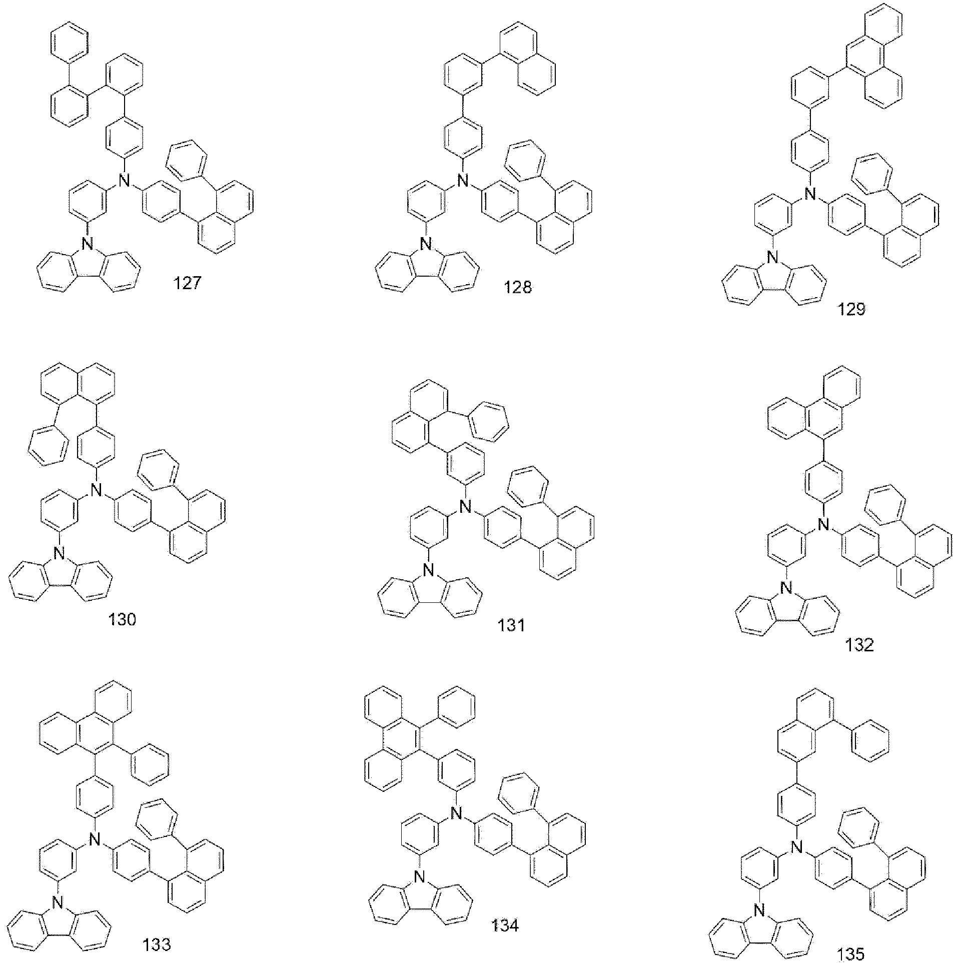

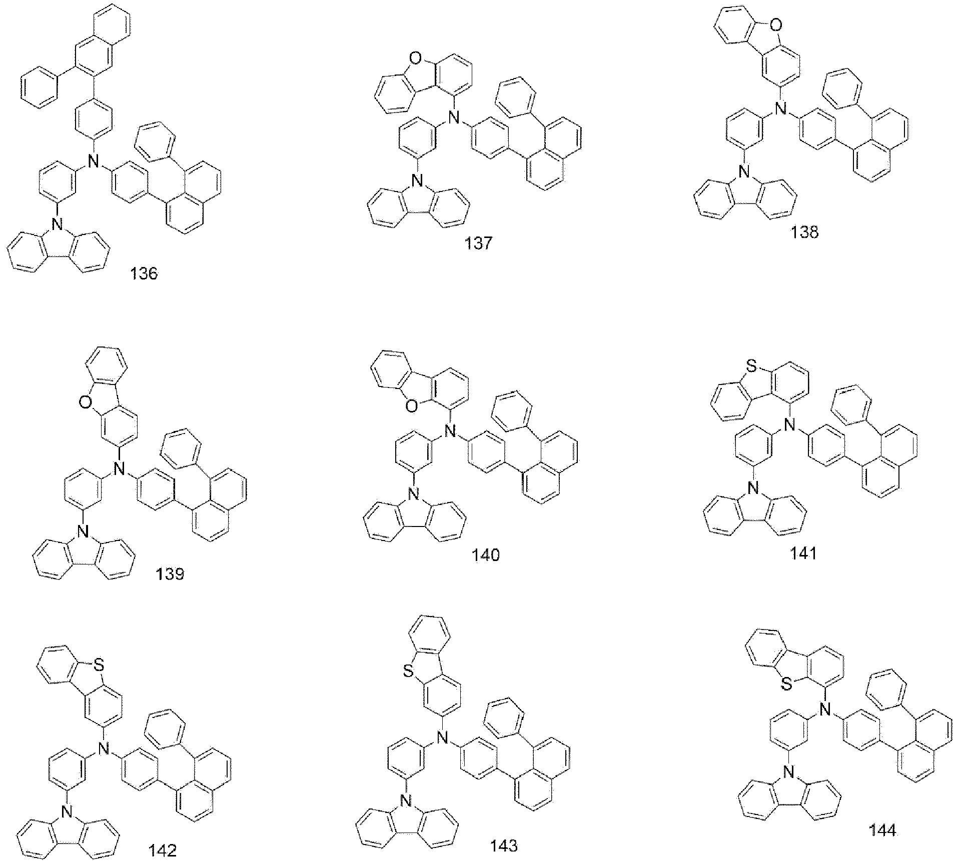

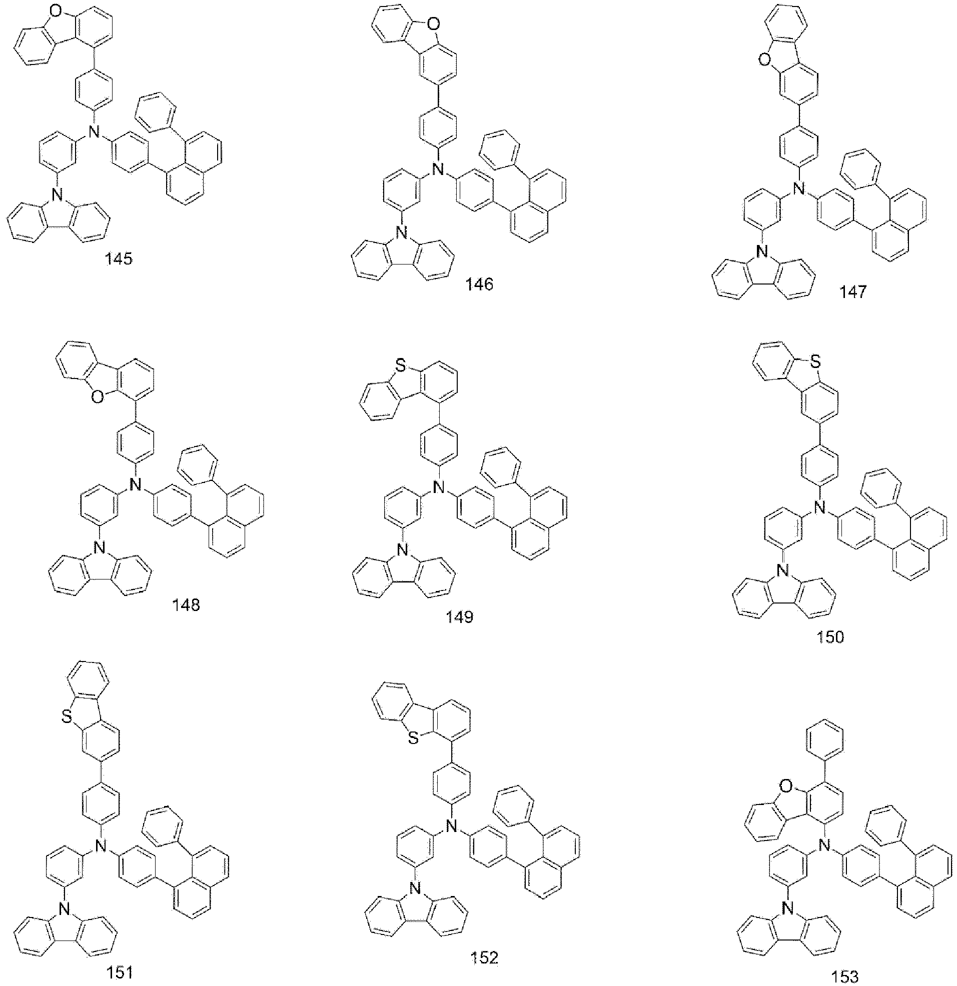

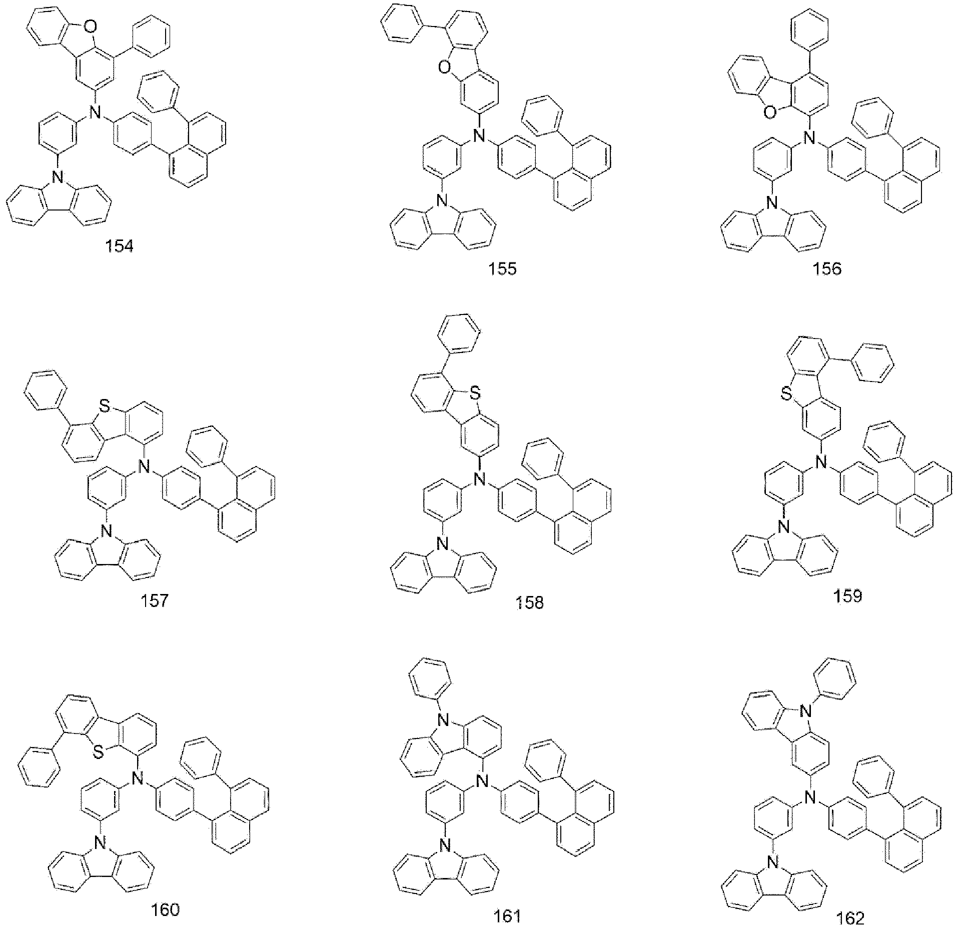

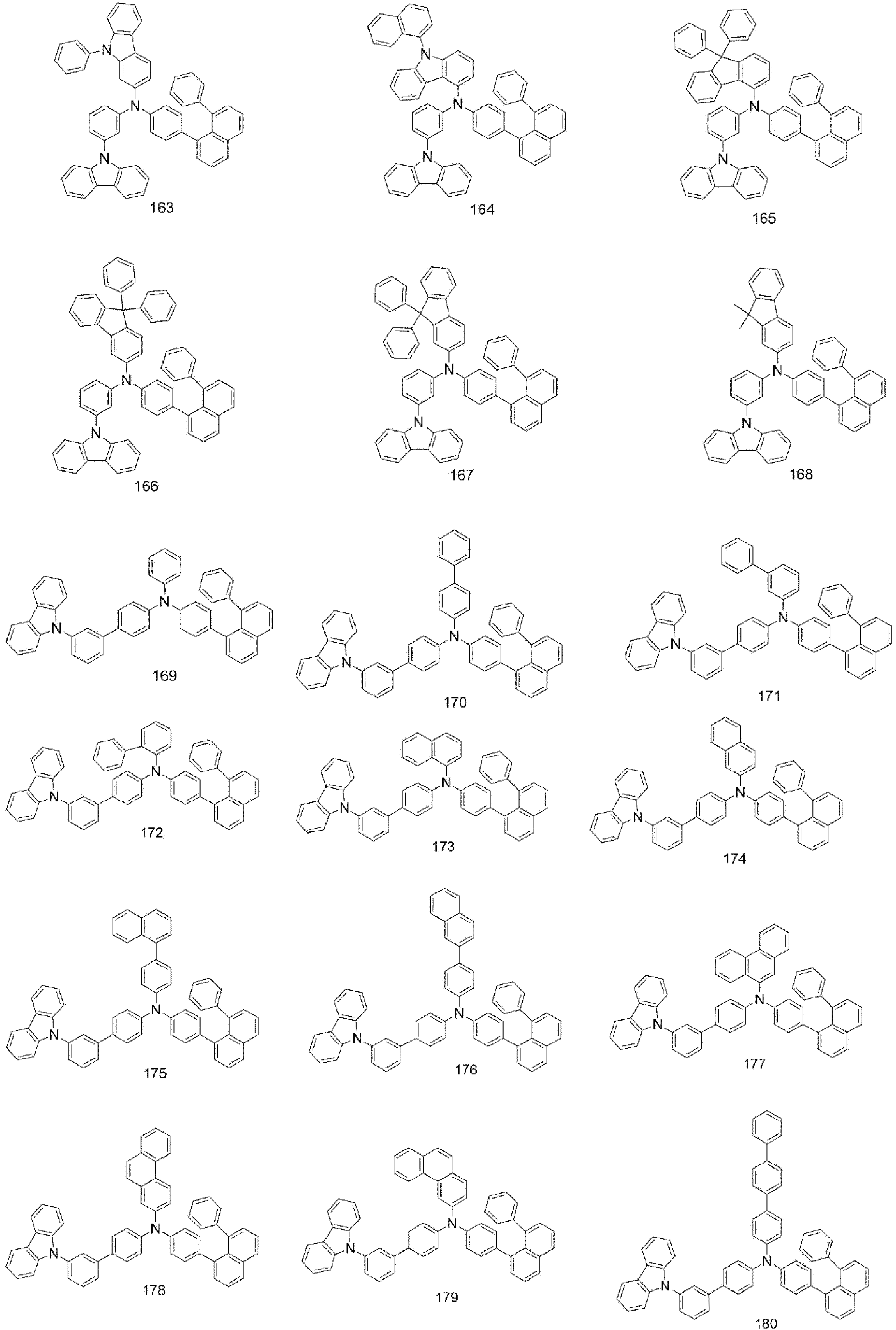

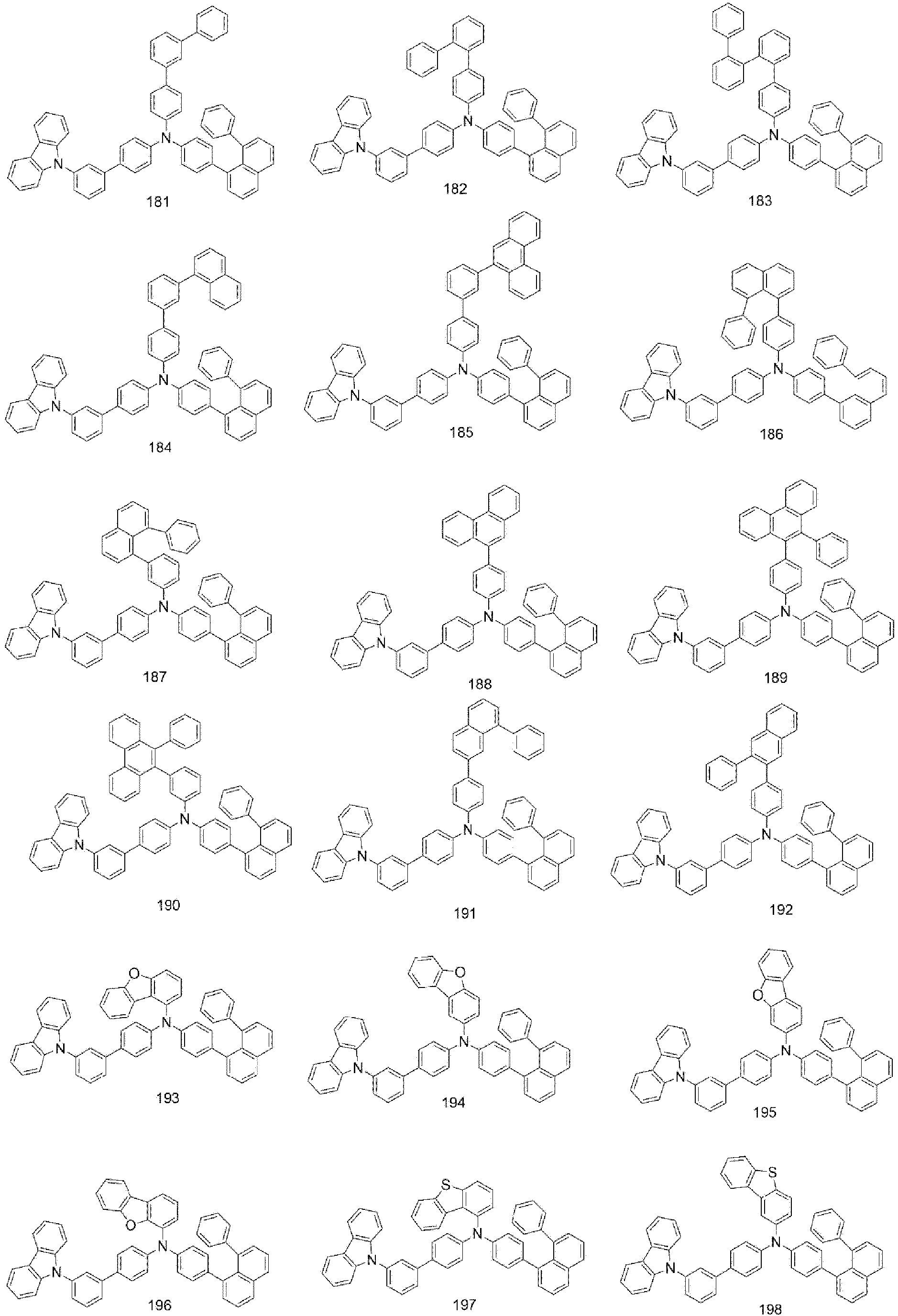

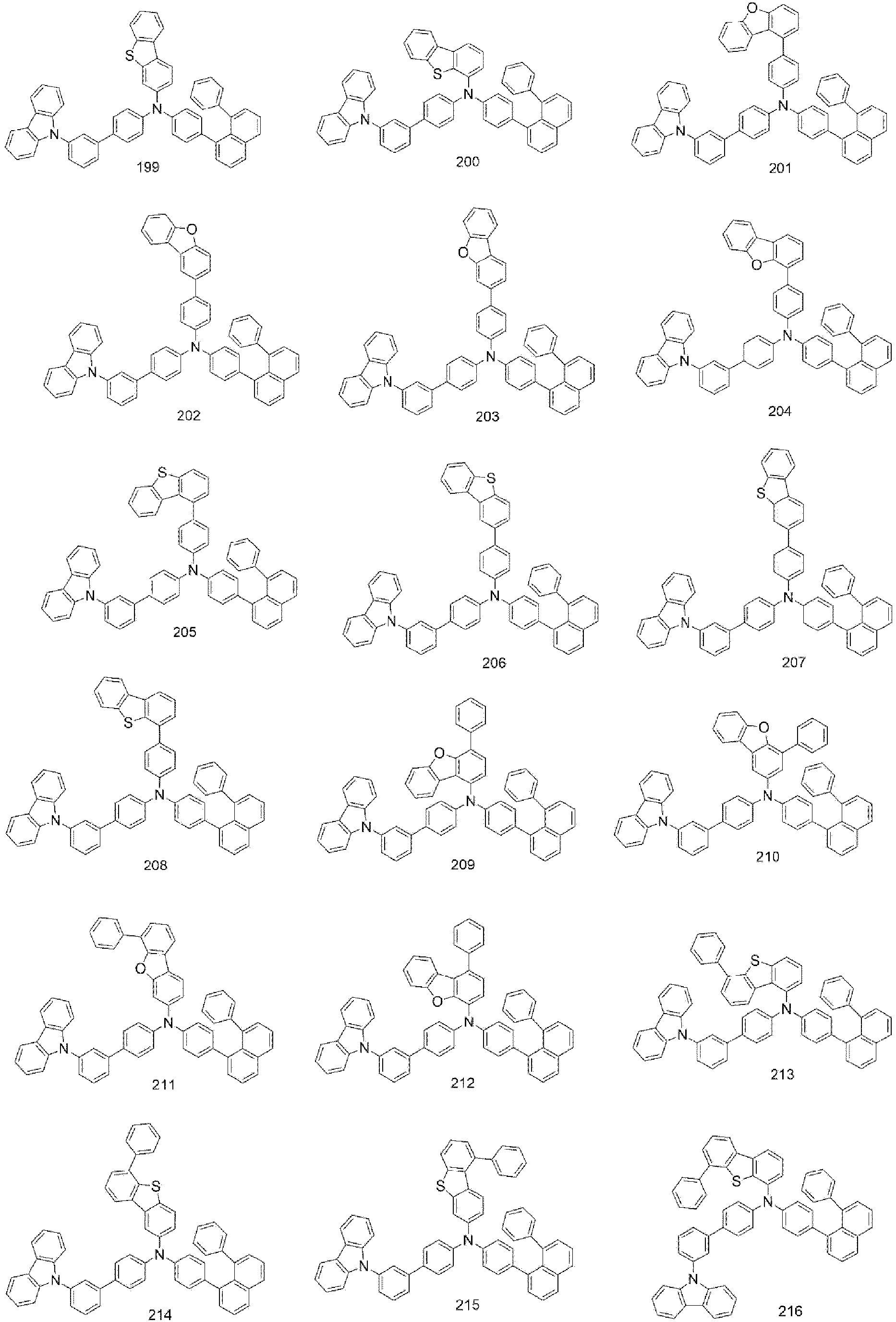

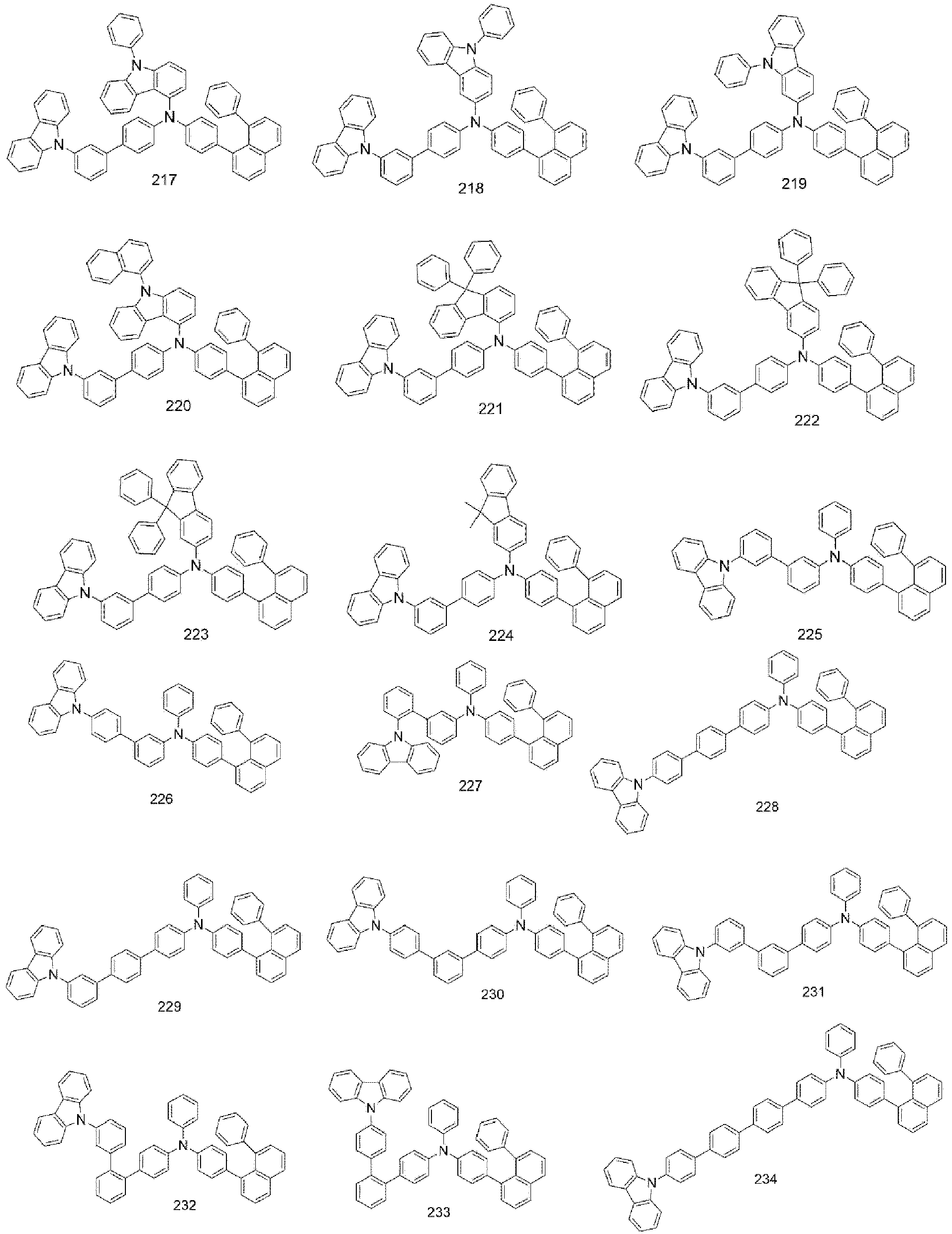

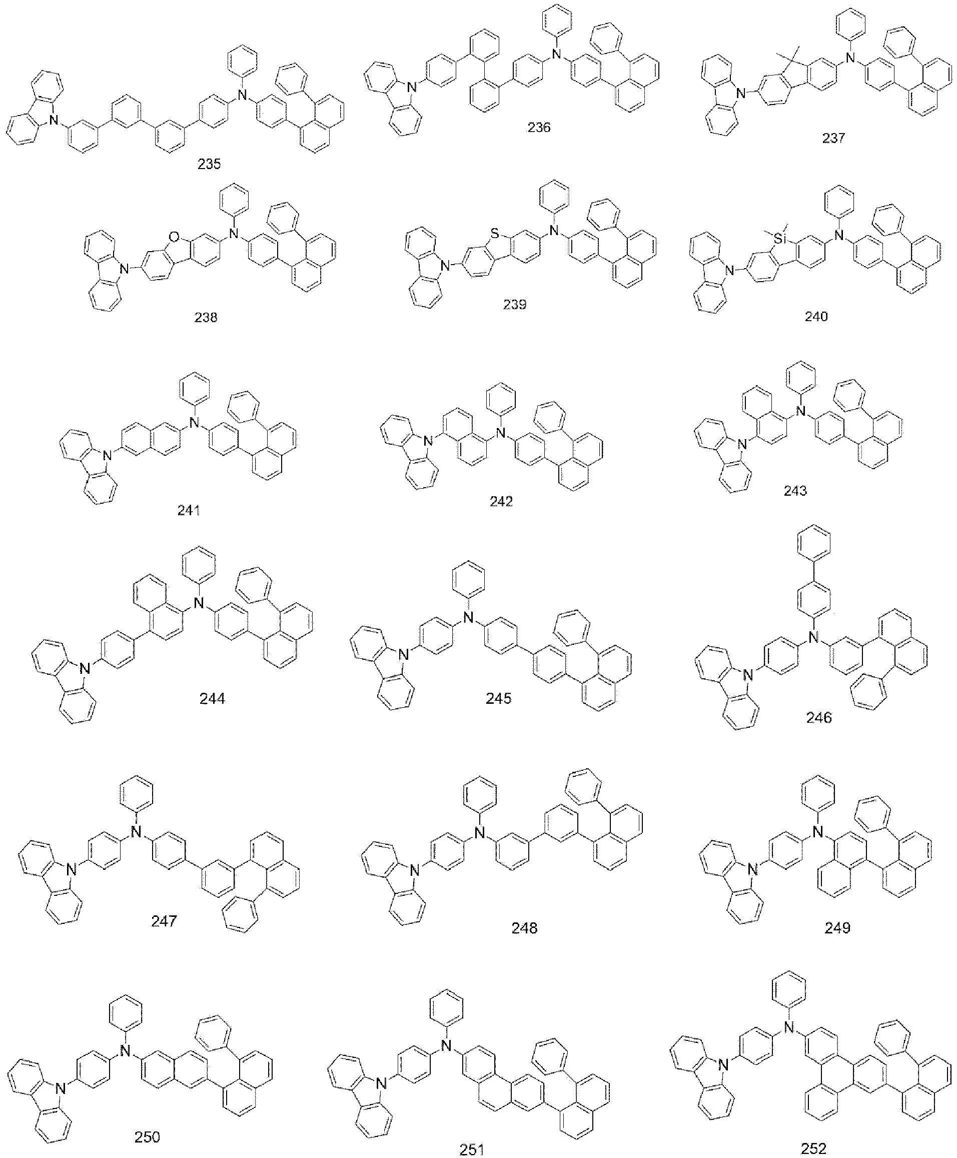

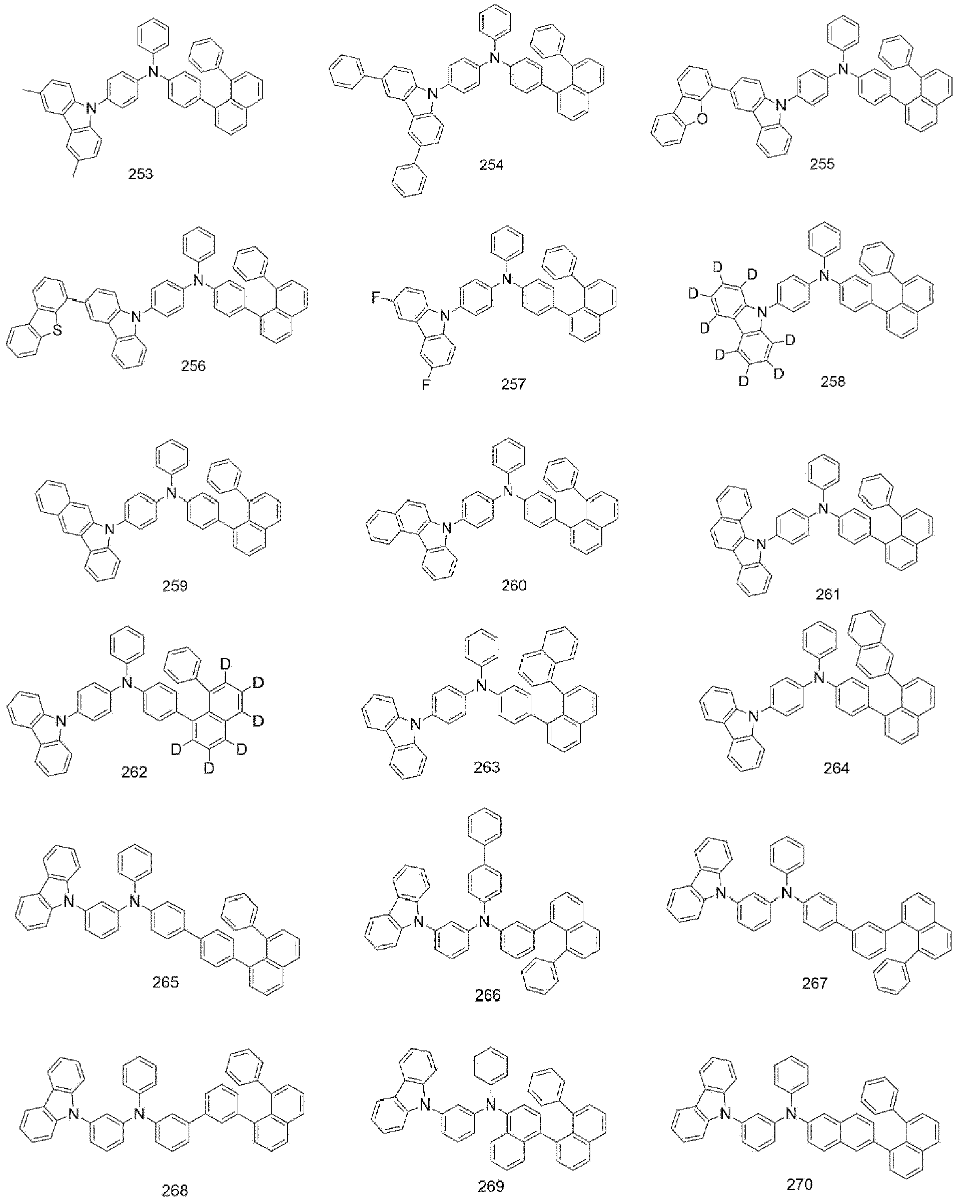

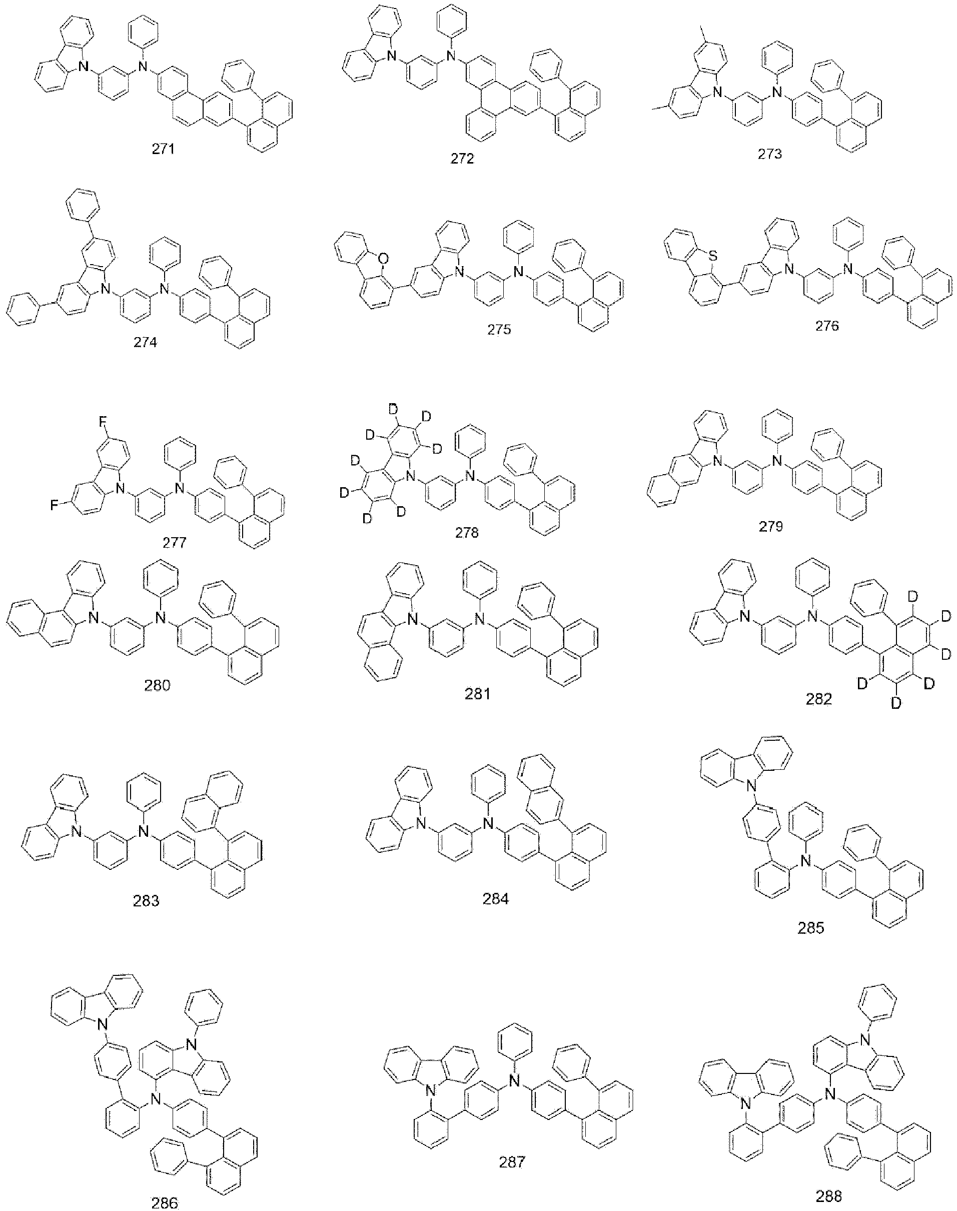

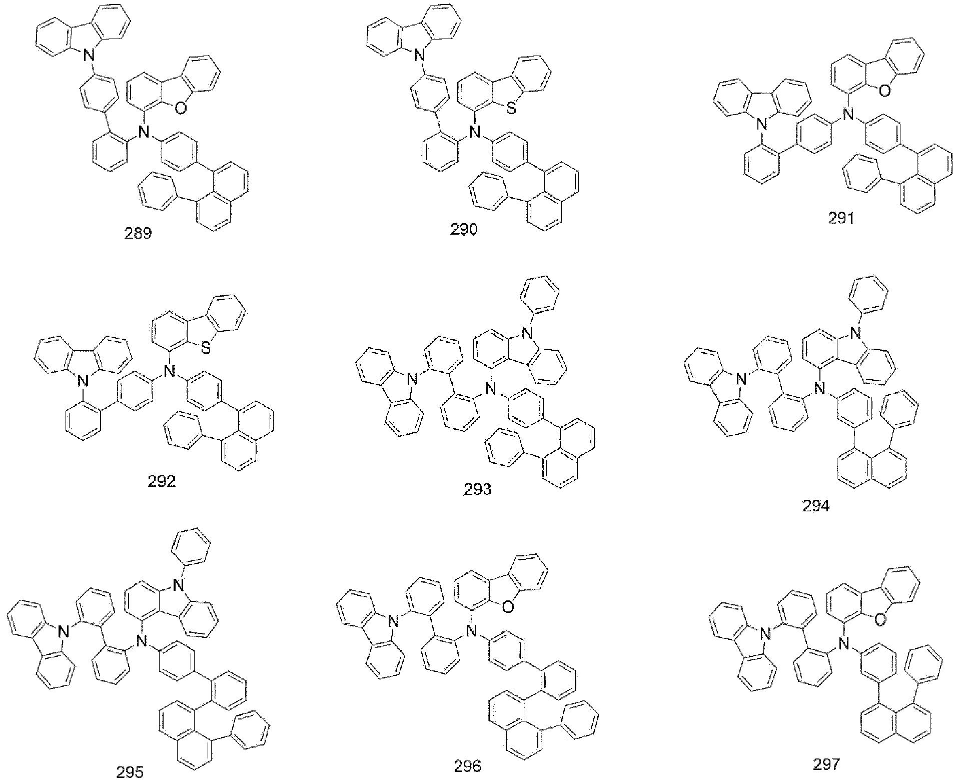

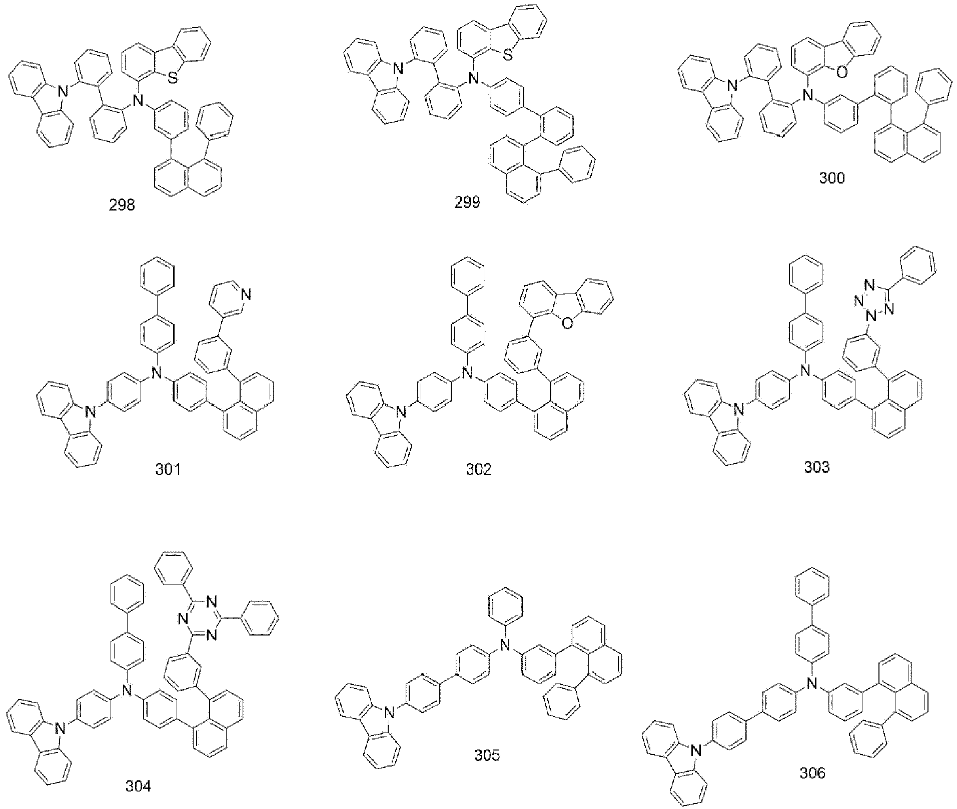

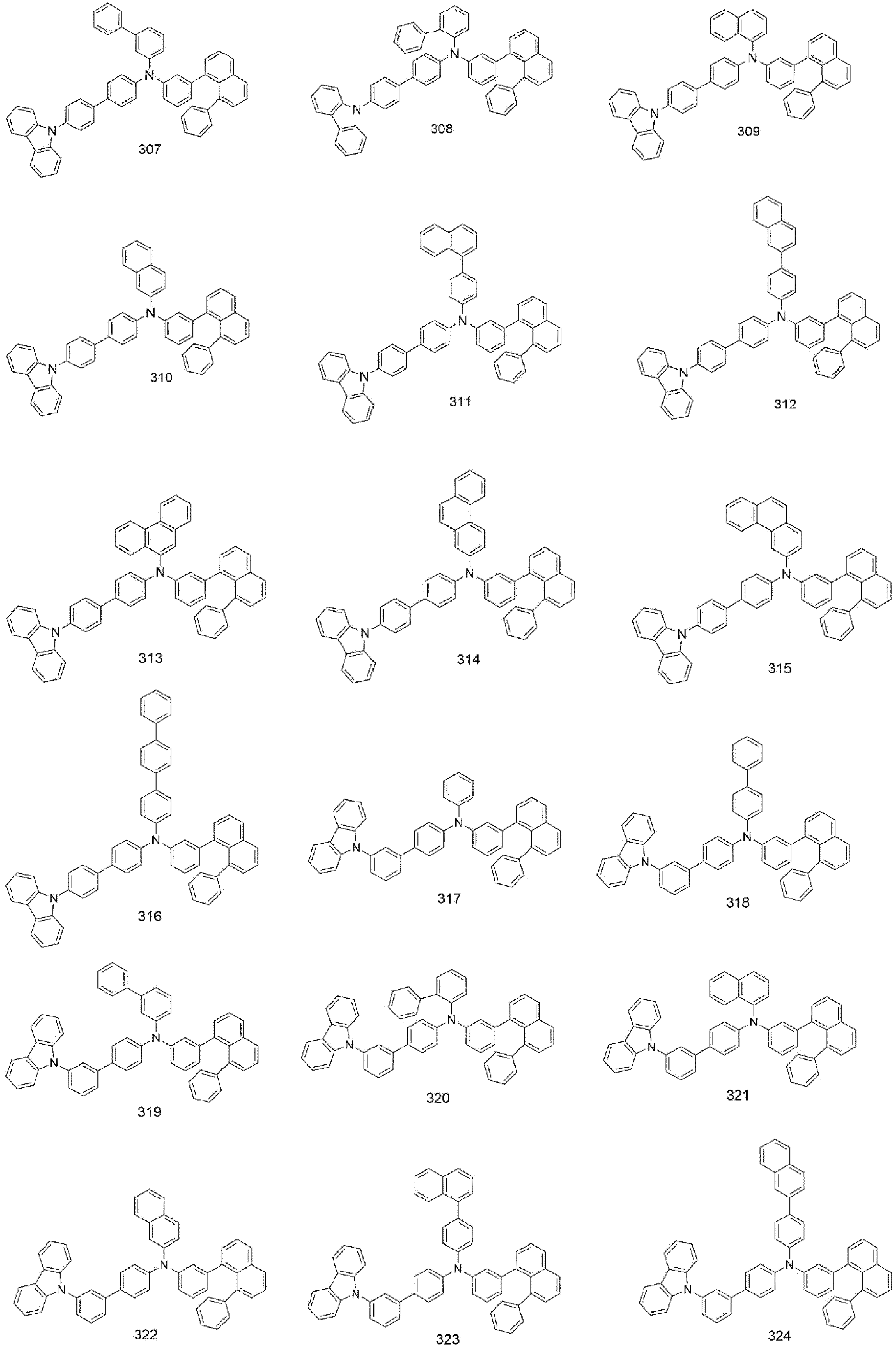

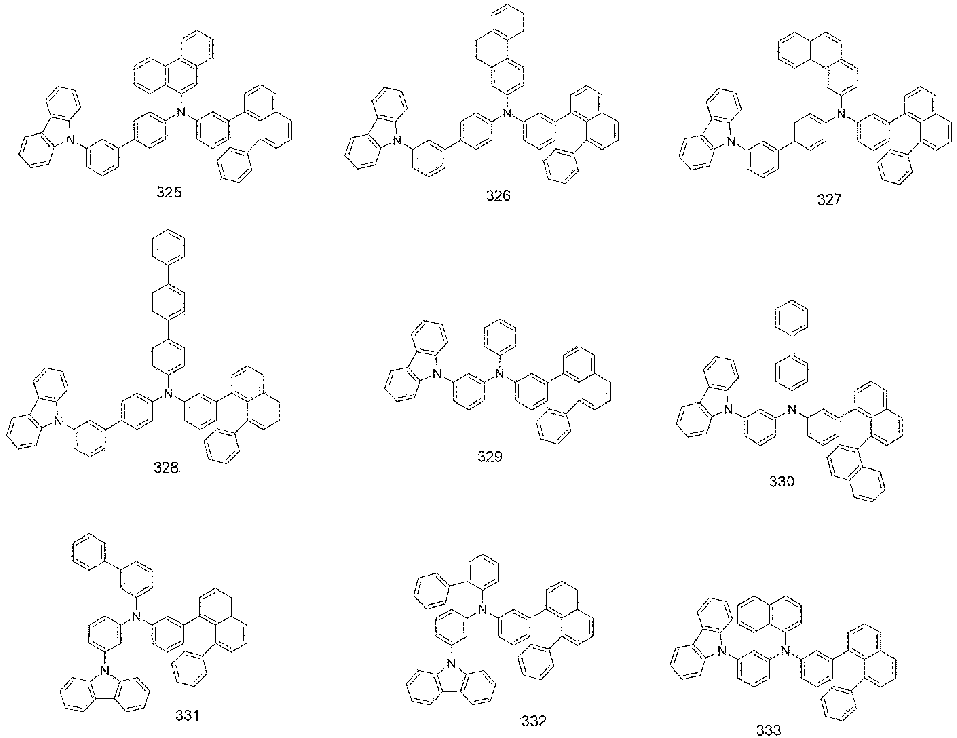

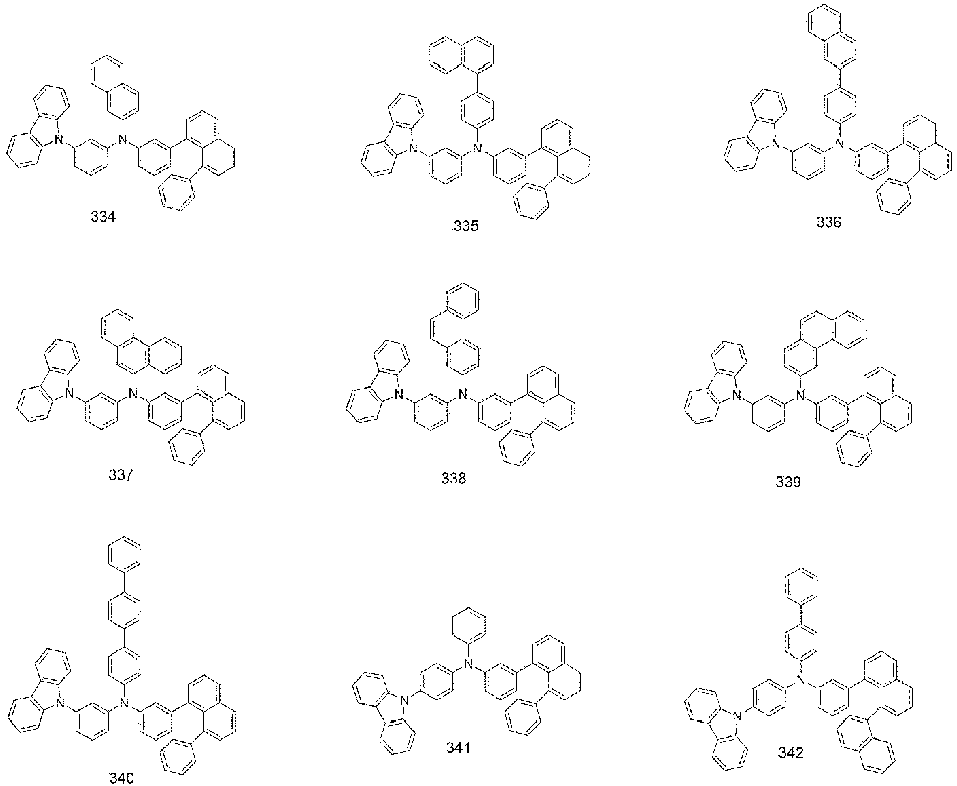

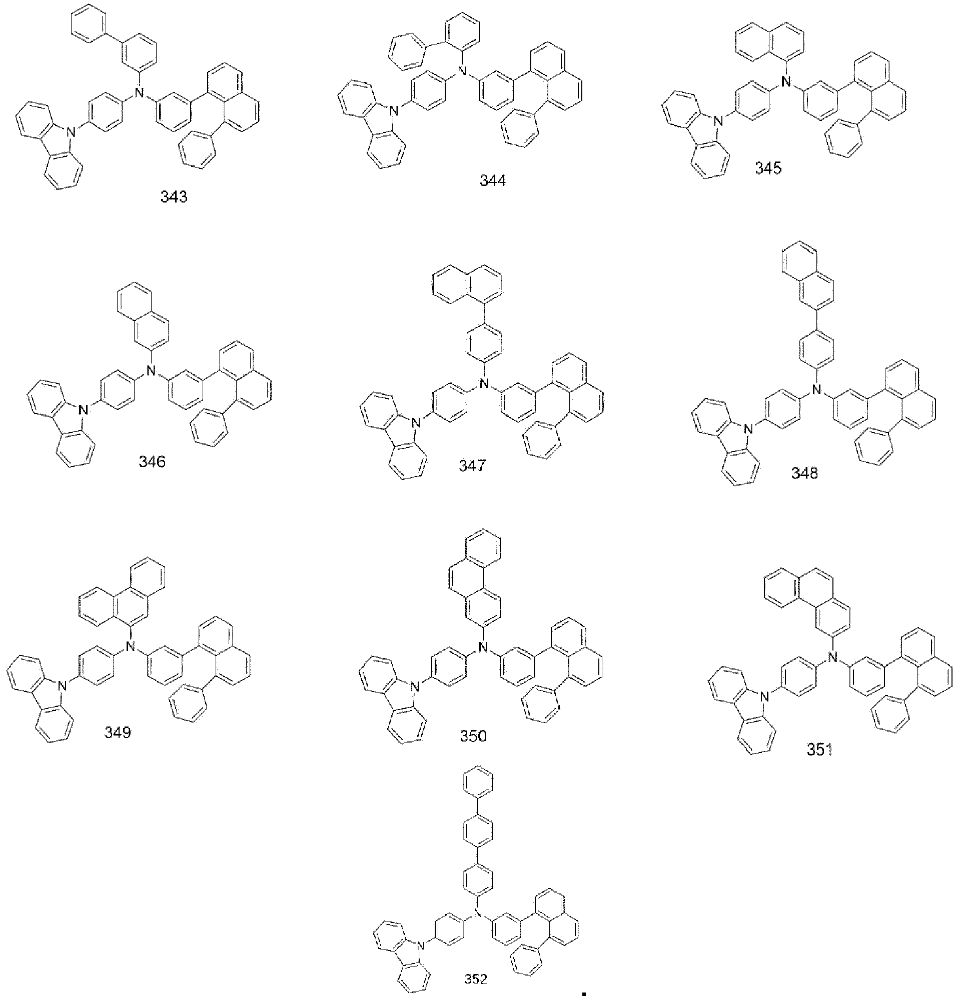

In one or more embodiments, the amine compound may be a tertiary amine compound. For example, in some embodiments, the amine compound may include a first substituent, a second substituent, and a third substituent.

The first substituent may be a substituent having a carbazole skeleton. For example, in one or more embodiments, the first substituent may be characterized in that the nitrogen atom at position 9 of carbazole is bonded to the amine nitrogen atom of the amine compound via a linker. In some embodiments, the amine compound may include the first substituent and may show high hole transport properties.

The second substituent may be a substituent having a naphthalene skeleton which is substituted with a substituted or unsubstituted phenyl group. For example, in one or more embodiments, the second substituent may be characterized in that the naphthalene skeleton is bonded to the amine nitrogen atom of the amine compound via a linker.

The third substituent may be an aryl group or a heteroaryl group. For example, in one or more embodiments, the third substituent may be an aryl group or a heteroaryl group directly bonded to the amine nitrogen atom of the amine compound.

The amine compound of the present disclosure may include the first to third substituents and may have improved electron tolerance, excellent or suitable hole transport properties and high stability. In some embodiments, the first to third substituents have excellent or suitable thermal stability and may suppress or reduce the deterioration of a material during a deposition process of the amine compound of the present disclosure. Accordingly, in one or more embodiments, the lifetime of a light emitting element including the amine compound of the present disclosure may be improved.

In one or more embodiments, in the light emitting element ED, the hole transport region HTR may further include a compound represented by Formula H-1.

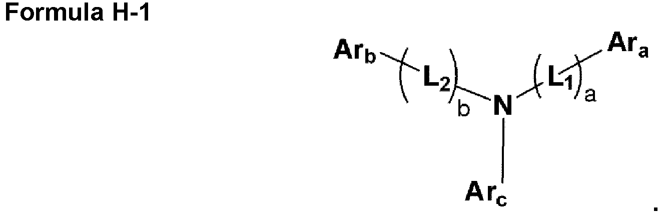

In Formula H-1, L₁ and L₂ may each independently be selected from a direct linkage, a substituted or unsubstituted arylene group of 6 to 30 ring-forming carbon atoms, and a substituted or unsubstituted heteroarylene group of 2 to 30 ring-forming carbon atoms. "a" and "b" may each independently be an integer of 0 to 10. In some embodiments, when "a" or "b" is an integer of 2 or more, a plurality of L_{1S} and L_{2S} may each independently be a substituted or unsubstituted arylene group of 6 to 30 ring-forming carbon atoms, or a substituted or unsubstituted heteroarylene group of 2 to 30 ring-forming carbon atoms.

In Formula H-1, Arₐ and Ar_{b} may each independently be a substituted or unsubstituted aryl group of 6 to 30 ring-forming carbon atoms, or a substituted or unsubstituted heteroaryl group of 2 to 30 ring-forming carbon atoms. In some embodiments, in Formula H-1, Ar_{c} may be a substituted or unsubstituted aryl group of 6 to 30 ring-forming carbon atoms.

The compound represented by Formula H-1 may be a monoamine compound. In some embodiments, the compound represented by Formula H-1 may be a diamine compound in which at least one selected from among Arₐ to Ar_{c} may include an amine group as a substituent. In some embodiments, the compound represented by Formula H-1 may be a carbazole-based compound in which at least one selected from among Arₐ and Ar_{b} may include a substituted or unsubstituted carbazole group, or a fluorene-based compound in which at least one among selected from Arₐ and Ar_{b} may include a substituted or unsubstituted fluorene group.

The compound represented by Formula H-1 may be represented by any one selected from among the compounds in Compound Group H. However, the compounds listed in Compound Group H are only illustrations, and the compound represented by Formula H-1 is not limited to the compounds represented in Compound Group H.

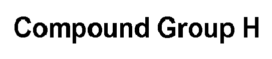

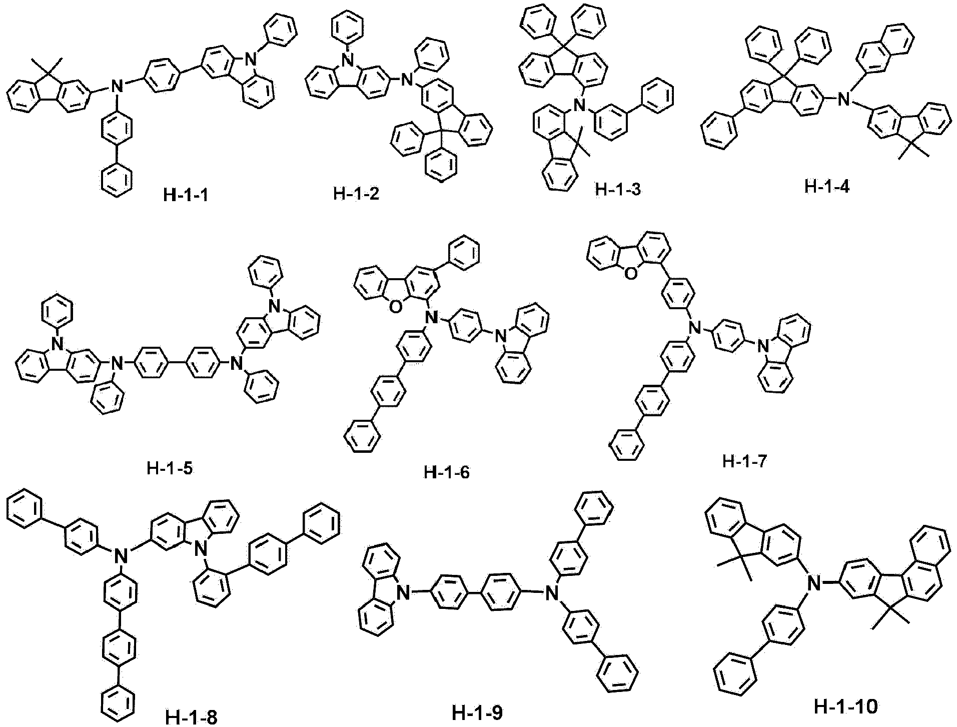

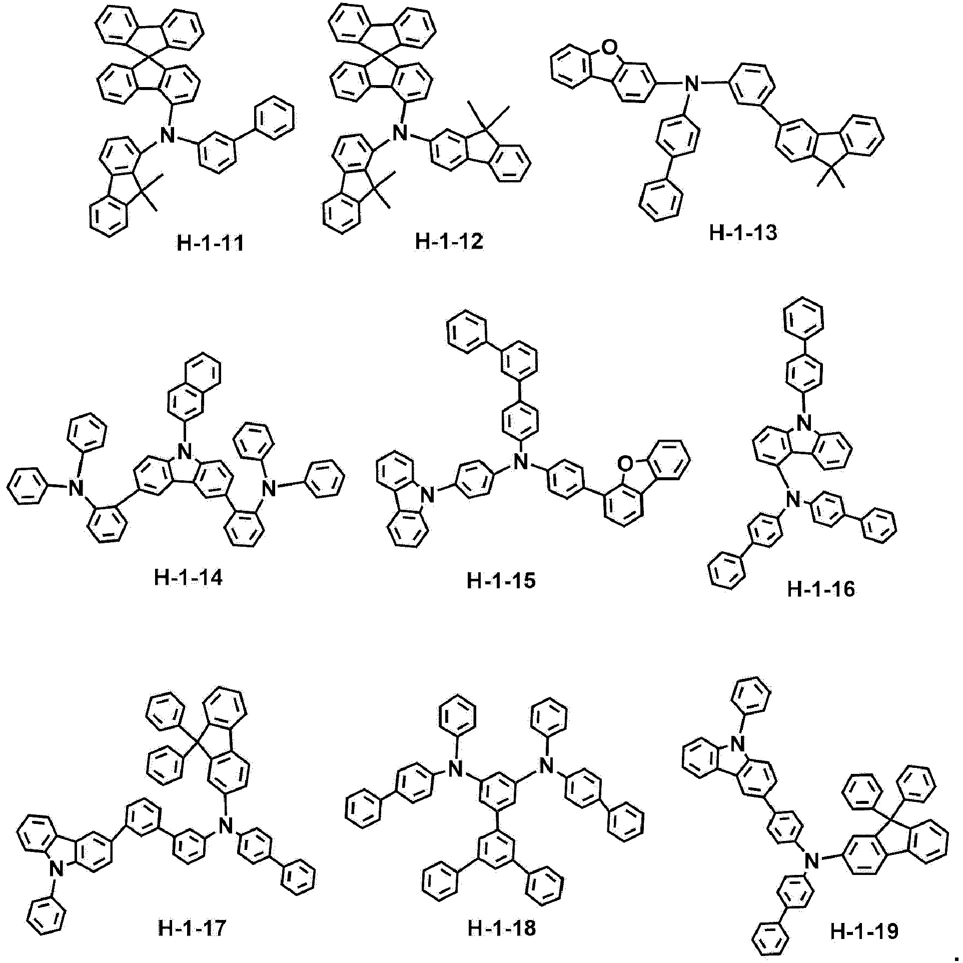

In addition, the hole transport region HTR may further include a suitable hole transport material.

For example, the hole transport region HTR may include a phthalocyanine compound such as copper phthalocyanine, N¹,N¹'-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4',4"-tris(N,N-diphenylamino)triphenylamine (TDATA), 4,4',4"-tris[N(1 -naphthyl)-N-phenylamino]-triphenylamine (1-TNATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(1-naphthalene-1-yl)-N,N'-diphenyl-benzidine (NPB or NPD), triphenylamine-containing polyetherketone (TPAPEK), 4-isopropyl-4'-methyldiphenyliodonium [tetrakis(pentafluorophenyl)borate], and/or dipyrazino[2,3-f:2',3'-h] quinoxaline-2,3,6,7,10,11-hexacarbonitrile (HAT-CN).

The hole transport region HTR may include carbazole derivatives such as N-phenyl carbazole and polyvinyl carbazole, fluorene-based derivatives, triphenylamine-based derivatives such as 4,4',4"-tris(N-carbazolyl)triphenylamine (TCTA), N,N'-bis(3-methylphenyl)-N,N'-diphenyl-[1,1'-biphenyl]-4,4'-diamine (TPD), N,N'-di(naphthalene-1-yl)-N,N'-diphenyl-benzidine (NPB), 4,4'-cyclohexylidene-bis[N,N-bis(4-methylphenyl)benzeneamine] (TAPC), 4,4'-bis[N,N'-(3-tolyl)amino]-3,3'-dimethylbiphenyl (HMTPD), 1,3-bis(N-carbazolyl)benzene (mCP), etc.

In some embodiments, the hole transport region HTR may include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the compounds of the hole transport region in at least one selected from among a hole injection layer HIL, hole transport layer HTL, and electron blocking layer EBL.

The thickness of the hole transport region HTR may be from about 100 Å to about 10,000 Å, for example, from about 100 Å to about 5,000 Å. When the hole transport region HTR includes a hole injection layer HIL, the thickness of the hole injection layer HIL may be, for example, from about 30 Å to about 1,000 Å.

When the hole transport region HTR includes a hole transport layer HTL, the thickness of the hole transport layer HTL may be from about 30 Å to about 1,000 Å. For example, when the hole transport region HTR includes an electron blocking layer EBL, the thickness of the electron blocking layer EBL may be from about 10 Å to about 1,000 Å. When the thicknesses of the hole transport region HTR, the hole injection layer HIL, the hole transport layer HTL, and the electron blocking layer EBL satisfy the above-described ranges, satisfactory hole transport properties may be achieved without substantial increase of a driving voltage.

The hole transport region HTR may further include a charge generating material to increase conductivity in addition to the above-described materials. The charge generating material may be dispersed uniformly or non-uniformly in the hole transport region HTR. The charge generating material may be, for example, a p-dopant. The p-dopant may include at least one selected from among metal halide compounds, quinone derivatives, metal oxides, and cyano group-containing compounds, without limitation. For example, the p-dopant may include metal halide compounds such as Cui and Rbl, quinone derivatives such as tetracyanoquinodimethane (TCNQ) and 2,3,5,6-tetrafluoro-7,7',8,8-tetracyanoquinodimethane (F4-TCNQ), metal oxides such as tungsten oxide and molybdenum oxide, cyano group-containing compounds such as dipyrazino[2,3-f: 2',3'-h] quinoxaline-2,3,6,7,10,11-hexacarbonitrile (HATCN) and 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), etc., without limitation.

As described above, the hole transport region HTR may further include a buffer layer in addition to the hole injection layer HIL, the hole transport layer HTL, and the electron blocking layer EBL. The buffer layer may compensate resonance distance according to the wavelength of light emitted from an emission layer EML and may increase emission efficiency. Materials included in the buffer layer may utilize materials which may be included in the hole transport region HTR.

The emission layer EML is provided on the hole transport region HTR. The emission layer EML may have a thickness of, for example, about 100 Å to about 1,000 Å or about 100 Å to about 300 Å. The emission layer EML may have a single layer formed utilizing a single material, a single layer formed utilizing a plurality of different materials, or a multi-layered structure having a plurality of layers formed utilizing a plurality of different materials.

In one or more embodiments, in the light emitting element ED, the emission layer EML may be to emit blue light. The light emitting element ED may include the amine compound of the present disclosure in a hole transport region HTR and may show high emission efficiency and long-life characteristics in a blue emission region. However, embodiments of the present disclosure are not limited thereto.

In one or more embodiments, in the light emitting element ED, the emission layer EML may include anthracene derivatives, pyrene derivatives, fluoranthene derivatives, chrysene derivatives, dihydrobenzanthracene derivatives, or triphenylene derivatives. For example, in some embodiments, the emission layer EML may include anthracene derivatives or pyrene derivatives.

In one or more embodiments, in the light emitting elements ED shown in FIG. 3 to FIG. 6, the emission layer EML may include a host and a dopant, and the emission layer EML may include a compound represented by Formula E-1. The compound represented by Formula E-1 may be utilized as a fluorescence host material.

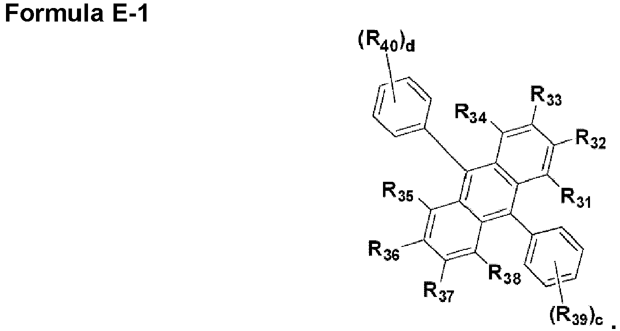

In Formula E-1, R₃₁ to R₄₀ may each independently be selected from a hydrogen atom, a deuterium atom, a halogen atom, a substituted or unsubstituted silyl group, a substituted or unsubstituted thio group, a substituted or unsubstituted oxy group, a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, and a substituted or unsubstituted heteroaryl group of 2 to 30 ring-forming carbon atoms, or may be combined with an adjacent group to form a ring. In some embodiments, R₃₁ to R₄₀ may be combined with an adjacent group to form a saturated hydrocarbon ring, an unsaturated hydrocarbon ring, a saturated heterocycle, or an unsaturated heterocycle.

In Formula E-1, "c" and "d" may each independently be an integer of 0 to 5.

Formula E-1 may be represented by any one selected from among Compound E1 to Compound E19.

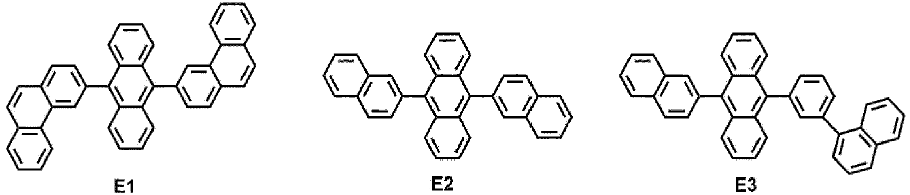

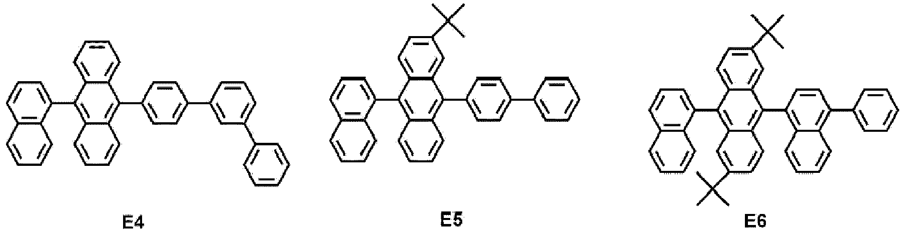

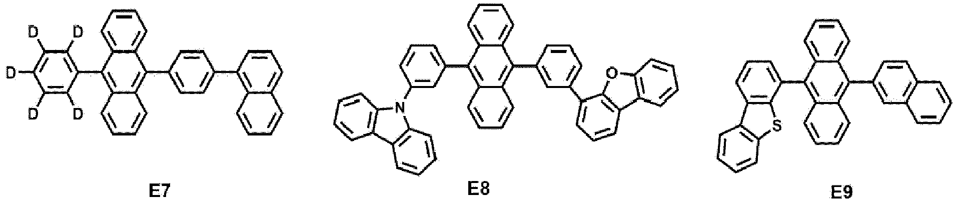

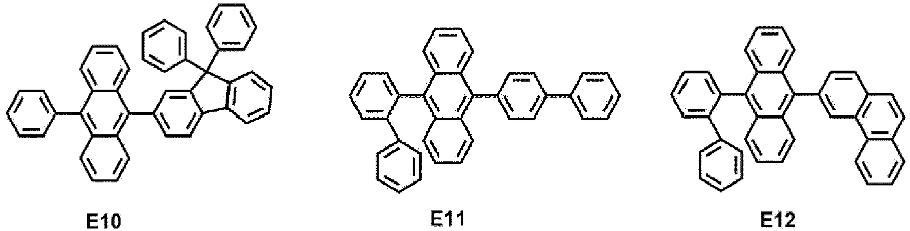

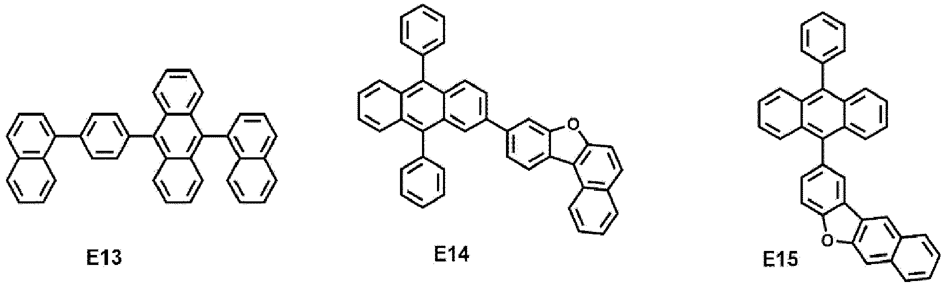

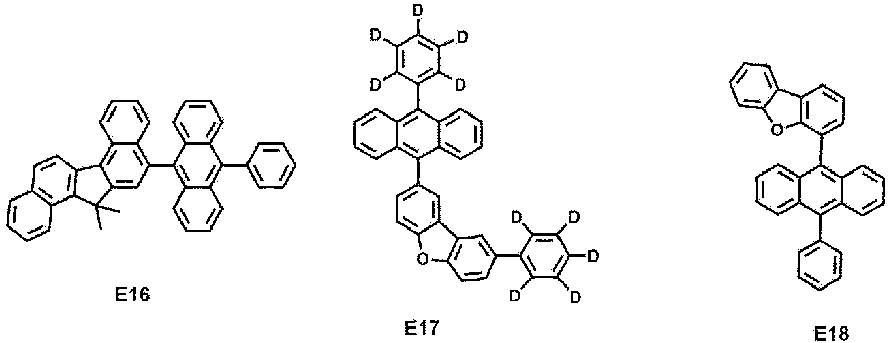

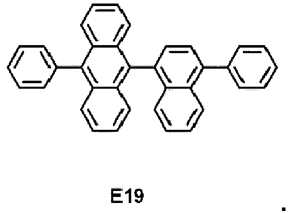

In one or more embodiments, the emission layer EML may include a compound represented by Formula E-2a or Formula E-2b. The compound represented by Formula E-2a or Formula E-2b may be utilized as a phosphorescence host material.

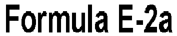

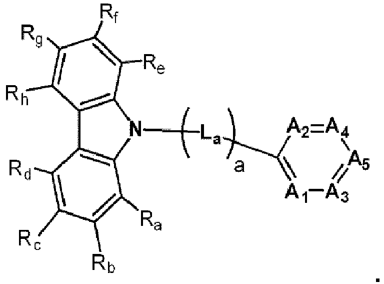

In Formula E-2a, "a" may be an integer of 0 to 10. Lₐ may be selected from a direct linkage, a substituted or unsubstituted arylene group of 6 to 30 ring-forming carbon atoms, and a substituted or unsubstituted heteroarylene group of 2 to 30 ring-forming carbon atoms. In some embodiments, when "a" is an integer of 2 or more, a plurality of Lₐs may each independently be selected from a substituted or unsubstituted arylene group of 6 to 30 ring-forming carbon atoms and a substituted or unsubstituted heteroarylene group of 2 to 30 ring-forming carbon atoms.

In some embodiments, in Formula E-2a, A₁ to A₅ may each independently be N or CRᵢ. Rₐ to Rᵢ may each independently be selected from a hydrogen atom, a deuterium atom, a substituted or unsubstituted amine group, a substituted or unsubstituted thio group, a substituted or unsubstituted oxy group, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, and a substituted or unsubstituted heteroaryl group of 2 to 30 ring-forming carbon atoms, and/or may be combined with an adjacent group to form a ring. Rₐ to Rᵢ may be combined with an adjacent group to form a hydrocarbon ring or a heterocycle including N, O, S, etc. as a ring-forming atom.

In some embodiments, in Formula E-2a, two or three selected from A₁ to A₅ may be N, and the remainder may be CRᵢ.

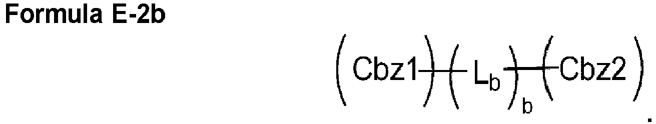

In Formula E-2b, Cbz1 and Cbz2 may each independently be selected from an unsubstituted carbazole group and a carbazole group substituted with an aryl group of 6 to 30 ring-forming carbon atoms. L_{b} may be selected from a direct linkage, a substituted or unsubstituted arylene group of 6 to 30 ring-forming carbon atoms, and a substituted or unsubstituted heteroarylene group of 2 to 30 ring-forming carbon atoms. "b" may be an integer of 0 to 10, and when "b" is an integer of 2 or more, a plurality of L_{b}s may each independently be selected from a substituted or unsubstituted arylene group of 6 to 30 ring-forming carbon atoms and a substituted or unsubstituted heteroarylene group of 2 to 30 ring-forming carbon atoms.

The compound represented by Formula E-2a or Formula E-2b may be represented by any one selected from among the compounds in Compound Group E-2. However, the compounds listed in Compound Group E-2 are only illustrations, and the compound represented by Formula E-2a or Formula E-2b is not limited to the compounds represented in Compound Group E-2.

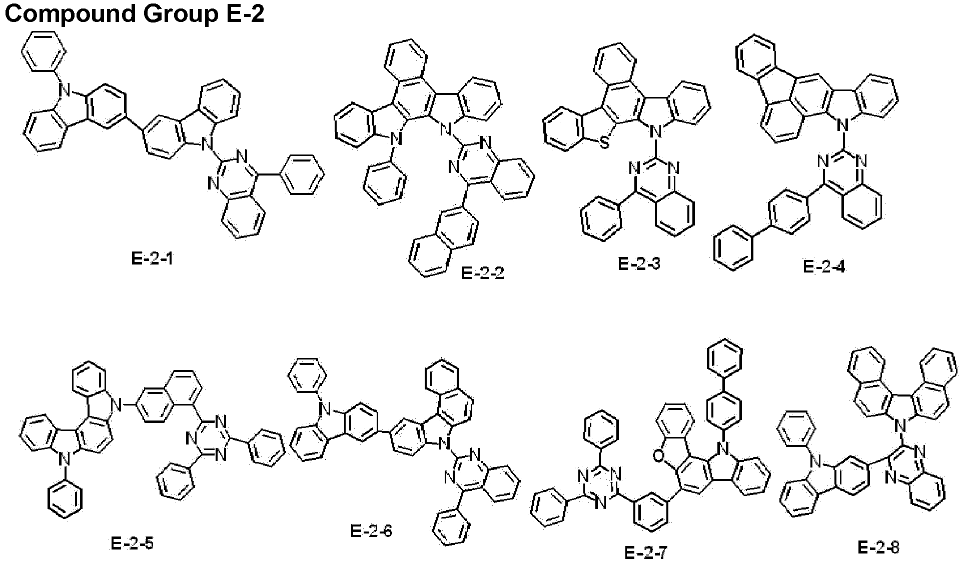

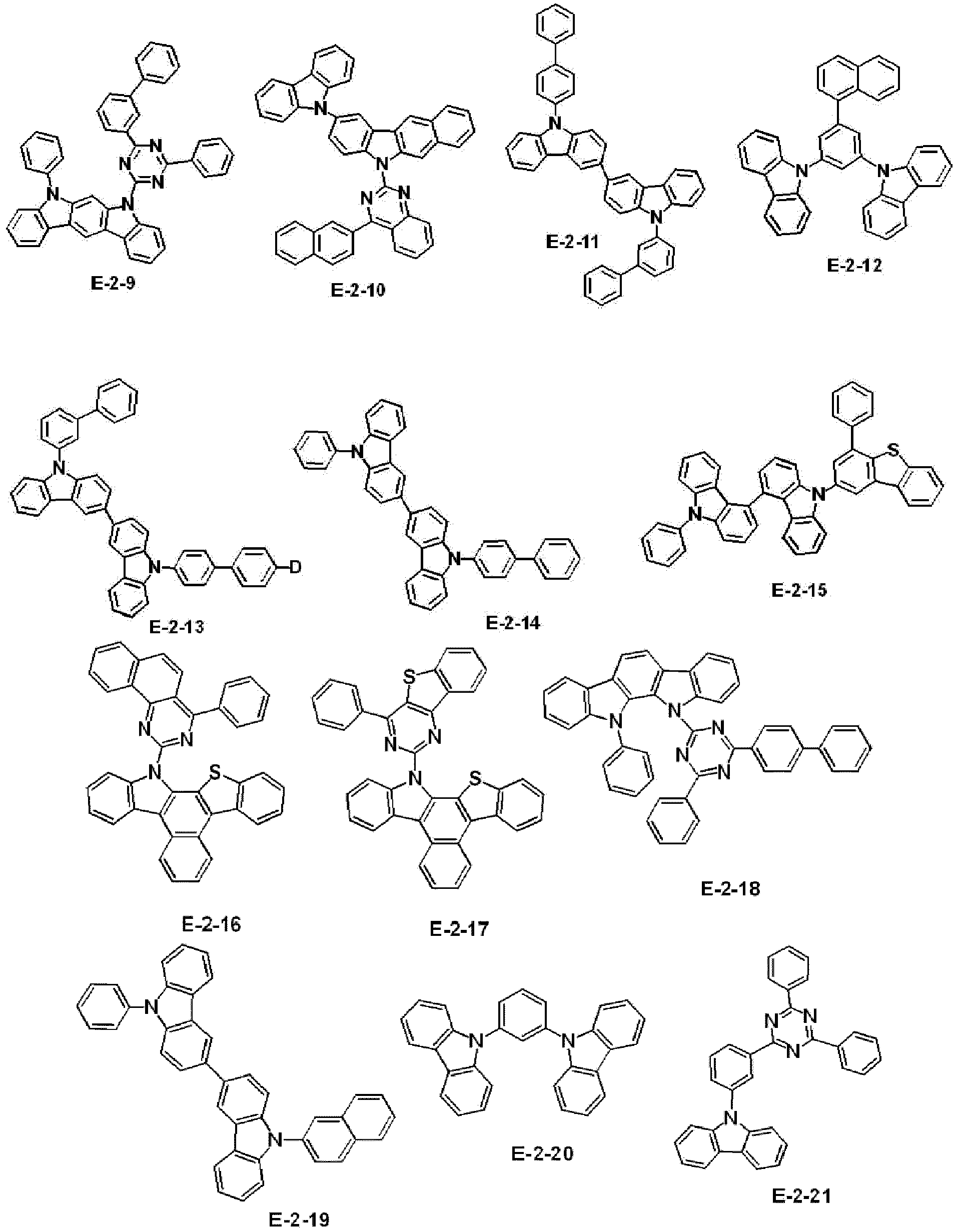

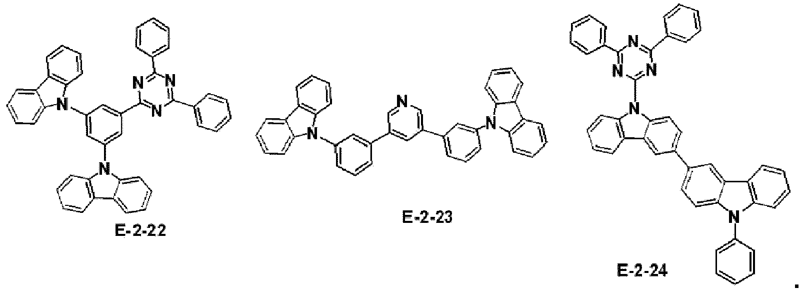

The emission layer EML may further include a material -suitable in the art as a host material. For example, the emission layer EML may include as a host material, at least one selected from among bis (4-(9H-carbazol-9-yl) phenyl) diphenylsilane (BCPDS), (4-(1-(4-(diphenylamino) phenyl) cyclohexyl) phenyl) diphenyl-phosphine oxide (POPCPA), bis[2-(diphenylphosphino)phenyl] ether oxide (DPEPO), 4,4'-bis(carbazol-9-yl)biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), and 1,3,5-tris(1-phenyl-1H-benzo[d]imidazole-2-yl)benzene (TPBi). However, embodiments of the present disclosure are not limited thereto. For example, tris(8-hydroxyquinolino)aluminum (Alqs), 9,10-di(naphthalene-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH₂), hexaphenylcyclotrisiloxane (DPSiOs), octaphenylcyclotetra siloxane (DPSiO₄), etc. may be utilized as the host material.

In one or more embodiments, the emission layer EML may include a compound represented by Formula M-a or Formula M-b. The compound represented by Formula M-a or Formula M-b may be utilized as a phosphorescence dopant material. In some embodiments, the compound represented by Formula M-a or Formula M-b may be utilized as an auxiliary dopant material.

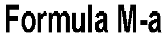

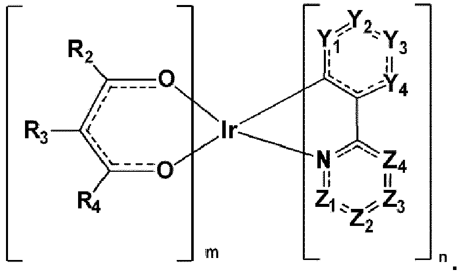

In Formula M-a, Y₁ to Y₄, and Z₁ to Z₄ may each independently be CR₁ or N, and R₁ to R₄ may each independently be selected from a hydrogen atom, a deuterium atom, a substituted or unsubstituted amine group, a substituted or unsubstituted thio group, a substituted or unsubstituted oxy group, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, and a substituted or unsubstituted heteroaryl group of 2 to 30 ring-forming carbon atoms, and/or may be combined with an adjacent group to form a ring. In Formula M-a, "m" is 0 or 1, and "n" is 2 or 3. In Formula M-a, when "m" is 0, "n" is 3, and when "m" is 1, "n" is 2.

The compound represented by Formula M-a may be utilized as a phosphorescence dopant.

The compound represented by Formula M-a may be represented by any one selected from among Compounds M-a1 to M-a25. However, Compounds M-a1 to Ma25 are illustrations, and the compound represented by Formula M-a is not limited to the compounds represented by Compounds M-a1 to M-a25.

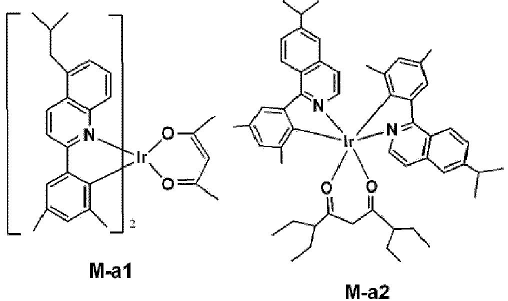

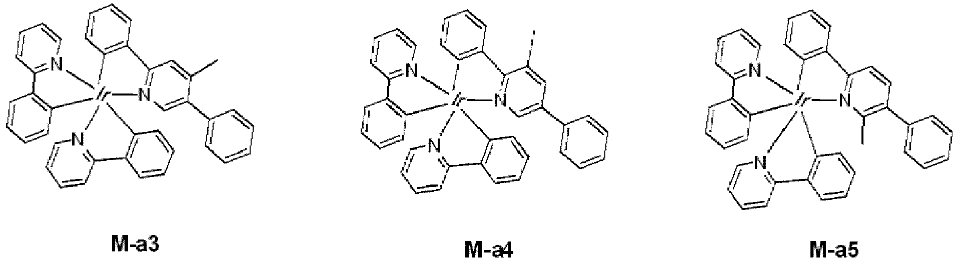

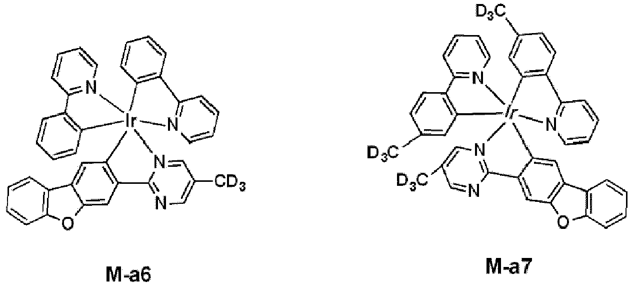

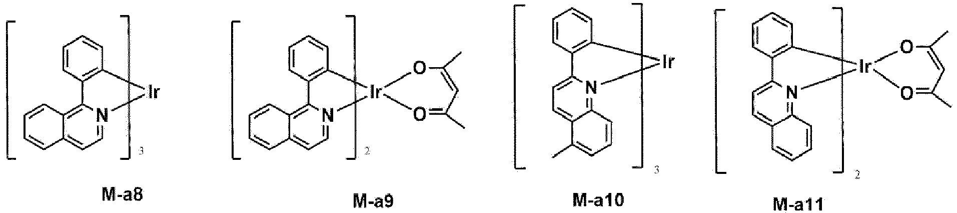

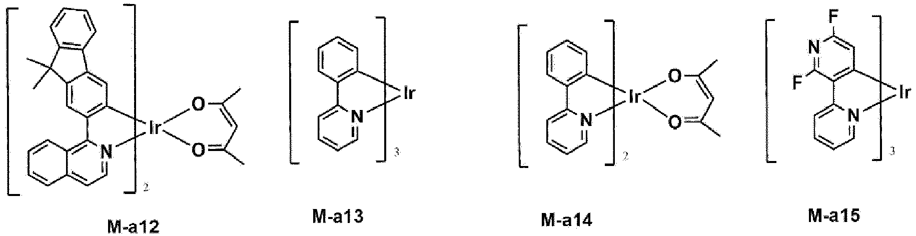

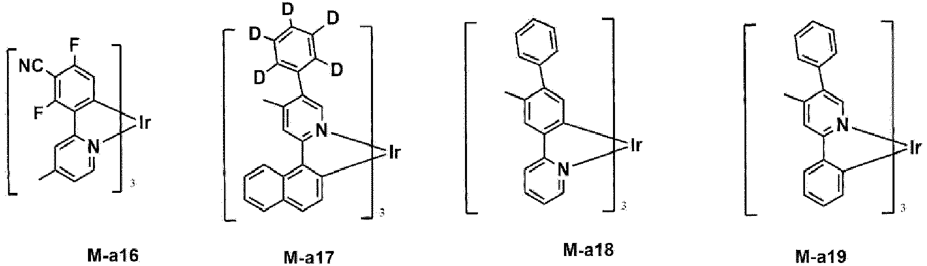

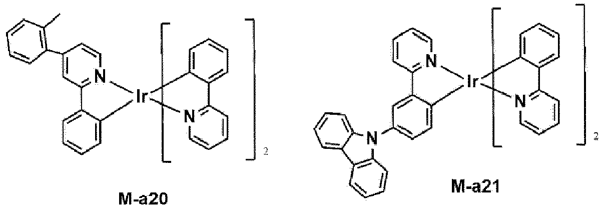

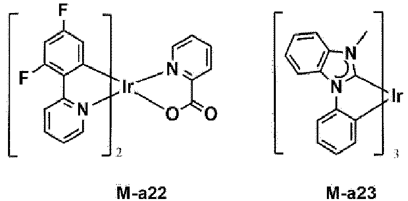

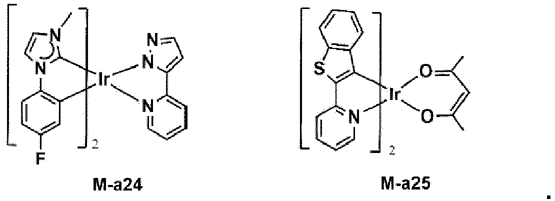

Compound M-a1 and Compound M-a2 may be utilized as red dopant materials, and Compound M-a3 to Compound M-a7 may be utilized as green dopant materials.

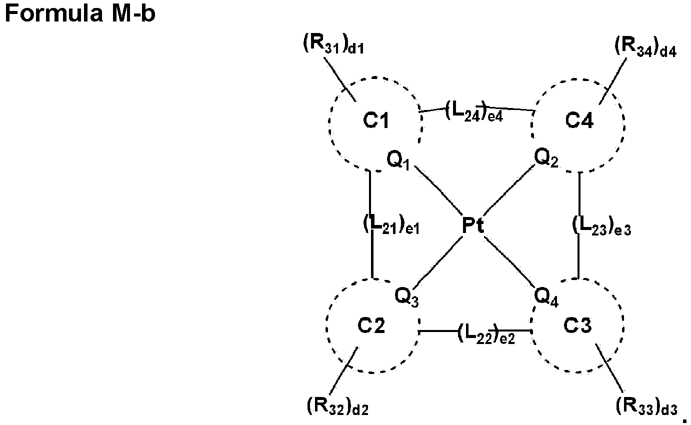

In Formula M-b, Q₁ to Q₄ may each independently be C or N, C1 to C4 may each independently be selected from a substituted or unsubstituted hydrocarbon ring of 5 to 30 ring-forming carbon atoms and a substituted or unsubstituted heterocycle of 2 to 30 ring-forming carbon atoms. L₂₁ to L₂₄ may each independently be selected from a direct linkage, ∗-O-∗ , ∗-S-∗ , 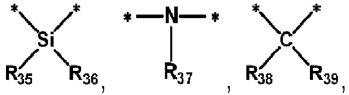 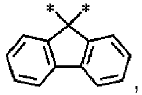 a substituted or unsubstituted divalent alkyl group of 1 to 20 carbon atoms, a substituted or unsubstituted arylene group of 6 to 30 ring-forming carbon atoms, and a substituted or unsubstituted heteroarylene group of 2 to 30 ring-forming carbon atoms, and e1 to e4 may each independently be 0 or 1. R₃₁ to R₃₉ may each independently be selected from a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, and a substituted or unsubstituted heteroaryl group of 2 to 30 ring-forming carbon atoms, and/or combined with an adjacent group to form a ring, and d1 to d4 may each independently be an integer of 0 to 4.

The compound represented by Formula M-b may be utilized as a blue phosphorescence dopant or a green phosphorescence dopant. In some embodiments, the compound represented by Formula M-b may be an auxiliary dopant and may be further included in the emission layer EML.

The compound represented by Formula M-b may be represented by any one selected from among Compound M-b-1 to Compound M-b-11. However, the compounds are illustrations, and the compound represented by Formula M-b is not limited to Compound M-b-1 to Compound M-b-11.

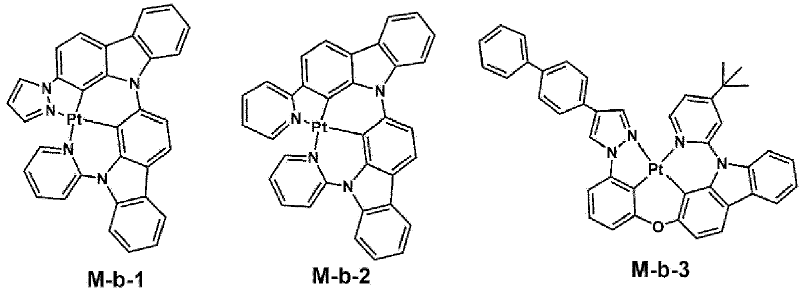

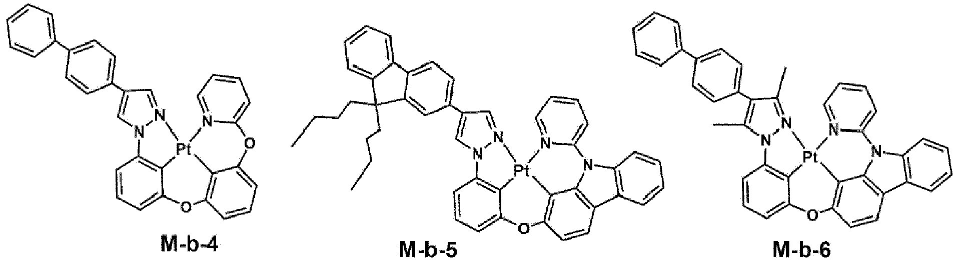

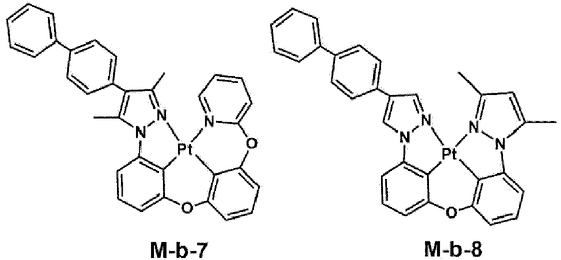

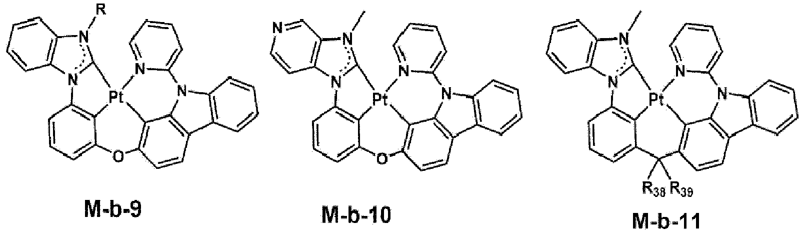

In the compounds above, R, R₃₈, and R₃₉ may each independently be selected from a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, and a substituted or unsubstituted heteroaryl group of 2 to 30 ring-forming carbon atoms.

In one or more embodiments, the emission layer EML may include any one selected from among Formula F-a to Formula F-c. The compounds represented by Formula F-a to Formula F-c may be utilized as fluorescence dopant materials.

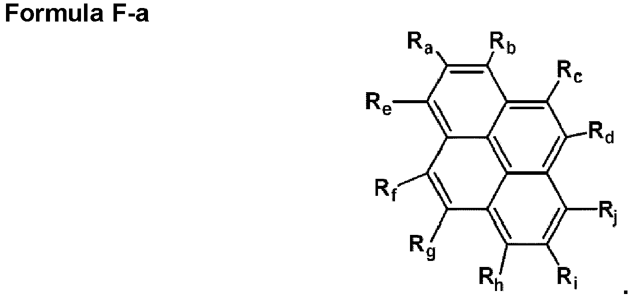

In Formula F-a, two selected from Rₐ to Rⱼ may each independently be substituted with ∗-NAr₁Ar₂. The remainder not substituted with ∗-NAr₁Ar₂ among Rₐ to Rⱼ may each independently be selected from a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, and a substituted or unsubstituted heteroaryl group of 2 to 30 ring-forming carbon atoms.

In ∗-NAr₁Ar₂, Ar₁ and Ar₂ may each independently be a substituted or unsubstituted aryl group of 6 to 30 ring-forming carbon atoms, or a substituted or unsubstituted heteroaryl group of 2 to 30 ring-forming carbon atoms. For example, at least one selected from among Ar₁ and Ar₂ may be a heteroaryl group including O or S as a ring-forming atom.

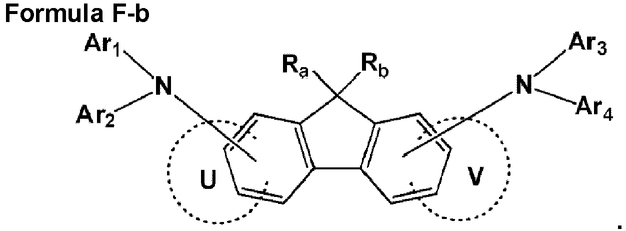

In Formula F-b, Rₐ and R_{b} may each independently be selected from a hydrogen atom, a deuterium atom, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, and a substituted or unsubstituted heteroaryl group of 2 to 30 ring-forming carbon atoms, or may be combined with an adjacent group to form a ring.

In Formula F-b, Ar₁ to Ar₄ may each independently be selected from a substituted or unsubstituted aryl group of 6 to 30 ring-forming carbon atoms and a substituted or unsubstituted heteroaryl group of 2 to 30 ring-forming carbon atoms.

In Formula F-b, U and V may each independently be selected from a substituted or unsubstituted hydrocarbon ring of 5 to 30 ring-forming carbon atoms and a substituted or unsubstituted heterocycle of 2 to 30 ring-forming carbon atoms.

In Formula F-b, the number of rings represented by U and V may each independently be 0 or 1. For example, in Formula F-b, when the number of U or V is 1, one ring forms a fused ring at the designated part by U or V, and when the number of U or V is 0, a ring is not present at the designated part by U or V. For example, when the number of U is 0, and the number of V is 1, or when the number of U is 1, and the number of V is 0, a fused ring having the fluorene core of Formula F-b may be a ring compound with four rings. In some embodiments, when the number of both U and V is 0, the fused ring having the fluorene core of Formula F-b may be a ring compound with three rings. In some embodiments, when the number of both U and V is 1, a fused ring having the fluorene core of Formula F-b may be a ring compound with five rings.

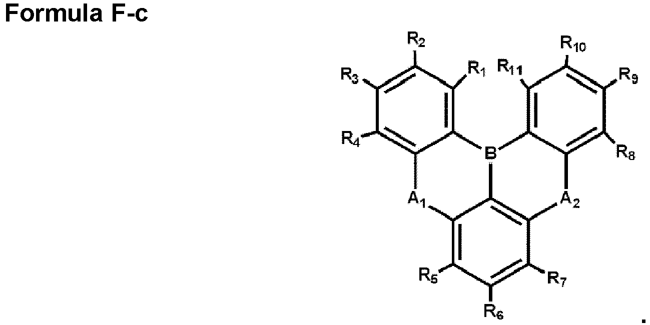

In Formula F-c, A₁ and A₂ may each independently be selected from O, S, Se, and NRₘ, and Rₘ may be selected from a hydrogen atom, a deuterium atom, a substituted or unsubstituted alkyl group of 1 to 20 carbon atoms, a substituted or unsubstituted aryl group of 6 to 30 ring-forming carbon atoms, and a substituted or unsubstituted heteroaryl group of 2 to 30 ring-forming carbon atoms. R₁ to R₁₁ may each independently be selected from a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group of 1 to 20 carbon atoms, a substituted or unsubstituted aryl group of 6 to 30 ring-forming carbon atoms, and a substituted or unsubstituted heteroaryl group of 2 to 30 ring-forming carbon atoms, and/or combined with an adjacent group to form a ring.

In Formula F-c, A₁ and A₂ may each independently be combined with the substituents of an adjacent ring to form a fused ring. For example, in some embodiments, when A₁ and A₂ may each independently be NRₘ, A₁ may be combined with R₄ or R₅ to form a ring. A₂ may be combined with R₇ or R₈ to form a ring.

In one or more embodiments, the emission layer EML may include as a suitable dopant material, styryl derivatives (for example, 1,4-bis[2-(3-N-ethylcarbazoryl)vinyl]benzene (BCzVB), 4-(di-p-tolylamino)-4'-[(di-p-tolylamino)styryl]stilbene (DPAVB), N-(4-((E)-2-(6-((E)-4-(diphenylamino)styryl)naphthalen-2-yl)vinyl)phenyl)-N-phenylbenzenamine (N-BDAVBi), and 4,4'-bis[2-(4-(N,N-diphenylamino)phenyl)vinyl]biphenyl (DPAVBi)), perylene and the derivatives thereof (for example, 2,5,8,11-tetra-t-butylperylene (TBP)), pyrene and the derivatives thereof (for example, 1,1-dipyrene, 1,4-dipyrenylbenzene, and/or 1,4-bis(N,N-diphenylamino)pyrene), etc.

In one or more embodiments, when a plurality of emission layers EML are included, at least one emission layer EML may include a suitable phosphorescence dopant material. For example, the phosphorescence dopant may utilize a metal complex including iridium (Ir), platinum (Pt), osmium (Os), gold (Au), titanium (Ti), zirconium (Zr), hafnium (Hf), europium (Eu), terbium (Tb) or thulium (Tm). For example, in some embodiments, iridium(III) bis(4,6-difluorophenylpyridinato-N,C2')picolinate (FIrpic), bis(2,4-difluorophenylpyridinato)-tetrakis(1 -pyrazolyl)borate iridium(III) (FIr6), and/or platinum octaethyl porphyrin (PtOEP) may be utilized as the phosphorescence dopant. However, embodiments of the present disclosure are not limited thereto.

In one or more embodiments, the emission layer EML may include a hole transport host and an electron transport host. In some embodiments, the emission layer EML may further include an auxiliary dopant and a light emitting dopant. In some embodiments, the auxiliary dopant may include a phosphorescence dopant material or a thermally activated delayed fluorescence dopant. For example, in one or more embodiments, the emission layer EML may include a hole transport host, an electron transport host, an auxiliary dopant, and a light emitting dopant.

In some embodiments, exciplex may be formed by the hole transport host and the electron transport host in the emission layer EML. In these embodiments, the triplet energy of the exciplex formed by the hole transport host and the electron transport host may correspond to T1 which is an energy gap between the LUMO energy level of the electron transport host and the HOMO energy level of the hole transport host.

In one or more embodiments, the triplet energy (T1) of the exciplex formed by the hole transport host and the electron transport host may be about 2.4 eV to about 3.0 eV. In some embodiments, the triplet energy of the exciplex may be a value smaller than the energy gap of each host material. Accordingly, the exciplex may have a triplet energy of about 3.0 eV or less, which is the energy gap between the hole transport host and the electron transport host.

In one or more embodiments, at least one emission layer EML may include a quantum dot material. The core of the quantum dot may be selected from II-VI group compounds, III-VI group compounds, I-III-VI group compounds, III-V group compounds, III-II-V group compounds, IV-VI group compounds, IV group elements, IV group compounds, and combinations thereof.

The II-VI group compound may be selected from the group consisting of: a binary compound selected from the group consisting of CdSe, CdTe, CdS, ZnS, ZnSe, ZnTe, ZnO, HgS, HgSe, HgTe, MgSe, MgS, and mixtures thereof; a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and mixtures thereof; and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, and mixtures thereof.

The III-VI group compound may include a binary compound such as In₂S₃, and In₂Se₃, a ternary compound such as InGaSs, and InGaSes, or optional combinations thereof.

The I-III-VI group compound may be selected from a ternary compound selected from the group consisting of AgInS, AgInS₂, CuInS, CuInS₂, AgGaS₂, CuGaS₂, CuGaO₂, AgGaO₂, AgAlO₂ and mixtures thereof, or a quaternary compound such as AgInGaS₂ and/or CuInGaS₂.

The III-V group compound may be selected from the group consisting of a binary compound selected from the group consisting of GaN, GaP, GaAs, GaSb, AlN, AlP, AlAs, AlSb, InN, InP, InAs, InSb, and mixtures thereof, a ternary compound selected from the group consisting of GaNP, GaNAs, GaNSb, GaPAs, GaPSb, AlNP, AlNAs, AlNSb, AlPAs, AlPSb, InGaP, InAlP, InNP, InNAs, InNSb, InPAs, InPSb, and mixtures thereof, and a quaternary compound selected from the group consisting of GaAlNP, GaAlNAs, GaAlNSb, GaAlPAs, GaAlPSb, GaInNP, GaInNAs, GaInNSb, GalnPAs, GalnPSb, InAlNP, InAlNAs, InAlNSb, InAlPAs, InAlPSb, and mixtures thereof. In some embodiments, the III-V group compound may further include a II group metal. For example, InZnP, etc. may be selected as a III-II-V group compound.

The IV-VI group compound may be selected from the group consisting of a binary compound selected from the group consisting of SnS, SnSe, SnTe, PbS, PbSe, PbTe, and mixtures thereof, a ternary compound selected from the group consisting of SnSeS, SnSeTe, SnSTe, PbSeS, PbSeTe, PbSTe, SnPbS, SnPbSe, SnPbTe, and mixtures thereof, and a quaternary compound selected from the group consisting of SnPbSSe, SnPbSeTe, SnPbSTe, and mixtures thereof. The IV group element may be selected from the group consisting of Si, Ge, and a mixture thereof. The IV group compound may be a binary compound selected from the group consisting of SiC, SiGe, and a mixture thereof.

In one or more embodiments, the binary compound, the ternary compound, or the quaternary compound may be present at substantially uniform concentration in a particle or may be present at a partially different concentration distribution state in substantially the same particle. In some embodiments, a core/shell structure in which one quantum dot wraps another quantum dot may be possible. The interface of the core and the shell may have a concentration gradient in which the concentration of an element present in the shell is decreased toward the center.

In some embodiments, the quantum dot may have the above-described core-shell structure including a core including a nanocrystal and a shell wrapping the core. The shell of the quantum dot may play the role of a protection layer for preventing or reducing the chemical deformation of the core to maintain semiconductor properties and/or a charging layer for imparting the quantum dot with electrophoretic properties. The shell may have a single layer or a multilayer. Examples of the shell of the quantum dot may include a metal or non-metal oxide, a semiconductor compound, or one or more combinations thereof.

For example, the metal or non-metal oxide suitable as a shell may include a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄ and NiO, or a ternary compound such as MgAl₂O₄, CoFe₂O₄, NiFe₂O₄ and CoMn₂O₄, but embodiments of the present disclosure are not limited thereto.

Also, the semiconductor compound suitable as a shell may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, etc., but embodiments of the present disclosure are not limited thereto.

The quantum dot may have a full width of half maximum (FWHM) of emission wavelength spectrum of about 45 nm or less, , about 40 nm or less, or about 30 nm or less. Within this range, color purity or color reproducibility may be improved. In some embodiments, light emitted via such quantum dot is emitted in all directions, and light view angle properties may be improved.

In addition, the shape of the quantum dot may be generally utilized shapes in the art, without specific limitation. In one or more embodiments, the shape of spherical, pyramidal, multi-arm, or cubic nanoparticle, nanotube, nanowire, nanofiber, nanoplate particle, etc. may be utilized.

The quantum dot may control the color of light emitted according to the particle size, and accordingly, the quantum dot may have one or more suitable emission colors such as blue, red, and green.

In one or more embodiments, in the light emitting elements ED, as shown in FIG. 3 to FIG. 6, the electron transport region ETR may be provided on the emission layer EML. The electron transport region ETR may include at least one selected from among a hole blocking layer HBL, an electron transport layer ETL, and an electron injection layer EIL. However, embodiments of the present disclosure are not limited thereto.

The electron transport region ETR may have a single layer formed utilizing a single material, a single layer formed utilizing a plurality of different materials, or a multi-layered structure having a plurality of layers formed utilizing a plurality of different materials.

For example, the electron transport region ETR may have a single layer structure of an electron injection layer EIL or an electron transport layer ETL, or a single layer structure formed utilizing an electron injection material and an electron transport material. Further, the electron transport region ETR may have a single layer structure formed utilizing a plurality of different materials, or a structure stacked from the emission layer EML of electron transport layer ETL/electron injection layer EIL, hole blocking layer HBL/electron transport layer ETL/electron injection layer EIL, electron transport layer ETL/buffer layer /electron injection layer EIL, and/or the like, without limitation. The thickness of the electron transport region ETR may be, for example, from about 1,000 Å to about 1,500 Å.

The electron transport region ETR may be formed utilizing one or more suitable methods such as a vacuum deposition method, a spin coating method, a casting method, a Langmuir-Blodgett (LB) method, an inkjet printing method, a laser printing method, and/or a laser induced thermal imaging (LITI) method.

The electron transport region ETR may include a compound represented by Formula ET-1.

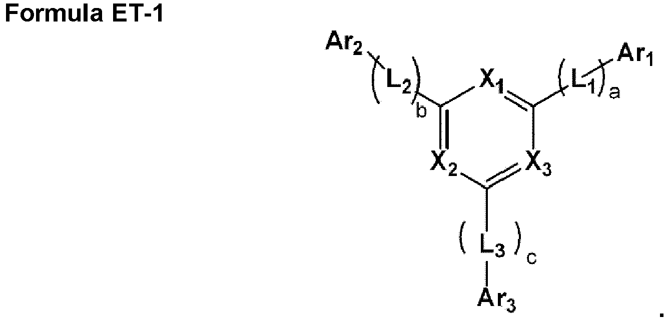

In Formula ET-1, at least one selected from among X₁ to X₃ is N, and the remainder are CRₐ. Rₐ may be selected from a hydrogen atom, a deuterium atom, a substituted or unsubstituted alkyl of 1 to 20 carbon atoms, a substituted or unsubstituted aryl group of 6 to 30 ring-forming carbon atoms, and a substituted or unsubstituted heteroaryl group of 2 to 30 ring-forming carbon atoms. Ar₁ to Ar₃ may each independently be selected from a hydrogen atom, a deuterium atom, a substituted or unsubstituted alkyl group of 1 to 20 carbon atoms, a substituted or unsubstituted aryl group of 6 to 30 ring-forming carbon atoms, and a substituted or unsubstituted heteroaryl group of 2 to 30 ring-forming carbon atoms.

In Formula ET-1, "a" to "c" may each independently be an integer of 0 to 10. In Formula ET-1, L₁ to L₃ may each independently be selected from a direct linkage, a substituted or unsubstituted arylene group of 6 to 30 ring-forming carbon atoms, and a substituted or unsubstituted heteroarylene group of 2 to 30 ring-forming carbon atoms. In some embodiments, when "a" to "c" are integers of 2 or more, L₁ to L₃ may each independently be selected from a substituted or unsubstituted arylene group of 6 to 30 ring-forming carbon atoms and a substituted or unsubstituted heteroarylene group of 2 to 30 ring-forming carbon atoms.

In one or more embodiments, the electron transport region ETR may include an anthracene-based compound. However, embodiment of the present disclosure are not limited thereto, and the electron transport region ETR may include, for example, tris(8-hydroxyquinolinato)aluminum (Alqs), 1,3,5-tri[(3-pyridyl)-phen-3-yl]benzene, 2,4,6-tris(3'-(pyridin-3-yl)biphenyl-3-yl)-1 ,3,5-triazine, 2-(4-(N-phenylbenzoimidazolyl-1-ylphenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (^{t}Bu-PBD), bis(2-methyl-8-quinolinolato-N1,O8)-(1,1'-biphenyl-4-olato)aluminum (BAlq), berylliumbis(benzoquinolin-10-olate (Bebq₂), 9,10-di(naphthalene-2-yl)anthracene (ADN), 1,3-bis[3,5-di(pyridin-3-yl)phenyl]benzene (BmPyPhB), diphenyl(4-(triphenylsilyl)phenyl)phosphine oxide (TSPO1), and/or mixtures thereof, without limitation.

The electron transport region ETR may include at least one selected from among Compounds ET1 to ET36.

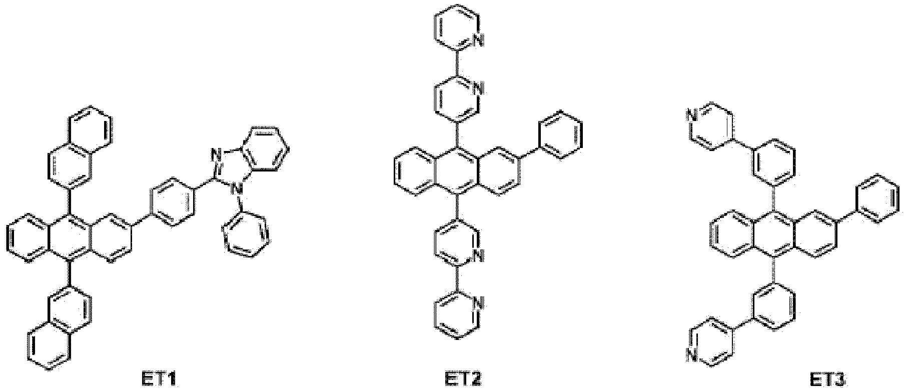

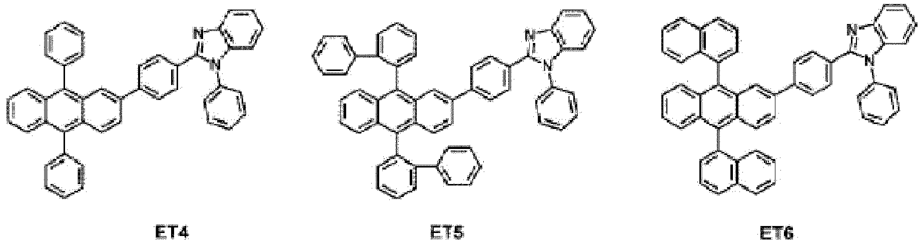

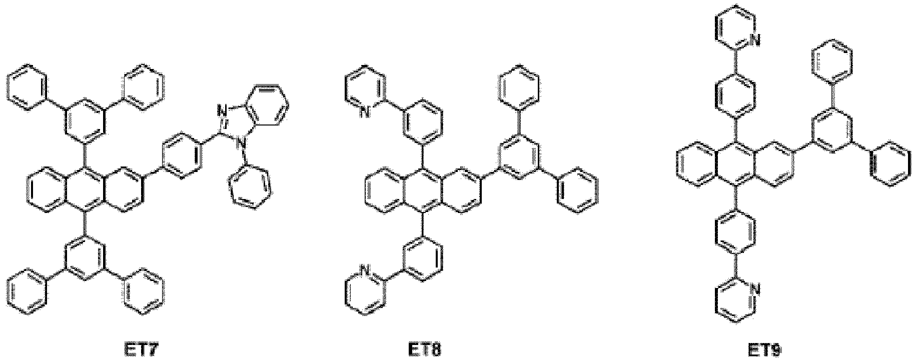

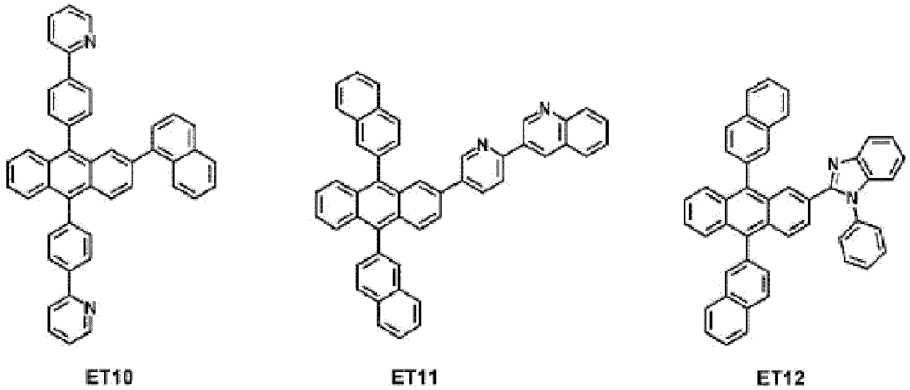

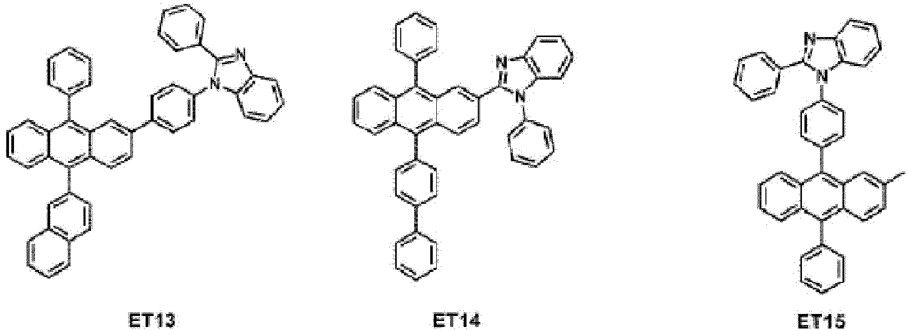

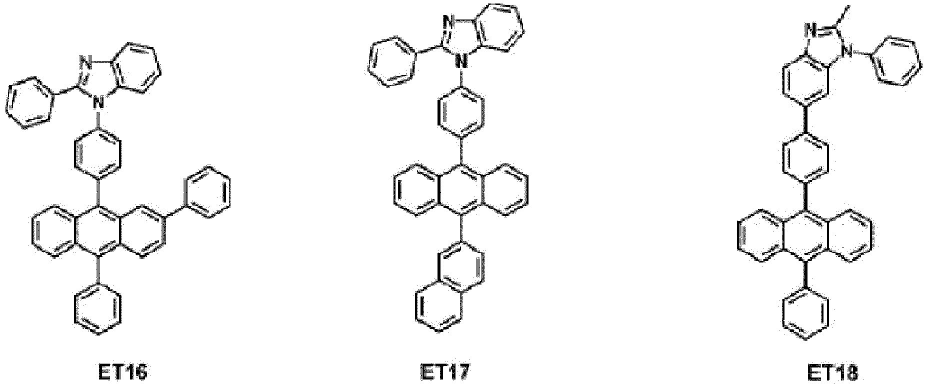

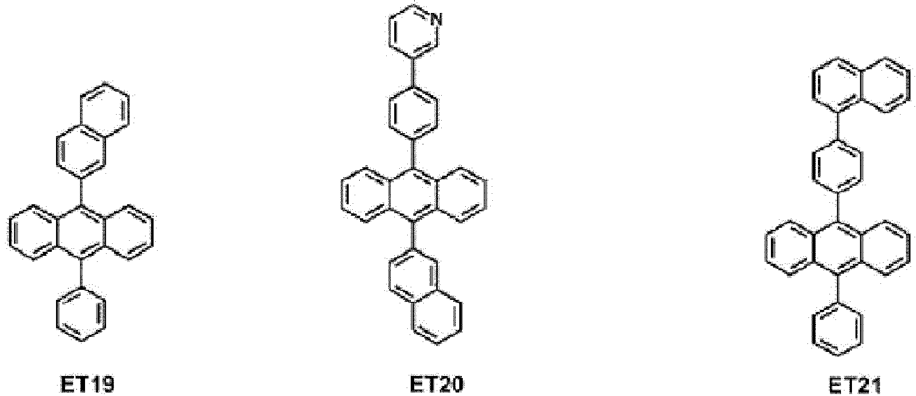

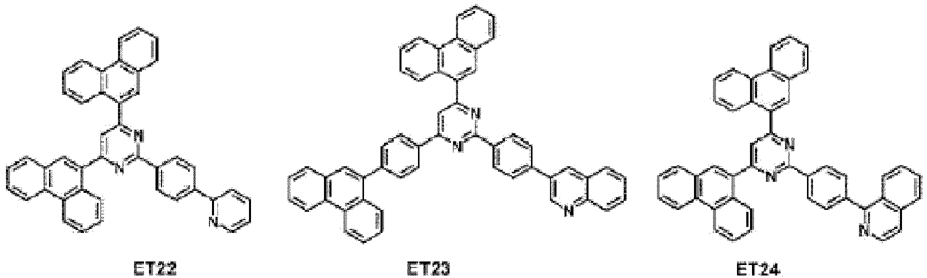

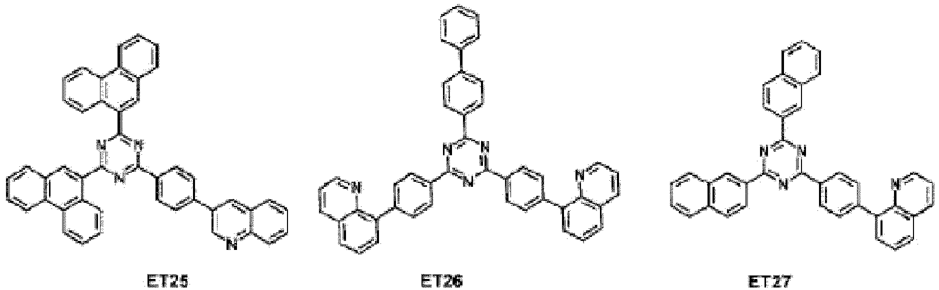

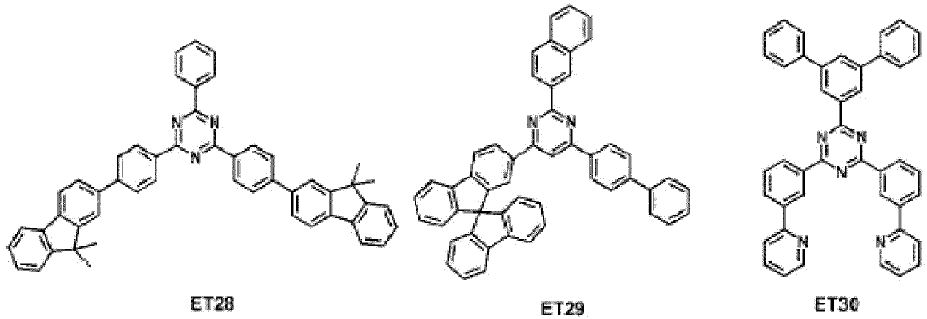

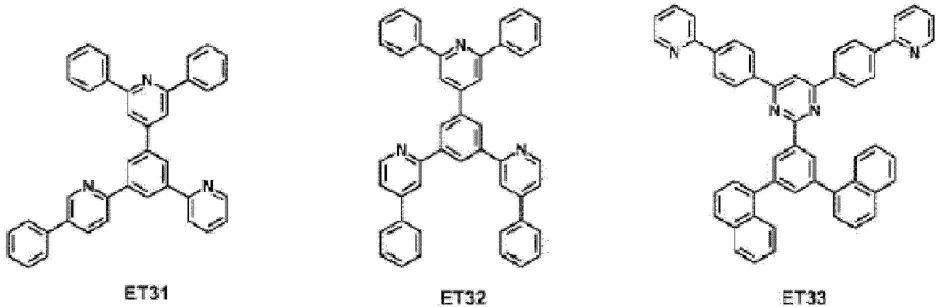

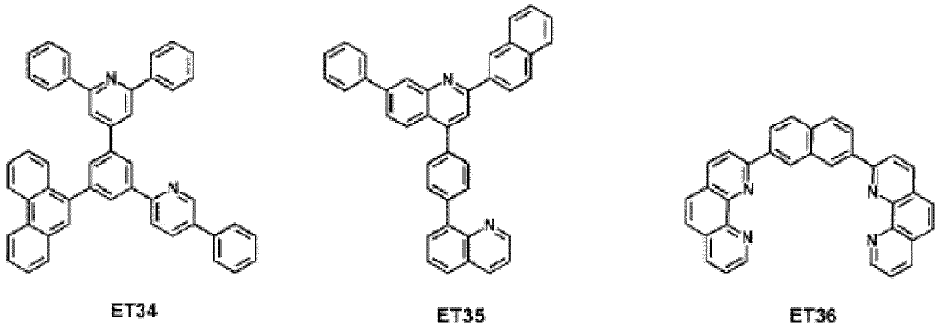

In some embodiments, the electron transport region ETR may include a metal halide such as LiF, NaCl, CsF, RbCl, Rbl, Cui and KI, a lanthanide metal such as Yb, or a co-depositing material of the metal halide and the lanthanide metal. For example, the electron transport region ETR may include KI:Yb, RbI:Yb, LiF:Yb, etc., as the co-depositing material. In some embodiments, the electron transport region ETR may utilize a metal oxide such as Li₂O and BaO, and/or 8-hydroxy-lithium quinolate (Liq). However, embodiments of the present disclosure are not limited thereto. In some embodiments, the electron transport region ETR also may be formed utilizing a mixture material of an electron transport material and an insulating organo metal salt. The organo metal salt may be a material having an energy band gap of about 4 eV or more. For example, the organo metal salt may include, for example, metal acetates, metal benzoates, metal acetoacetates, metal acetylacetonates, and/or metal stearates.

In some embodiments, the electron transport region ETR may include at least one selected from among 2,9-dimethyl-4,7-diphenyl-1,10-phenanthroline (BCP), diphenyl(4-(triphenylsilyl)phenyl)phosphine oxide (TSPO1) and 4,7-diphenyl-1,10-phenanthroline (Bphen) in addition to the aforementioned materials. However, embodiments of the present disclosure are not limited thereto.

The electron transport region ETR may include the compounds of the electron transport region in at least one selected from among an electron injection layer EIL, an electron transport layer ETL, and a hole blocking layer HBL.

When the electron transport region ETR includes the electron transport layer ETL, the thickness of the electron transport layer ETL may be from about 100 Å to about 1,000 Å, for example, from about 150 Å to about 500 Å. When the thickness of the electron transport layer ETL satisfies the above-described range, satisfactory electron transport properties may be obtained without substantial increase of a driving voltage. When the electron transport region ETR includes the electron injection layer EIL, the thickness of the electron injection layer EIL may be from about 1 Å to about 100 Å, for example, from about 3 Å to about 90 Å. When the thickness of the electron injection layer EIL satisfies the above-described range, satisfactory electron injection properties may be obtained without inducing substantial increase of a driving voltage.

The second electrode EL2 may be provided on the electron transport region ETR. The second electrode EL2 may be a common electrode. The second electrode EL2 may be a cathode or an anode, but embodiments of the present disclosure are not limited thereto. For example, when the first electrode EL1 is an anode, the second cathode EL2 may be a cathode, and when the first electrode EL1 is a cathode, the second electrode EL2 may be an anode. The second electrode EL2 may include at least one selected from among Ag, Mg, Cu, Al, Pt, Pd, Au, Ni, Nd, Ir, Cr, Li, Ca, LiF, Mo, Ti, W, In, Sn, and Zn, compounds of two or more selected therefrom, mixtures of two or more selected therefrom, and oxides thereof.

The second electrode EL2 may be a transmissive electrode, a transflective electrode, or a reflective electrode. When the second electrode EL2 is the transmissive electrode, the second electrode EL2 may include a transparent metal oxide, for example, ITO, IZO, ZnO, ITZO, etc.

When the second electrode EL2 is the transflective electrode or the reflective electrode, the second electrode EL2 may include Ag, Mg, Cu, Al, Pt, Pd, Au, Ni, Nd, Ir, Cr, Li, Ca, LiF/Ca (stacked structure of LiF and Ca), LiF/Al (stacked structure of LiF and Al), Mo, Ti, Yb (ytterbium), W, compounds including thereof, or mixtures thereof (for example, AgMg, AgYb, or MgYb). In some embodiments, the second electrode EL2 may have a multi-layered structure including a reflective layer or a transflective layer formed utilizing the above-described materials and a transparent conductive layer formed utilizing ITO, IZO, ZnO, ITZO, etc. For example, the second electrode EL2 may include the aforementioned metal materials, combinations of two or more metal materials selected from the aforementioned metal materials, or oxides of the aforementioned metal materials.

In some embodiments, the second electrode EL2 may be connected with an auxiliary electrode. When the second electrode EL2 is connected with the auxiliary electrode, the resistance of the second electrode EL2 may decrease.

In some embodiments, on the second electrode EL2 in the light emitting element ED, a capping layer CPL may be further disposed thereon. The capping layer CPL may include a multilayer or a single layer.

In one or more embodiments, the capping layer CPL may be an organic layer or an inorganic layer. For example, when the capping layer CPL includes an inorganic material, the inorganic material may include an alkali metal compound such as LiF, an alkaline earth metal compound such as SiON, SiNx, SiOy, etc.

For example, when the capping layer CPL includes an organic material, the organic material may include α-NPD, NPB, TPD, m-MTDATA, Alqs, CuPc, N4,N4,N4',N4'-tetra(biphenyl-4-yl) biphenyl-4,4'-diamine (TPD15), 4,4',4"-tris(carbazol-9-yl) triphenylamine (TCTA), etc., or includes an epoxy resin, or acrylate such as methacrylate. In some embodiments, a capping layer CPL may include at least one selected from among Compounds P1 to P5, but embodiments of the present disclosure are not limited thereto.

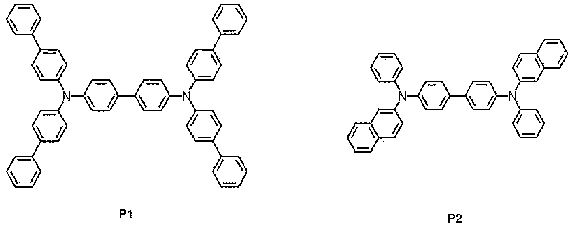

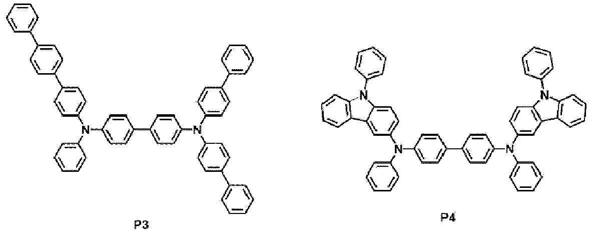

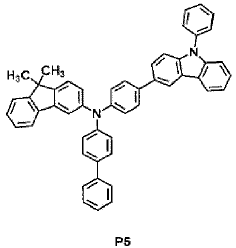

The refractive index of the capping layer CPL may be about 1.6 or more. Particularly, the refractive index of the capping layer CPL with respect to light in a wavelength range of about 550 nm to about 660 nm may be about 1.6 or more.

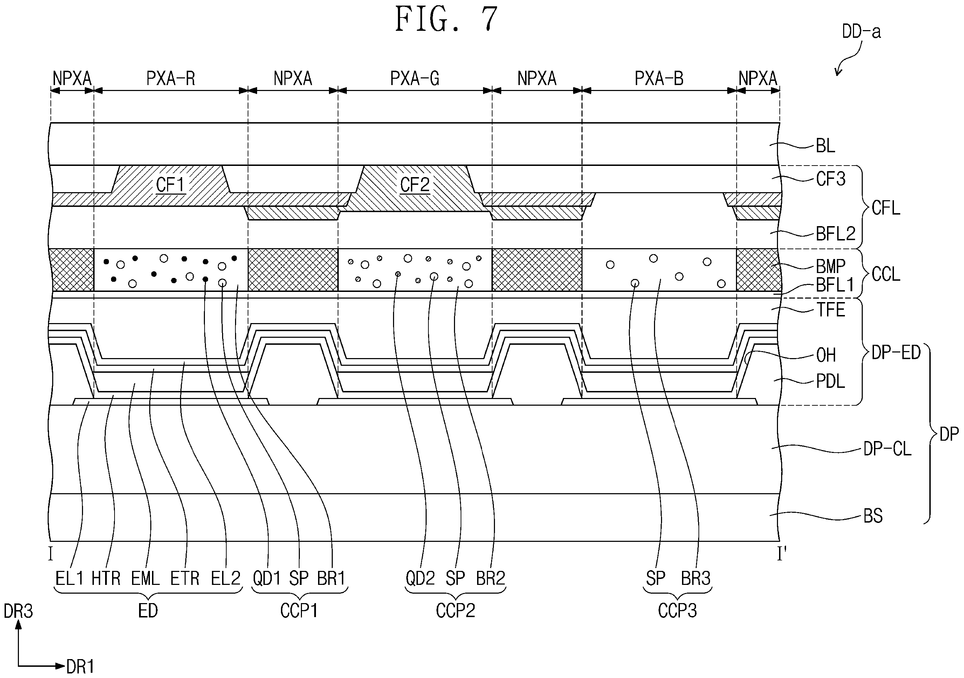

FIG. 7 to FIG. 10 are cross-sectional views on display devices according to embodiments. Hereinafter, in the explanation on the display devices of embodiments, referring to FIG. 7 to FIG. 10, the overlapping parts with the explanation on FIG. 1 to FIG. 6 will not be explained again, and the different features will be explained chiefly.

Referring to FIG. 7, a display device DD-a according to an embodiment may include a display panel DP including a display element layer DP-ED, a light controlling layer CCL disposed on the display panel DP, and a color filter layer CFL.

In an embodiment shown in FIG. 7, the display panel DP includes a base layer BS, a circuit layer DP-CL provided on the base layer BS and a display element layer DP-ED, and the display element layer DP-ED may include a light emitting element ED.

The light emitting element ED may include a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. The structures of the light emitting elements of FIG. 3 to FIG. 6 may be applied to the structure of the light emitting element ED shown in FIG. 7.

The hole transport region HTR of the light emitting element ED included in the display device DD-a according to an embodiment may include the amine compound of the present disclosure.

Referring to FIG. 7, the emission layer EML may be disposed in an opening portion OH defined in a pixel definition film PDL. For example, the emission layer EML divided by the pixel definition film PDL and correspondingly provided to each of luminous areas PXA-R, PXA-G and PXA-B may emit light in the same wavelength region. In the display device DD-a of an embodiment, the emission layer EML may emit blue light. In some embodiment of the present disclosure, different from the drawings, the emission layer EML may be provided as a common layer for all luminous areas PXA-R, PXA-G and PXA-B.

The light controlling layer CCL may be disposed on the display panel DP. The light controlling layer CCL may include a light converter. The light converter may be a quantum dot or a phosphor. The light converter may transform the wavelength of light provided and then emit. That is, the light controlling layer CCL may be a layer including a quantum dot or a layer including a phosphor.

The light controlling layer CCL may include multiple light controlling parts CCP1, CCP2 and CCP3. The light controlling parts CCP1, CCP2 and CCP3 may be separated from one another.

Referring to FIG. 7, a partition pattern BMP may be disposed between the separated light controlling parts CCP1, CCP2 and CCP3, but an embodiment of the inventive concept is not limited thereto. In FIG. 7, the partition pattern BMP is shown not to be overlapped with the light controlling parts CCP1, CCP2 and CCP3, but at least a portion of the edge of the light controlling parts CCP1, CCP2 and CCP3 may be overlapped with the partition pattern BMP.

The light controlling layer CCL may include a first light controlling part CCP1 including a first quantum dot QD1 converting first color light provided from the light emitting element ED into second color light, a second light controlling part CCP2 including a second quantum dot QD2 converting first color light into third color light, and a third light controlling part CCP3 transmitting first color light.

In an embodiment, the first light controlling part CCP1 may provide red light which is the second color light, and the second light controlling part CCP2 may provide green light which is the third color light. The third light controlling part CCP3 may transmit and provide blue light which is the first color light provided from the light emitting element ED. For example, the first quantum dot QD1 may be a red quantum dot, and the second quantum dot QD2 may be a green quantum dot. On the quantum dots QD1 and QD2, the same contents as those described above may be applied.

In addition, the light controlling layer CCL may further include a scatterer SP. The first light controlling part CCP1 may include the first quantum dot QD1 and the scatterer SP, the second light controlling part CCP2 may include the second quantum dot QD2 and the scatterer SP, and the third light controlling part CCP3 may not include a quantum dot but include the scatterer SP.

The scatterer SP may be an inorganic particle. For example, the scatterer SP may include at least one among TiO₂, ZnO, Al₂O₃, SiO₂, and hollow silica. The scatterer SP may include at least one among TiO₂, ZnO, Al₂O₃, SiO₂, and hollow silica, or may be a mixture of two or more materials selected among TiO₂, ZnO, Al₂O₃, SiO₂, and hollow silica.

Each of the first light controlling part CCP1, the second light controlling part CCP2, and the third light controlling part CCP3 may include base resins BR1, BR2 and BR3 dispersing the quantum dots QD1 and QD2 and the scatterer SP. In an embodiment, the first light controlling part CCP1 may include the first quantum dot QD1 and the scatterer SP dispersed in the first base resin BR1, the second light controlling part CCP2 may include the second quantum dot QD2 and the scatterer SP dispersed in the second base resin BR2, and the third light controlling part CCP3 may include the scatterer SP dispersed in the third base resin BR3. The base resins BR1, BR2 and BR3 are mediums in which the quantum dots QD1 and QD2 and the scatterer SP are dispersed, and may be composed of various resin compositions which may be generally referred to as a binder. For example, the base resins BR1, BR2 and BR3 may be acrylic resins, urethane-based resins, silicone-based resins, epoxy-based resins, etc. The base resins BR1, BR2 and BR3 may be transparent resins. In an embodiment, the first base resin BR1, the second base resin BR2 and the third base resin BR3 may be the same or different from each other.

The light controlling layer CCL may include a barrier layer BFL1. The barrier layer BFL1 may play the role of blocking the penetration of moisture and/or oxygen (hereinafter, will be referred to as "moisture/oxygen"). The barrier layer BFL1 may block the exposure of the light controlling parts CCP1, CCP2 and CCP3 to humidity/oxygen. In some embodiments of the present disclosure, the barrier layer BFL1 may cover the light controlling parts CCP1, CCP2 and CCP3. In addition, the barrier layer BFL2 may be provided between the light controlling parts CCP1, CCP2 and CCP3 and filters CF1, CF2 and CF3.

The barrier layers BFL1 and BFL2 may include at least one inorganic layer. That is, the barrier layers BFL1 and BFL2 may be formed by including an inorganic material. For example, the barrier layers BFL1 and BFL2 may be formed by including silicon nitride, aluminum nitride, zirconium nitride, titanium nitride, hafnium nitride, tantalum nitride, silicon oxide, aluminum oxide, titanium oxide, tin oxide, cerium oxide and silicon oxynitride or a metal thin film securing light transmittance. In some embodiments of the present disclosure, the barrier layers BFL1 and BFL2 may further include an organic layer. The barrier layers BFL1 and BFL2 may be composed of a single layer or multiple layers.

In the display device DD-a of an embodiment, the color filter layer CFL may be disposed on the light controlling layer CCL. For example, the color filter layer CFL may be disposed directly on the light controlling layer CCL. In this case, the barrier layer BFL2 may be omitted.

The color filter layer CFL may include filters CF1, CF2 and CF3. The color filter layer CFL may include a first filter CF1 transmitting second color light, a second filter CF2 transmitting third color light, and a third filter CF3 transmitting first color light. For example, the first filter CF1 may be a red filter, the second filter CF2 may be a green filter, and the third filter CF3 may be a blue filter. Each of the filters CF1, CF2 and CF3 may include a polymer photosensitive resin and a pigment or dye. The first filter CF1 may include a red pigment or dye, the second filter CF2 may include a green pigment or dye, and the third filter CF3 may include a blue pigment or dye. However, embodiments of the present disclosure are not limited thereto, and the third filter CF3 may not include the pigment or dye. The third filter CF3 may include a polymer photosensitive resin and not include a pigment or dye. The third filter CF3 may be transparent. The third filter CF3 may be formed using a transparent photosensitive resin.

In addition, in an embodiment, the first filter CF1 and the second filter CF2 may be yellow filters. The first filter CF1 and the second filter CF2 may be provided in one body without distinction. The first to third filters CF1, CF2 and CF3 may be disposed corresponding to the red luminous area PXA-R, the green luminous area PXA-G and the blue luminous area PXA-B, respectively.

Though not shown, the color filter layer CFL may further include a light blocking part (not shown). The light blocking part may be a black matrix. The light blocking part may be formed by including an organic light blocking material or an inorganic light blocking material, including a black pigment or black dye. The light blocking part may prevent light leakage and may divide the boundaries among adjacent filters CF1, CF2 and CF3.

On the color filter layer CFL, a base substrate BL may be disposed. The base substrate BL may be a member providing a base surface on which the color filter layer CFL, the light controlling layer CCL, etc. are disposed. The base substrate BL may be a glass substrate, a metal substrate, a plastic substrate, etc. However, embodiments of the present disclosure are not limited thereto, and the base substrate BL may be an inorganic layer, an organic layer or a composite material layer. In addition, different from the drawing, the base substrate BL may be omitted in an embodiment.

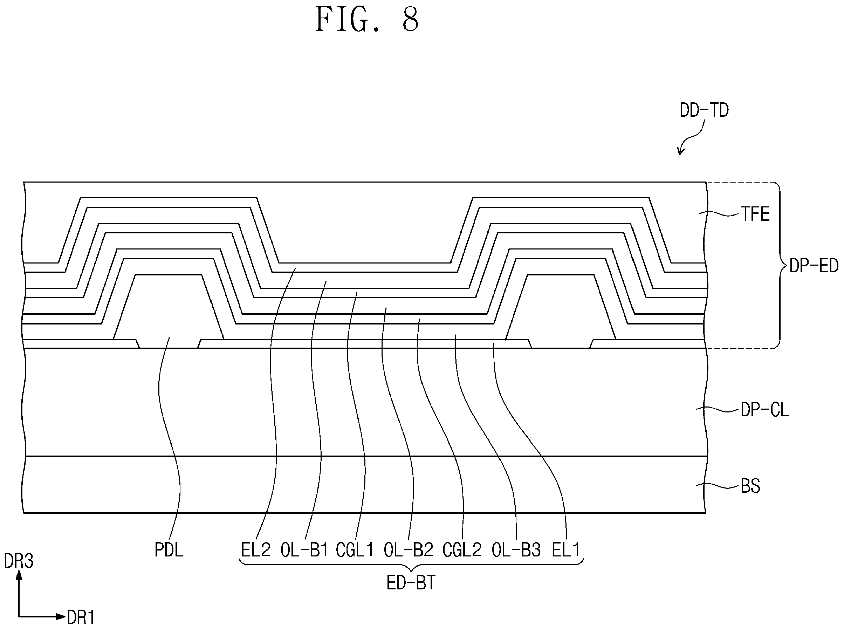

FIG. 8 is a cross-sectional view showing a portion of the display device according to an embodiment. FIG. 8 shows another embodiment of a portion corresponding to the display panel DP in FIG. 7. In a display device DD-TD of an embodiment, the light emitting element ED-BT may include multiple light emitting structures OL-B1, OL-B2 and OL-B3. The light emitting element ED-BT may include oppositely disposed first electrode EL1 and second electrode EL2, and the multiple light emitting structures OL-B1, OL-B2 and OL-B3 stacked in order in a thickness direction and provided between the first electrode EL1 and the second electrode EL2. Each of the light emitting structures OL-B1, OL-B2 and OL-B3 may include an emission layer EML (FIG. 7), and a hole transport region HTR and an electron transport region ETR (FIG. 7) disposed with the emission layer EML therebetween.

That is, the light emitting element ED-BT included in the display device DD-TD of an embodiment may be a light emitting element of a tandem structure including multiple emission layers.

In an embodiment shown in FIG. 8, light emitted from the light emitting structures OL-B1, OL-B2 and OL-B3 may be all blue light. However, an embodiment of the inventive concept is not limited thereto, and the wavelength regions of light emitted from the light emitting structures OL-B1, OL-B2 and OL-B3 may be different from each other. For example, the light emitting element ED-BT including the multiple light emitting structures OL-B1, OL-B2 and OL-B3 emitting light in different wavelength regions may emit white light (e.g., a combined white light).

Between neighboring light emitting structures OL-B1, OL-B2 and OL-B3, charge generating layers CGL1 and CGL2 may be disposed. The charge generating layers CGL1 and CGL2 may include a p-type or kind charge generating layer and/or an n-type or kind charge generating layer.

In one or more embodiments, in at least one selected from among the light emitting structures OL-B1, OL-B2, and OL-B3, included in the display device DD-TD, the amine compound of the present disclosure may be included.

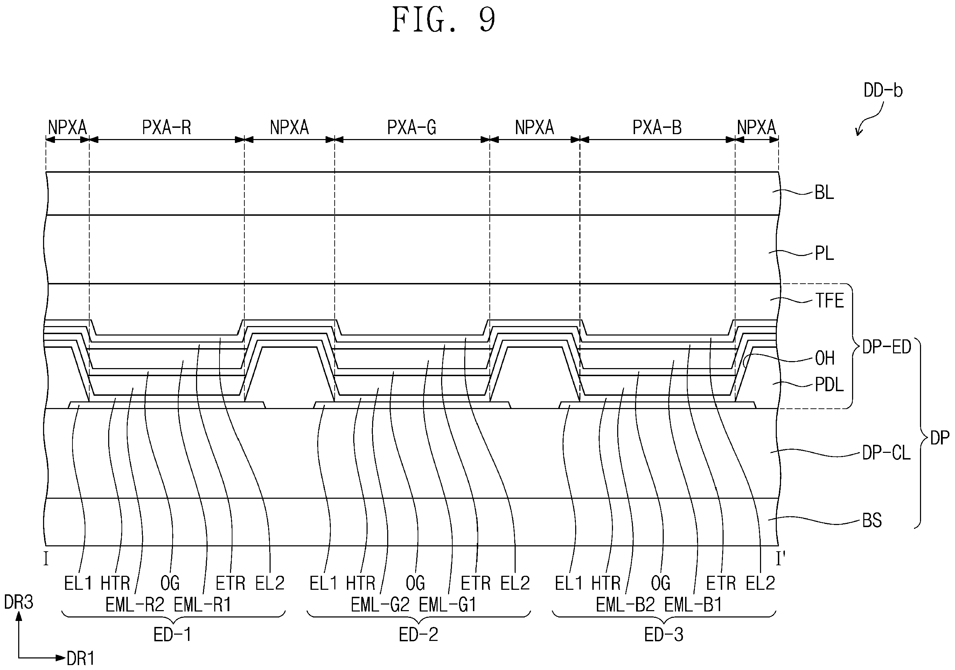

Referring to FIG. 9, a display device DD-b according to one or more embodiments may include light emitting elements ED-1, ED-2, and ED-3, formed by stacking two emission layers. Compared to the display device DD shown in FIG. 2, the display device DD-b shown in FIG. 9 is different in that first to third light emitting elements ED-1, ED-2, and ED-3 include two emission layers stacked in a thickness direction, each. In the first to third light emitting elements ED-1, ED-2, and ED-3, two emission layers may be to emit light in substantially the same wavelength region.

The first light emitting element ED-1 may include a first red emission layer EML-R1 and a second red emission layer EML-R2. The second light emitting element ED-2 may include a first green emission layer EML-G1 and a second green emission layer EML-G2. In addition, the third light emitting element ED-3 may include a first blue emission layer EML-B1 and a second blue emission layer EML-B2. Between the first red emission layer EML-R1 and the second red emission layer EML-R2, between the first green emission layer EML-G1 and the second green emission layer EML-G2, and between the first blue emission layer EML-B1 and the second blue emission layer EML-B2, an emission auxiliary part OG may be disposed.

The emission auxiliary part OG may include a single layer or a multilayer. The emission auxiliary part OG may include a charge generating layer. In some embodiments, the emission auxiliary part OG may include an electron transport region, a charge generating layer, and a hole transport region stacked in order (in the stated order). The emission auxiliary part OG may be provided as a common layer in all of the first to third light emitting elements ED-1, ED-2, and ED-3. However, embodiments of the present disclosure are not limited thereto, and the emission auxiliary part OG may be patterned and provided in an opening portion OH defined in a pixel definition film PDL.

The first red emission layer EML-R1, the first green emission layer EML-G1, and the first blue emission layer EML-B1 may be disposed between the electron transport region ETR and the emission auxiliary part OG. The second red emission layer EML-R2, the second green emission layer EML-G2, and the second blue emission layer EML-B2 may be disposed between the hole transport region HTR and the emission auxiliary part OG.

For example, the first light emitting element ED-1 may include a first electrode EL1, a hole transport region HTR, a second red emission layer EML-R2, an emission auxiliary part OG, a first red emission layer EML-R1, an electron transport region ETR, and a second electrode EL2, stacked in order (in the stated order). The second light emitting element ED-2 may include a first electrode EL1, a hole transport region HTR, a second green emission layer EML-G2, an emission auxiliary part OG, a first green emission layer EML-G1, an electron transport region ETR, and a second electrode EL2, stacked in order (in the stated order). The third light emitting element ED-3 may include a first electrode EL1, a hole transport region HTR, a second blue emission layer EML-B2, an emission auxiliary part OG, a first blue emission layer EML-B1, an electron transport region ETR, and a second electrode EL2, stacked in order (in the stated order).

In some embodiments, an optical auxiliary layer PL may be disposed on a display element layer DP-ED. The optical auxiliary layer PL may include a polarization layer. The optical auxiliary layer PL may be disposed on a display panel DP and may control reflected light at the display panel DP by external light. In one or more embodiments, different from the drawings, the optical auxiliary layer PL may not be provided in the display device.

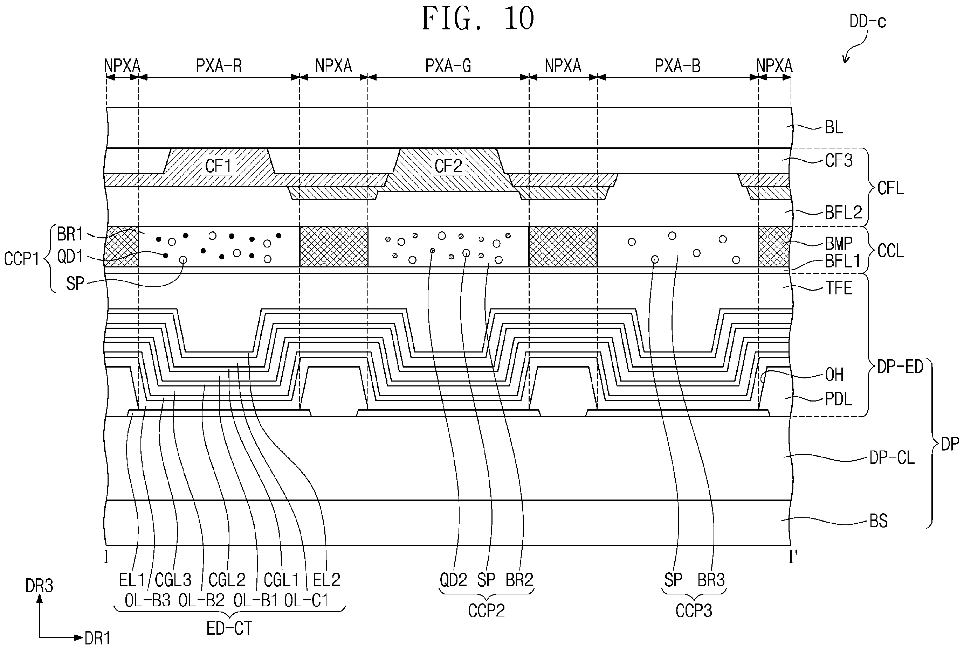

Different from FIG. 8 and FIG. 9, according to one or more embodiments, a display device DD-c shown in FIG. 10 may include four light emitting structures OL-B1, OL-B2, OL-B3, and OL-C1. A light emitting element ED-CT may include oppositely disposed first electrode EL1 and second electrode EL2, and first to fourth light emitting structures OL-B1, OL-B2, OL-B3, and OL-C1 disposed between the first electrode EL1 and the second electrode EL2. In a thickness direction, the third light emitting structures OL-B3, the second light emitting structures OL-B2, the first light emitting structures OL-B1, and the fourth light emitting structures OL-C1 are stacked in order(in the stated order). Between the first to fourth light emitting structures OL-B1, OL-B2, OL-B3, and OL-C1, charge generating layers CGL1, CGL2, and CGL3 may be disposed. Among the four light emitting structures, the first to third light emitting structures OL-B1, OL-B2, and OL-B3 may be to emit blue light, and the fourth light emitting structure OL-C1 may be to emit green light. However, embodiments of the present disclosure are not limited thereto, for example, the first to fourth light emitting structures OL-B1, OL-B2, OL-B3, and OL-C1 may be to emit light of different wavelengths.

Charge generating layers CGL1, CGL2, and CGL3 disposed among neighboring light emitting structures OL-B1, OL-B2, OL-B3, and OL-C1 may include a p-type or kind charge generating layer and/or an n-type or kind charge generating layer.

In one or more embodiments, in at least one selected from among the light emitting structures OL-B1, OL-B2, OL-B3, and OL-C1, included in the display device DD-c, the amine compound of the present disclosure may be included.

The light emitting element ED according to one or more embodiments of the present disclosure may include the amine compound of the present disclosure in at least one functional layer disposed between the first electrode EL1 and the second electrode EL2 to show improved emission efficiency and improved life characteristics. The light emitting element ED according to one or more embodiments may include the amine compound of the present disclosure in at least one selected from among a hole transport region HTR, an emission layer EML, and an electron transport region ETR, disposed between the first electrode EL1 and the second electrode EL2, or in a capping layer CPL. For example, the amine compound of the present disclosure may be included in the hole transport region HTR of the light emitting element ED, and the light emitting element ED may show high emission efficiency and long-life characteristics.

The amine compound of the present disclosure may include first to third substituents and may improve the stability of a material and improve hole transport properties. Accordingly, the lifetime and emission efficiency of the light emitting element including the amine compound of the present disclosure may be improved. In some embodiments, the light emitting element of one or more embodiments of the present disclosure may include the amine compound according to one or more embodiments in a hole transport layer to show improved emission efficiency and lifetime characteristics.

Hereinafter, referring to embodiments and comparative embodiments, the amine compound according to one or more embodiments and the light emitting element according to one or more embodiments of the present disclosure will be explained in more detail. In addition, the embodiments are illustrations to assist the understanding of the present disclosure, but the scope of the present disclosure is not limited thereto.

### Examples

### 1. Synthesis of amine compounds

First, the synthetic methods of the amine compounds according to one or more embodiments will be explained in more detail illustrating the synthetic methods of Compound 3, Compound 57, Compound 115, Compound 131, Compound 169, Compound 173, Compound 241, Compound 246, Compound 266, Compound 274, Compound 275, and Compound 317, disclosed in Table 1. In addition, the synthetic methods of the amine compounds explained hereinafter are embodiments, and the synthetic method of the amine compound of the present disclosure is not limited to the embodiments.

### Synthetic method of compounds

### 1) Synthesis of Compound 3

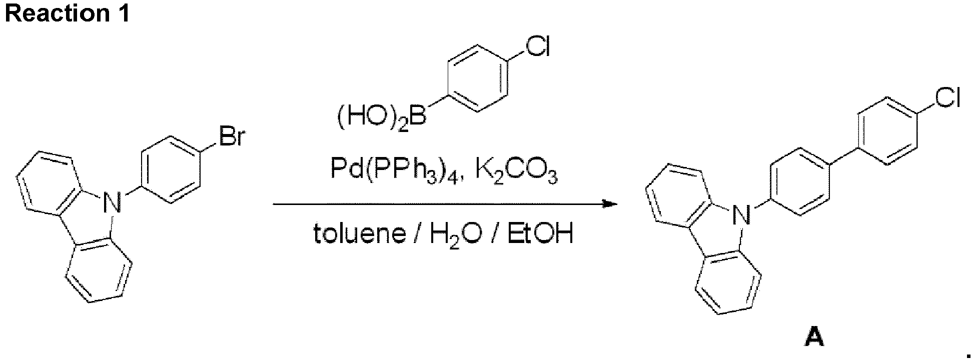

Under an Ar atmosphere, to a 1 L, three-neck flask, 9-(4-bromophenyl)carbazole (20.0 g), (4-chlorophenyl)boronic acid (9.70 g), tetrakis(triphenylphosphine)palladium(0) (Pd(PPh₃)₄, 3.5 g), and potassium carbonate (K₂CO₃, 17.2 g) were added and dissolved in a mixture solvent (a volume ratio of 10:2:1, 300 mL) of toluene, water and ethanol, followed by heating and stirring at about 80 °C for about 5 hours. After cooling at room temperature, the precipitate thus produced was collected and washed with toluene and ethanol to obtain Intermediate A (18.9 g, yield 86%). The molecular weight of Intermediate A measured by FAB-MS was 353.

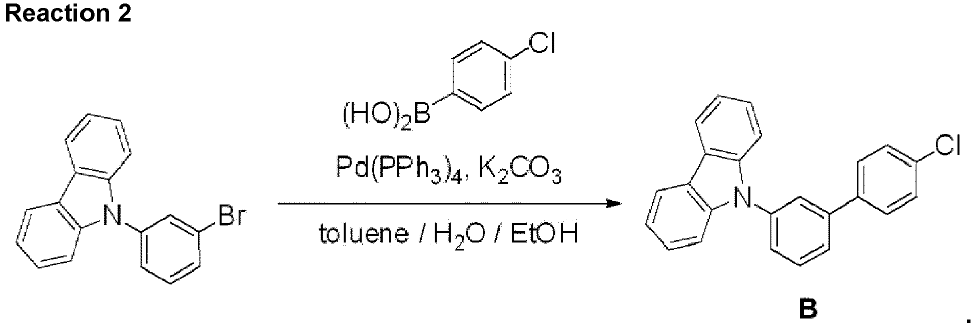

Under an Ar atmosphere, to a 1 L, three-neck flask, 9-(3-bromophenyl)carbazole (20.0 g), (4-chlorophenyl)boronic acid (9.70 g), Pd(PPh₃)₄ (3.5 g), and K₂CO₃ (17.3 g) were added and dissolved in a mixture solvent (a volume ratio of 10:2:1, 300 mL) of toluene, water and ethanol, followed by heating and stirring at about 80 °C for about 6 hours. Water was added, and extraction with CH₂Cl₂ was performed. Organic layers were collected and dried over MgSO₄, and the solvent was removed under a reduced pressure. The composition thus obtained was purified by silica gel column chromatography to obtain Intermediate B (17.3 g, yield 79%). The molecular weight of Intermediate B measured by FAB-MS was 353.

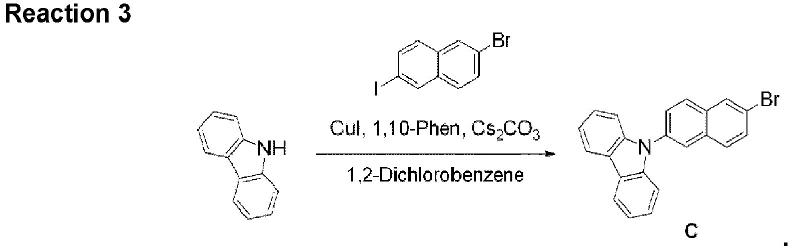

Under an Ar atmosphere, to a 1 L, three-neck flask, carbazole (10.0 g), 2-bromo-6-iodonaphthalene (30.0 g), copper(I) iodide (Cui, 1.2 g), 1,10-phenanthroline (1,10-Phen, 2.2 g), and cesium carbonate (Cs₂CO₃, 58 g) were added and dissolved in 1,2-dichlorobenzene (300 mL), followed by heating and stirring at about 150 °C for about 12 hours. Water was added, and extraction with CH₂Cl₂ was performed. Organic layers were collected and dried over MgSO₄, and the solvent was removed under a reduced pressure. The composition thus obtained was purified by silica gel column chromatography to obtain Intermediate C (12.9 g, yield 58%). The molecular weight of Intermediate C measured by FAB-MS was 372.

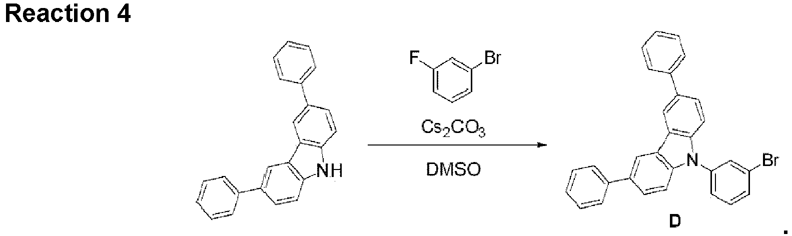

Under an Ar atmosphere, to a 500 mL, three-neck flask, 3,6-diphenylcarbazole (10.0 g), 1-bromo-3-fluorobenzene (8.2 g), and Cs₂CO₃ (20 g) were added and dissolved in DMSO (150 mL), followed by heating and stirring at about 150 °C for about 20 hours. After cooling at room temperature, the reaction solvent was poured into water, the precipitate thus produced was collected and washed with toluene and ethanol to obtain Intermediate D (9.65 g, yield 65%). The molecular weight of Intermediate D measured by FAB-MS was 474.

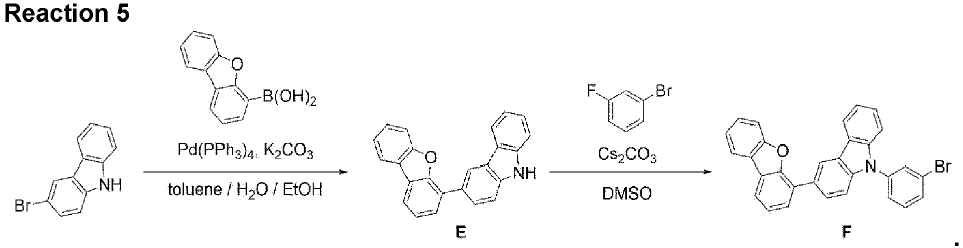

By the same method as the synthesis of Intermediate B, Intermediate E was obtained (10.8 g, yield 80%) from 3-bromocarbazole (10.0 g) and dibenzofuran-4-ylboronic acid (8.6 g). The molecular weight of Intermediate E measured by FAB-MS was 333.

By the same method as the synthesis of Intermediate D, Intermediate F was obtained (8.2 g, yield 56%) from Intermediate E (10.0 g) and 1-bromo-3-fluorobenzene (7.9 g). The molecular weight of Intermediate F measured by FAB-MS was 488.

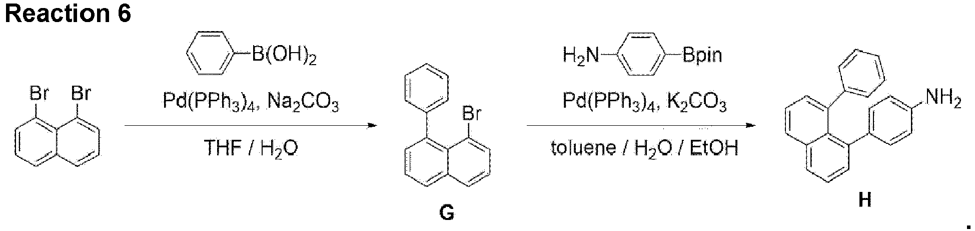

Under an Ar atmosphere, to a 1 L, three-neck flask, 1,8-dibromonaphthalene (20.0 g), phenylboronic acid (8.52 g), Pd(PPh₃)₄ (4.0 g), and sodium carbonate (Na₂CO₃, 15 g) were added and dissolved in a mixture solvent (a volume ratio of 8:2, 350 mL) of THF and water, followed by heating and stirring at about 70 °C for about 10 hours. Water was added, and extraction with CH₂Cl₂ was performed. Organic layers were collected and dried over MgSO₄, and the solvent was removed under a reduced pressure. The composition thus obtained was purified by silica gel column chromatography to obtain Intermediate G (14.8 g, yield 75%). The molecular weight of Intermediate G measured by FAB-MS was 283.

Under an Ar atmosphere, to a 1 L, three-neck flask, Intermediate G (20.0 g), 4-(4,4,5,5-tetramethyl-1,3,2-dioxaborolan-2-yl)aniline (15.5 g), Pd(PPh₃)₄ (4.0 g), and K₂CO₃ (20 g) were added and dissolved in a mixture solvent (a volume ratio of 10:2:1, 350 mL) of toluene, water and ethanol, followed by heating and stirring at about 80 °C for about 10 hours. Water was added, and extraction with CH₂Cl₂ was performed. Organic layers were collected and dried over MgSO₄, and the solvent was removed under a reduced pressure. The composition thus obtained was purified by silica gel column chromatography to obtain Intermediate H (15.8 g, yield 76%). The molecular weight of Intermediate H measured by FAB-MS was 295.

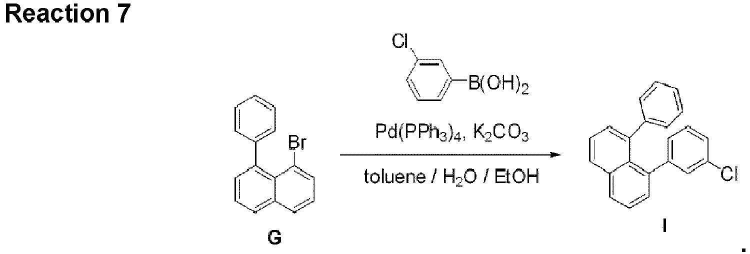

By the same method as the synthesis of Intermediate H, Intermediate I was obtained (7.45 g, yield 67%) from Intermediate G (10.0 g) and (3-chlorophenyl)boronic acid (5.52 g). The molecular weight of Intermediate I measured by FAB-MS was 314.

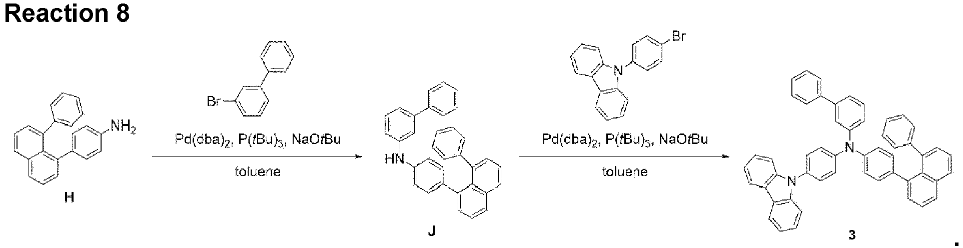

Under an Ar atmosphere, to a 500 mL, three-neck flask, Intermediate H (10.0 g), 3-bromobiphenyl (7.89 g), bis(dibenzylideneacetone)palladium(0) (Pd(dba)₂, 0.97 g), and sodium tert-butoxide (NaOtBu, 3.26 g) were added and dissolved in toluene (170 mL), and tri-tert-butylphosphine (P(tBu)s, 2.0 M in toluene, 1.5 mL) was added thereto, followed by stirring at room temperature for about 6 hours. Water was added, and extraction with CH₂Cl₂ was performed. Organic layers were collected and dried over MgSO₄, and the solvent was removed under a reduced pressure. The composition thus obtained was purified by silica gel column chromatography to obtain Intermediate J (12.8 g, yield 85%). The molecular weight of Intermediate J measured by FAB-MS was 447.

Under an Ar atmosphere, to a 200 mL, three-neck flask, Intermediate J (3.0 g), 9-(4-bromophenyl)carbazole (2.16 g), Pd(dba)₂ (0.2 g), and NaOtBu (1.0 g) were added and dissolved in toluene (50 mL), and P(tBu)s (2.0 M in toluene, 0.3 mL) was added thereto, followed by heating and stirring at about 100 °C for about 6 hours. Water was added, and extraction with CH₂Cl₂ was performed. Organic layers were collected and dried over MgSO₄, and the solvent was removed under a reduced pressure. The composition thus obtained was purified by silica gel column chromatography to obtain Compound 3 (3.51 g, yield 76%). The molecular weight of Compound 3 measured by FAB-MS was 688.

### 2) Synthesis of Compound 57

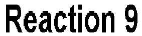

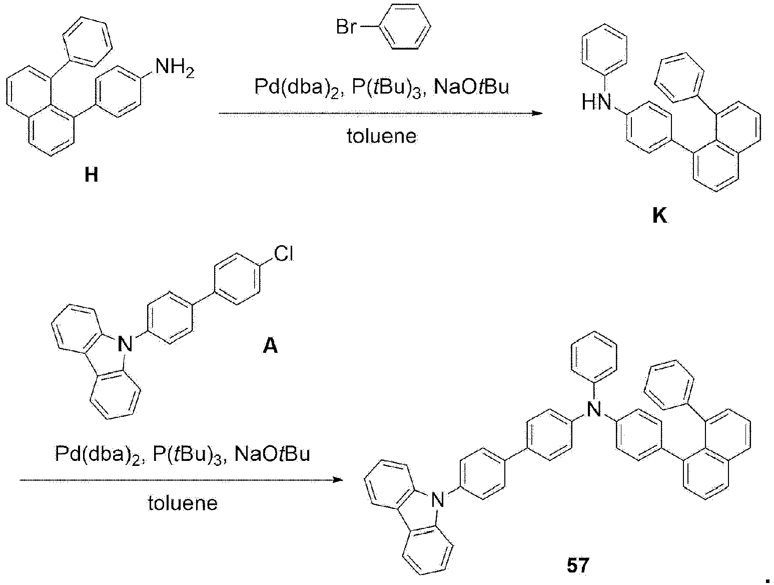

By the same method as the synthesis of Intermediate J, Intermediate K was obtained (21.9 g, yield 87%) by utilizing Intermediate H (20.0 g) instead of Intermediate H (10.0 g) and utilizing bromobenzene (10.6 g) instead of 3-bromobiphenyl. The molecular weight of Intermediate K measured by FAB-MS was 371.

By the same method as the synthesis of Compound 3, Compound 57 was obtained (4.67 g, yield 84%) by utilizing Intermediate K (3.0 g) instead of Intermediate J and utilizing Intermediate A (2.85 g) instead of 9-(4-bromophenyl)carbazole. The molecular weight of Compound 57 measured by FAB-MS was 688.

### 3) Synthesis of Compound 115

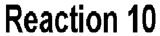

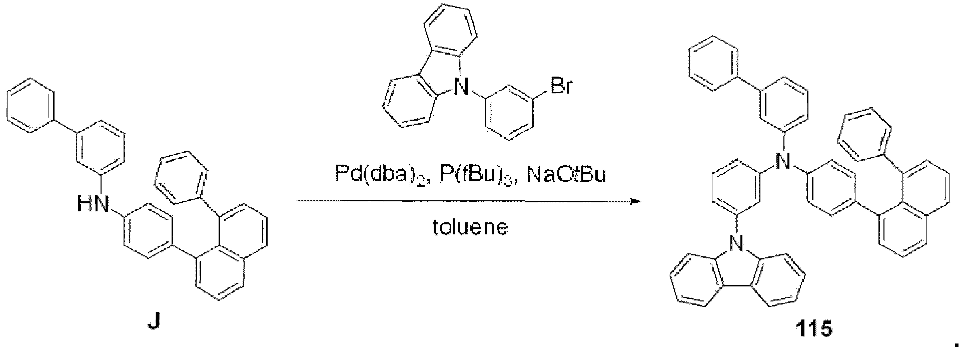

By the same method as the synthesis of Compound 3, Compound 115 was obtained (3.65 g, yield 79%) by utilizing 9-(3-bromophenyl)carbazole (2.16 g) instead of 9-(4-bromophenyl)carbazole. The molecular weight of Compound 115 measured by FAB-MS was 688.

### 4) Synthesis of Compound 131

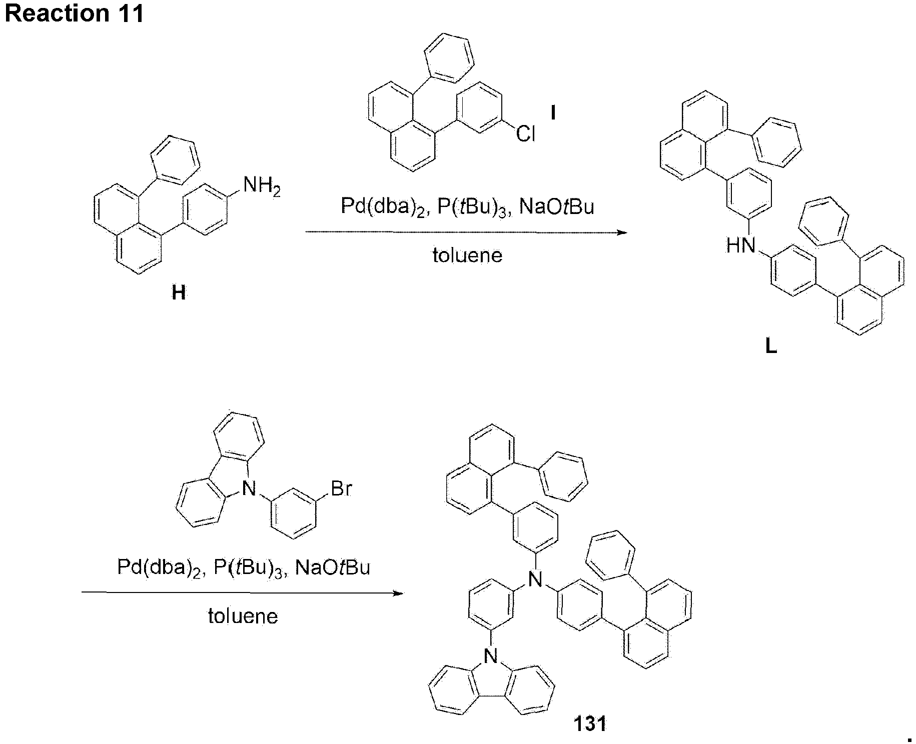

By the same method as the synthesis of Compound 3, Intermediate L was obtained (5.63 g, yield 58%) by utilizing Intermediate H (5.0 g) instead of Intermediate J and utilizing Intermediate I (5.32 g) instead of 9-(4-bromophenyl)carbazole. The molecular weight of Intermediate L measured by FAB-MS was 573.

By the same method as the synthesis of Compound 3, Compound 131 was obtained (3.32 g, yield 78%) by utilizing Intermediate L (3.0 g) instead of Intermediate J and utilizing 9-(3-bromophenyl)carbazole (1.68 g) instead of 9-(4-bromophenyl)carbazole. The molecular weight of Compound 131 measured by FAB-MS was 815.

### 5) Synthesis of Compound 169

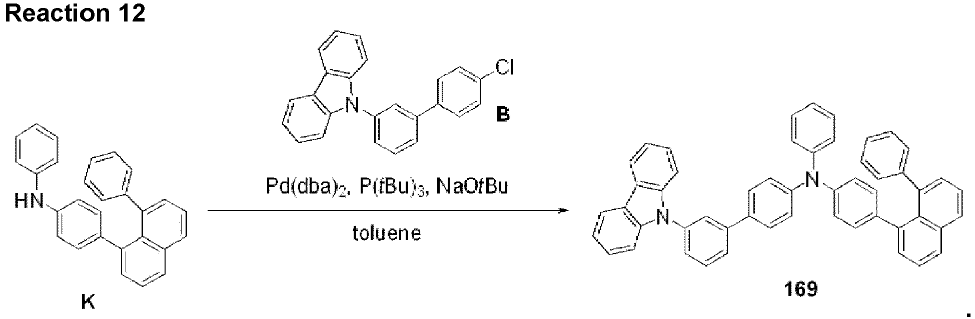

By the same method as the synthesis of Compound 3, Compound 169 was obtained (4.73 g, yield 85%) by utilizing Intermediate K (3.0 g) instead of Intermediate J and utilizing Intermediate B (2.86 g) instead of 9-(4-bromophenyl)carbazole. The molecular weight of Compound 169 measured by FAB-MS was 688.

### 6) Synthesis of Compound 173

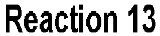

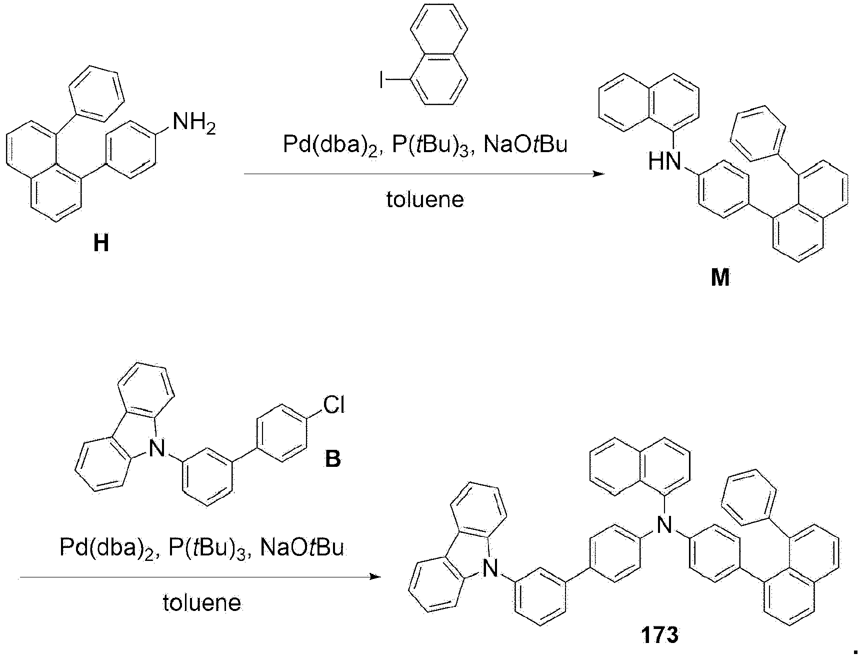

By the same method as the synthesis of Intermediate J, Intermediate M was obtained (4.42 g, yield 62%) by utilizing Intermediate H (5.0 g) instead of Intermediate H (10.0 g) and 1-iodonaphthalene (4.30 g) instead of 3-bromobiphenyl. The molecular weight of Intermediate M measured by FAB-MS was 421.

By the same method as the synthesis of Compound 3, Compound 173 was obtained (4.42 g, yield 84%) by utilizing Intermediate M (3.0 g) instead of Intermediate J and utilizing Intermediate B (2.52 g) instead of 9-(4-bromophenyl)carbazole. The molecular weight of Compound 173 measured by FAB-MS was 738.

### 7) Synthesis of Compound 241

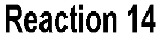

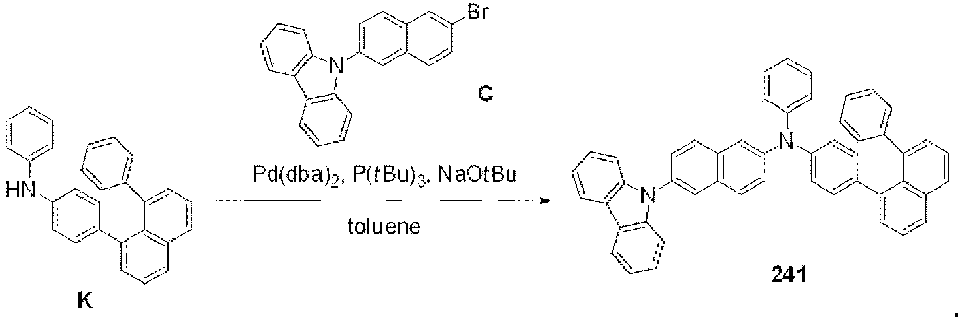

By the same method as the synthesis of Compound 3, Compound 241 was obtained (4.55 g, yield 85%) by utilizing Intermediate K (3.0 g) instead of Intermediate J and utilizing Intermediate C (3.00 g) instead of 9-(4-bromophenyl)carbazole. The molecular weight of Compound 241 measured by FAB-MS was 662.

### 8) Synthesis of Compound 246

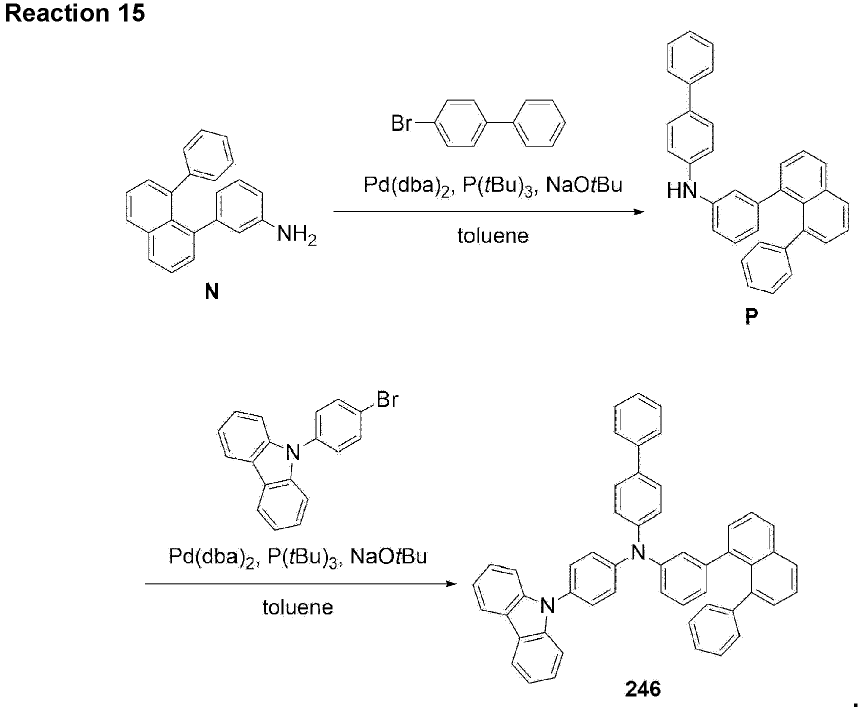

By the same method as the synthesis of Intermediate K, Intermediate P was obtained (10.7 g, yield 71%) by utilizing Intermediate N (10.0 g) instead of Intermediate H and 4-bromobiphenyl (7.9 g) instead of bromobenzene. The molecular weight of Intermediate P measured by FAB-MS was 447.

By the same method as the synthesis of Compound 3, Compound 246 was obtained (3.79 g, yield 82%) by utilizing Intermediate P (3.0 g) instead of Intermediate J and utilizing 9-(4-bromophenyl)carbazole (2.16 g). The molecular weight of Compound 246 measured by FAB-MS was 688.

### 9) Synthesis of Compound 266

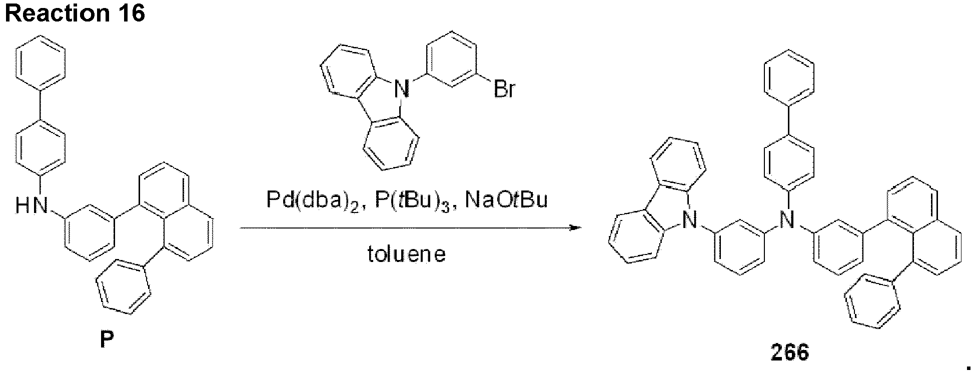

By the same method as the synthesis of Compound 3, Compound 266 was obtained (3.51 g, yield 76%) by utilizing Intermediate P (3.0 g) instead of Intermediate J and utilizing 9-(3-bromophenyl)carbazole (2.16 g) instead of 9-(4-bromophenyl)carbazole. The molecular weight of Compound 266 measured by FAB-MS was 688.

### 10) Synthesis of Compound 274

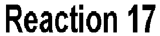

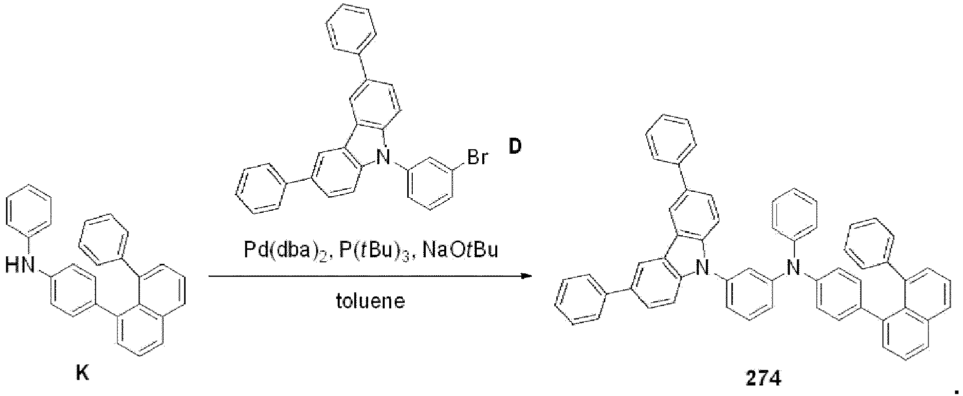

By the same method as the synthesis of Compound 3, Compound 274 was obtained (4.57 g, yield 74%) by utilizing Intermediate K (3.0 g) instead of Intermediate J and utilizing Intermediate D (3.83 g) instead of 9-(4-bromophenyl)carbazole. The molecular weight of Compound 274 measured by FAB-MS was 764.

### 11) Synthesis of Compound 275

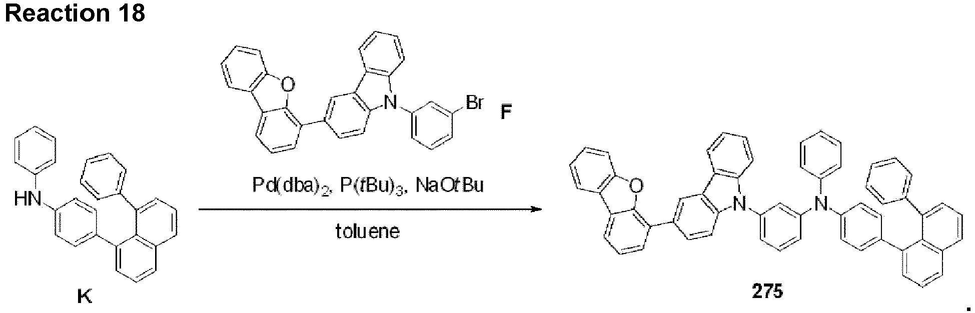

By the same method as the synthesis of Compound 3, Compound 275 was obtained (4.59 g, yield 73%) by utilizing Intermediate K (3.0 g) instead of Intermediate J and utilizing Intermediate F (3.94 g) instead of 9-(4-bromophenyl)carbazole. The molecular weight of Compound 275 measured by FAB-MS was 778.

### 12) Synthesis of Compound 317

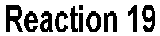

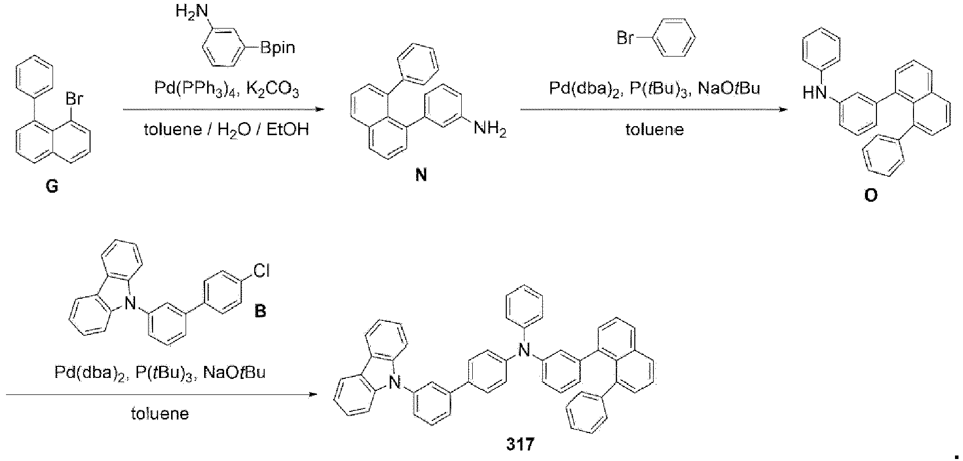

By the same method as the synthesis of Intermediate H, Intermediate N was obtained (13.8 g, yield 60%) by utilizing Intermediate G (3.0 g), and 3-(4,4,5,5-tetramethyl-1,3,2-dioxaborolan-2-yl)aniline (15.5 g) instead of 4-(4,4,5,5-tetramethyl-1,3,2-dioxaborolan-2-yl)aniline. The molecular weight of Intermediate N measured by FAB-MS was 295.

By the same method as the synthesis of Intermediate K, Intermediate O was obtained (12.9 g, yield 79%) by utilizing Intermediate N (13.0 g) instead of Intermediate H, and utilizing bromobenzene (6.91 g). The molecular weight of Intermediate O measured by FAB-MS was 371.

By the same method as the synthesis of Compound 3, Compound 317 was obtained (4.45 g, yield 80%) by utilizing Intermediate O (3.0 g) instead of Intermediate J and utilizing Intermediate B (2.86 g) instead of 9-(4-bromophenyl)carbazole. The molecular weight of Compound 317 measured by FAB-MS was 688.

### 2. Manufacture and evaluation of light emitting elements

### (1) Manufacture of light emitting element

Light emitting elements including the amine compound of one or more embodiments in a hole transport layer were manufactured.

A glass substrate on which ITO with a thickness of about 1500 Å was patterned, was washed with ultrapure water, and treated with UV ozone for about 10 minutes to form a first electrode. Then, 2-TNATA was deposited to a thickness of about 600 Å to form a hole injection layer. After that, the Example Compound or Comparative Compound was deposited to a thickness of about 300 Å to form a hole transport layer.

Then, an emission layer with a thickness of about 250 Å was formed utilizing ADN doped with 3% of TBP. Then, Alqs was deposited to a thickness of about 250 Å to form an electron transport layer, and LiF was deposited to a thickness of about 10 Å to form an electron injection layer.

After that, a second electrode was formed by providing aluminum (Al) to a thickness of about 1000 Å. In the Examples, the hole injection layer, the hole transport layer, the emission layer, the electron transport layer, the electron injection layer and the second electrode were formed utilizing a vacuum deposition apparatus.

### Example Compounds

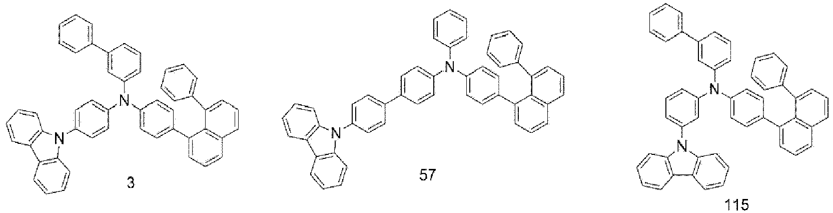

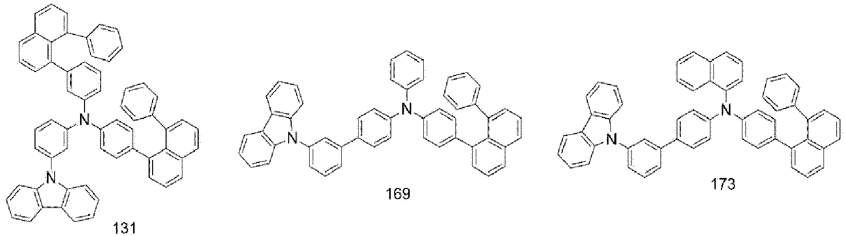

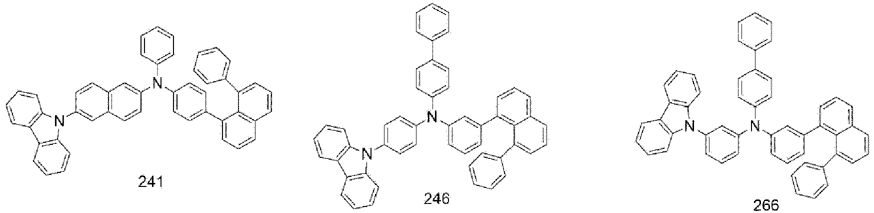

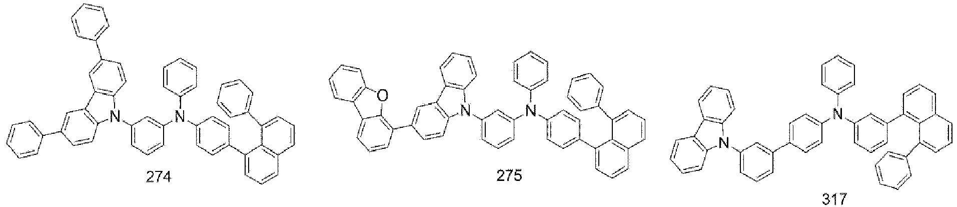

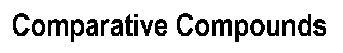

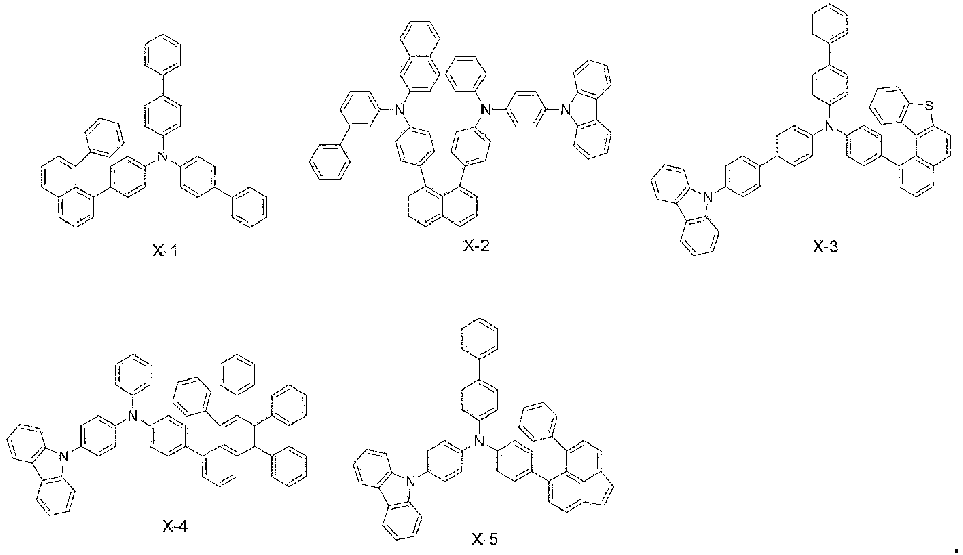

In addition, the compounds of the functional layers utilized for the manufacture of the light emitting elements are as follows.

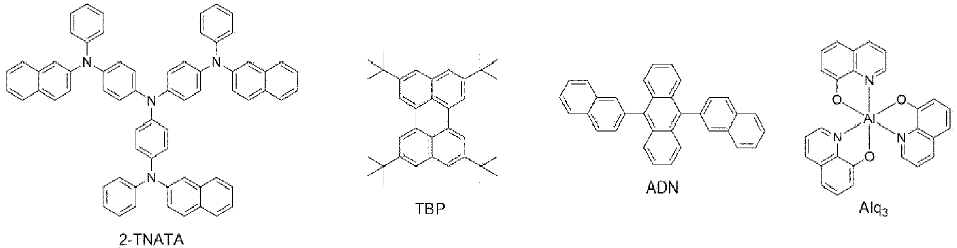

### (2) Evaluation of light emitting element

Table 1 shows evaluation results on the light emitting elements of Examples 1 to 12, and Comparative Examples 1 to 5. In the evaluation results of the properties of the Examples and Comparative Examples, shown in Table 1, the emission efficiency shows efficiency values at a current density of about 10 mA/cm², and the half-life shows luminance half-life from about 1000 cd/m². For the evaluation of the emission properties of the light emitting elements, a light distribution characteristics measurement system of C9920-11 which is a product of Hamamatsu Photonics K.K., was utilized.

In Table 1, each of the emission efficiency and lifetime is shown as a relative value based on 100 for Comparative Example 1.

**[Table 1]**

| Division | Hole transport layer (HTL) material | Emission efficiency (cd/A) | Lifetime LT50 |
|---|---|---|---|
| Example 1 | Example Compound 3 | 108% | 120% |
| Example 2 | Example Compound 57 | 107% | 160% |
| Example 3 | Example Compound 115 | 108% | 150% |
| Example 4 | Example Compound 131 | 110% | 150% |
| Example 5 | Example Compound 169 | 107% | 155% |
| Example 6 | Example Compound 173 | 108% | 160% |
| Example 7 | Example Compound 241 | 107% | 130% |
| Example 8 | Example Compound 246 | 110% | 110% |
| Example 9 | Example Compound 266 | 110% | 120% |
| Example 10 | Example Compound 274 | 110% | 130% |
| Example 11 | Example Compound 275 | 110% | 110% |
| Example 12 | Example Compound 317 | 109% | 160% |
| Comparative Example 1 | Comparative Compound X-1 | 100% | 100% |
| Comparative Example 2 | Comparative Compound X-2 | 98% | 50% |
| Comparative Example 3 | Comparative Compound X-3 | 100% | 90% |
| Comparative Example 4 | Comparative Compound X-4 | 97% | 60% |
| Comparative Example 5 | Comparative Compound X-5 | 95% | 40% |

Referring to the results of Table 1, it can be see that the light emitting elements of Example 1 to Example 12, utilizing the amine compounds according to the present disclosure as the hole transport layer materials, show excellent or suitable emission efficiency and long-life characteristics when compared to the light emitting elements of Comparative Example 1 to Comparative Example 5, utilizing the amine compounds of the Comparative Examples as the hole transport layer materials.

Comparative Compound X-1 utilized in Comparative Example 1 does not include a carbazole substituent connected with the nitrogen atom of an amine, and it is thought that emission efficiency and lifetime characteristics were deteriorated when compared to the Examples.

Comparative Compound X-2 utilized in Comparative Example 2 is a diamine compound, and it is thought that emission efficiency and lifetime characteristics were deteriorated when compared to the Examples utilizing monoamine compounds.

In Comparative Compound X-3 utilized in Comparative Example 3, not phenylnaphthyl but naphtho benzothiophene is connected with the nitrogen atom of an amine via a linker, and it is thought that emission efficiency and lifetime characteristics were deteriorated when compared to the Examples.

Comparative Compound X-4 utilized in Comparative Example 4 includes a carbazole substituent connected via a phenylene group and a naphthyl group connected via a phenylene group with the nitrogen atom of an amine, but four phenyl groups are connected with one benzene ring included in the naphthyl group, and it is thought that emission efficiency and lifetime characteristics were deteriorated when compared to the Examples.

Comparative Compound X-5 utilized in Comparative Example 5 includes a carbazole substituent connected with the nitrogen atom of an amine via a phenylene group, and a biphenyl group connected with the nitrogen atom of an amine, but another substituent includes a fused structure of cyclopentadiene with naphthalene, and it is thought that emission efficiency and lifetime characteristics were deteriorated when compared to the Examples.

The amine compound of the present disclosure includes a first substituent having a carbazole skeleton connected via a linker, a second substituent which is a naphthyl group substituted with a substituted or unsubstituted phenyl group, connected via a linker, and a third substituent having an aryl group or a heteroaryl group skeleton, and may have high stability and high hole transport capacity.

The light emitting element of the present disclosure includes the amine compound of one or more embodiments in a hole transport layer, and hole transport capacity may be improved, material deterioration may be suppressed or reduced, and emission efficiency and lifetime characteristics may be improved.

The light emitting element includes the amine compound of one or more embodiments and may show high emission efficiency and long-life characteristics.

When the amine compound of the present disclosure is applied in a light emitting element, high emission efficiency and long lifetime characteristics may be shown.

As used herein, the term "substantially," "about," and similar terms are used as terms of approximation and not as terms of degree, and are intended to account for the inherent deviations in measured or calculated values that would be recognized by those of ordinary skill in the art. "About," "substantially," or "approximately," as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this disclosure is intended to include all higher numerical limitations subsumed therein. Accordingly, Applicant reserves the right to amend this disclosure, including the claims, to expressly recite any sub-range subsumed within the ranges expressly recited herein.

## Claims

1. An amine compound represented by Formula 1: 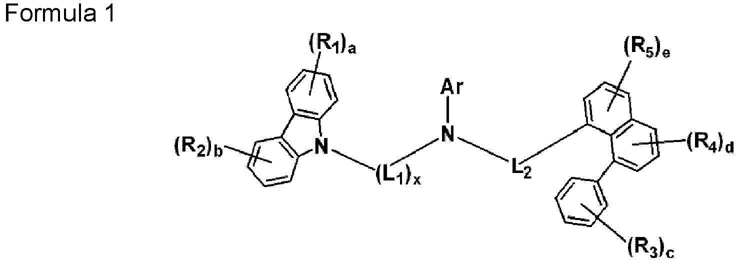 in Formula 1,
L₁ and L₂ are each independently selected from a substituted or unsubstituted arylene group of 6 to 30 ring-forming carbon atoms and a substituted or unsubstituted heteroarylene group of 2 to 30 ring-forming carbon atoms,
"x" is 1 or 2,
Ar is a substituted or unsubstituted aryl group or a substituted or unsubstituted heteroaryl group,
R₁ and R₂ are each independently selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms, and/or combined with an adjacent group to form a ring,
R₃ is selected from a hydrogen atom, a deuterium atom, a halogen atom, a substituted or unsubstituted silyl group, a substituted or unsubstituted alkenyl group of 2 to 30 carbon atoms, a substituted or unsubstituted aryl group of 6 to 30 ring-forming carbon atoms, and a substituted or unsubstituted heteroaryl group of 2 to 30 ring-forming carbon atoms, and/or combined with an adjacent group to form a ring, where a case where R₃ is an amine group-substituted aryl group is excluded,
R₄ and R₅ are each independently selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms,
"a" and "b" are each independently an integer of 0 to 4,
"c" is an integer of 0 to 5,
"d" is an integer of 0 to 3,
"e" is an integer of 0 to 3,
at least one R₄ is a hydrogen atom, and
a chemical structure of the amine compound in which optional hydrogen atom is substituted with deuterium atom is included.

2. The amine compound of claim 1, wherein the amine compound represented by Formula 1 is a monoamine compound.

3. The amine compound of any one of the preceding claims, wherein Ar is selected from a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted phenanthryl group, a substituted or unsubstituted fluorene group, a substituted or unsubstituted carbazole group, a substituted or unsubstituted dibenzofuran group, and a substituted or unsubstituted dibenzothiophene group.

4. The amine compound of any one of the preceding claims, wherein the amine compound represented by Formula 1 is represented by any one selected from among Formula 1-1 to Formula 1-3: 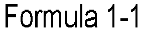 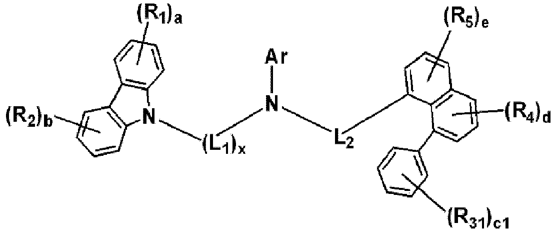 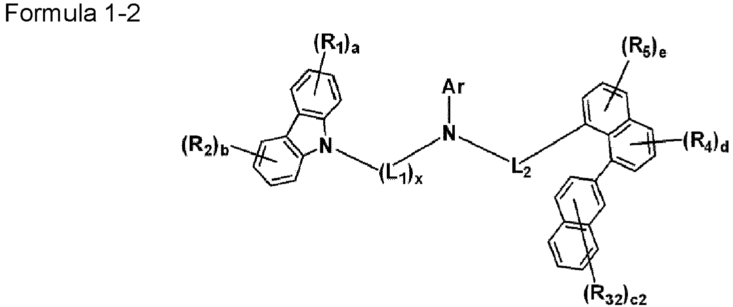 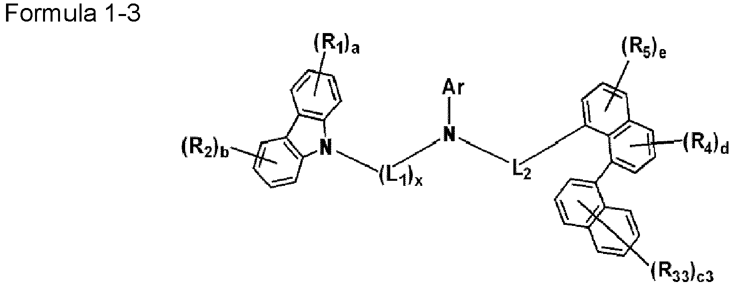 in Formula 1-1 to Formula 1-3,
R₃₁, R₃₂, and R₃₃ are each independently selected from a hydrogen atom, a deuterium atom, a halogen atom, a substituted or unsubstituted silyl group, a substituted or unsubstituted alkenyl group of 2 to 30 carbon atoms, a substituted or unsubstituted aryl group of 6 to 30 ring-forming carbon atoms, and a substituted or unsubstituted heteroaryl group of 2 to 30 ring-forming carbon atoms,
c1 is an integer of 0 to 5,
c2 and c3 are each an integer of 0 to 7,
where a case in which each of R₃₁, R₃₂, and R₃₃ is an amine group-substituted aryl group is excluded, and
Ar, R₁, R₂, R₄, R₅, L₁, L₂, "a", "b", "d", "e", and "x" are the same as defined in Formula 1.

5. The amine compound of any one of the preceding claims, wherein L₁ is represented by at least one selected from among substituents in Substituent Group S:
Substituent Group S 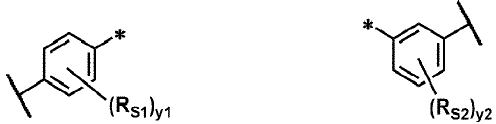 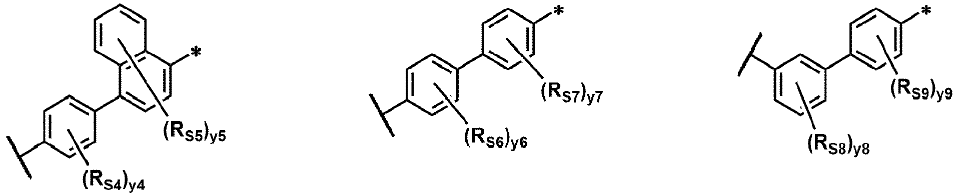 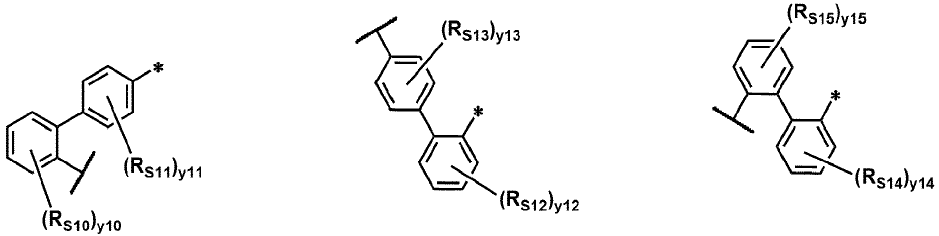 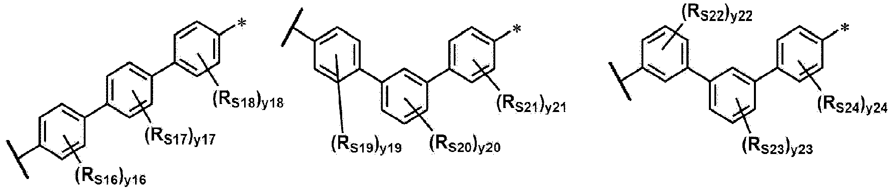 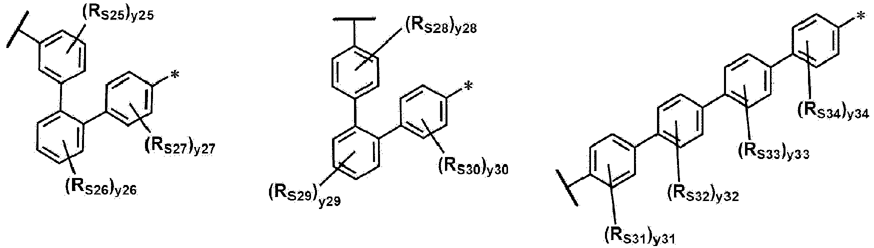 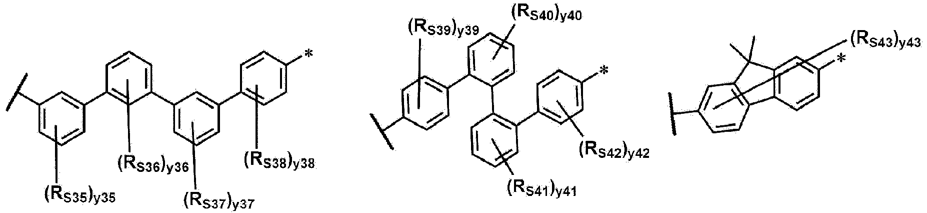 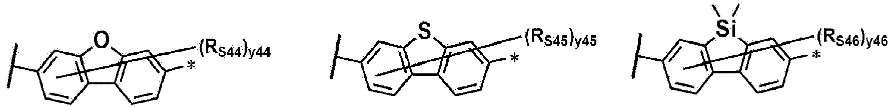 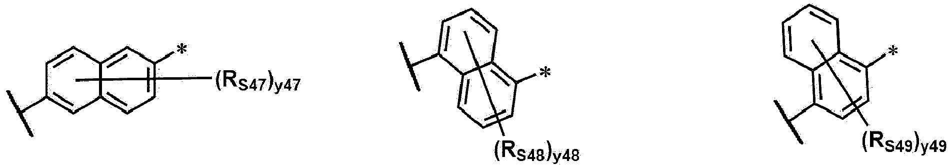 in Substituent Group S,
R_{S1}, R_{S2}, and R_{S4} to R_{S49} are each independently selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms,
y1, y2, y4, and y6 to y42 are each independently an integer of 0 to 4, and
y5 and y43 to y49 are each independently an integer of 0 to 6,
in Substituent Group S, " 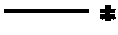 " means a position connected with an amine nitrogen atom of Formula 1, and 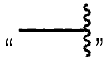 means a position connected with a nitrogen atom of carbazole in Formula 1.

6. The amine compound of any one of claims 1 to 3, wherein the amine compound represented by Formula 1 is represented by any one selected from among Formula 2-1 to Formula 2-6: 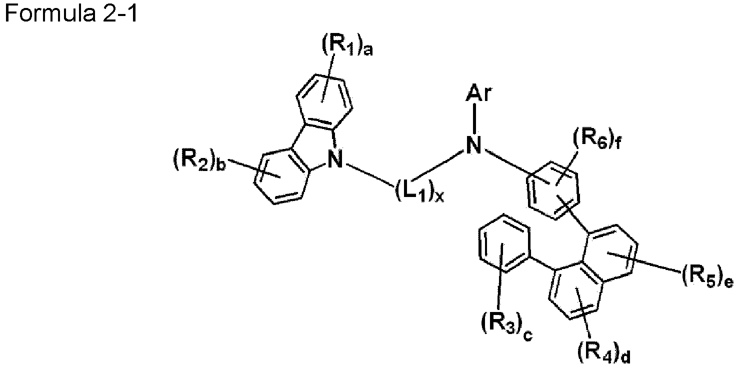 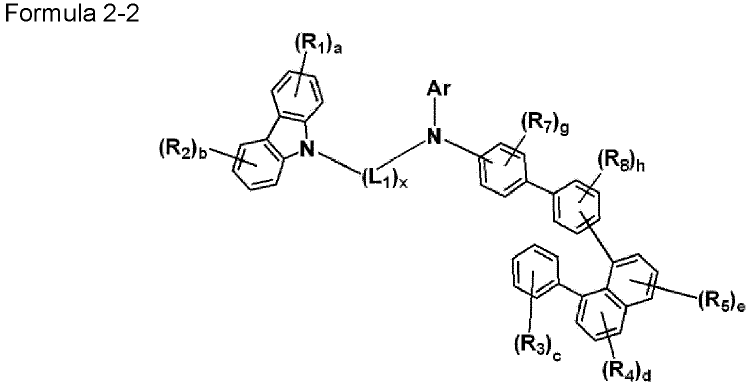 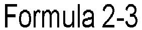 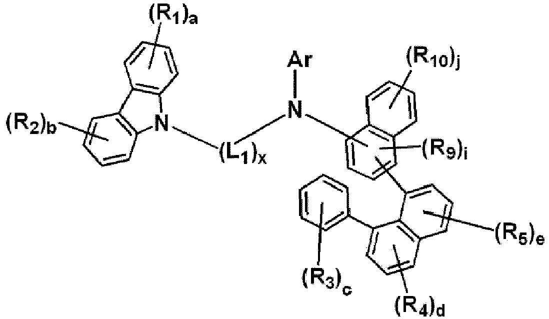 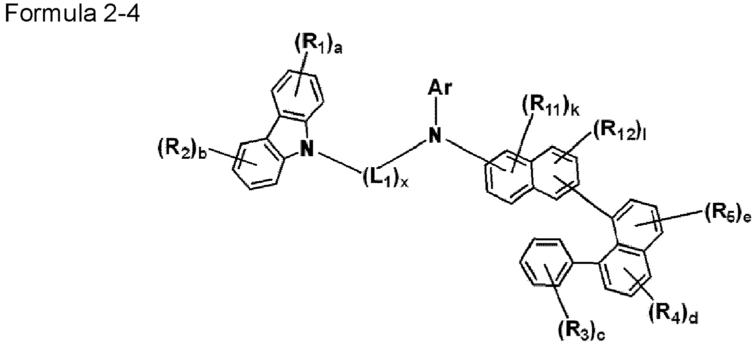 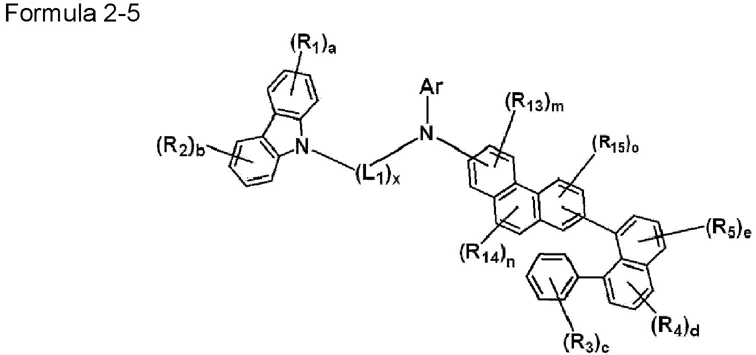 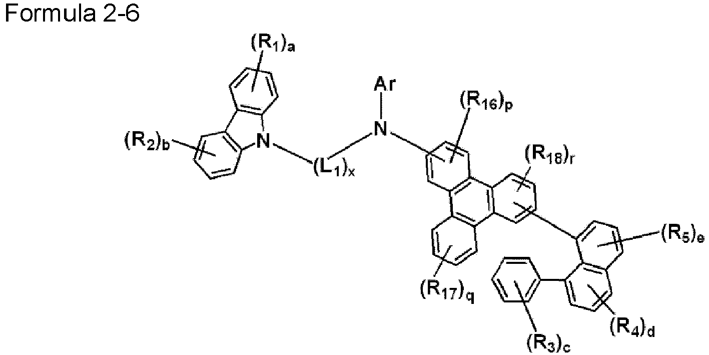 in Formula 2-1 to Formula 2-6,
R₆ to R₁₈ are each independently selected from a hydrogen atom, a deuterium atom, a halogen atom, a substituted or unsubstituted silyl group, a substituted or unsubstituted aryl group of 6 to 30 ring-forming carbon atoms, and a substituted or unsubstituted heteroaryl group of 2 to 30 ring-forming carbon atoms,
"f", "g", "h", "j" and "q" are each independently an integer of 0 to 4,
"i" and "n" are each independently an integer of 0 to 2,
"k", "l", "m", "o", "p" and "r" are each independently an integer of 0 to 3, and
Ar, R₁ to R₅, L₁, "a" to "e", and "x" are the same as defined in Formula 1.

7. The amine compound of any one of the preceding claims, wherein R₁ and R₂ are each independently selected from a hydrogen atom, a deuterium atom, a substituted or unsubstituted methyl group, a substituted or unsubstituted phenyl group, a substituted or unsubstituted dibenzofuran group, and a substituted or unsubstituted dibenzoheterole group, and/or combined with an adjacent group to form a ring.

8. The amine compound of any one of claims 1 to 3 and 5 to 7, wherein R₃ is selected from a hydrogen atom, a deuterium atom, a substituted or unsubstituted allyl group, a substituted or unsubstituted pyridine group, a substituted or unsubstituted tetrazole group, a substituted or unsubstituted dibenzofuran group, and a substituted or unsubstituted triazine group, and/or combined with an adjacent group to form a ring.

9. The amine compound of any one of the preceding claims, wherein R₄ and R₅ are each independently selected from a hydrogen atom and a deuterium atom.

10. The amine compound of any one of claims 1 to 4 and 5 to 9, wherein L₁ is selected from a substituted or unsubstituted phenylene group, a substituted or unsubstituted divalent biphenyl group, a substituted or unsubstituted divalent terphenyl group, a substituted or unsubstituted divalent quaterphenyl group, a substituted or unsubstituted divalent fluorene group, a substituted or unsubstituted divalent dibenzofuran group, a substituted or unsubstituted divalent dibenzoheterole group, a substituted or unsubstituted divalent dibenzosilole group, and a substituted or unsubstituted divalent naphthyl group.

11. The amine compound of any one of claims 1 to 5 and 7 to 10, wherein L₂ is selected from a substituted or unsubstituted phenylene group, a substituted or unsubstituted divalent biphenyl group, a substituted or unsubstituted divalent naphthyl group, a substituted or unsubstituted divalent phenanthryl group, and a substituted or unsubstituted divalent triphenylenyl group.

12. The amine compound of claim 1, wherein the amine compound represented by Formula 1 is represented by any one selected from among compounds in Compound Group 1: 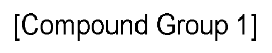 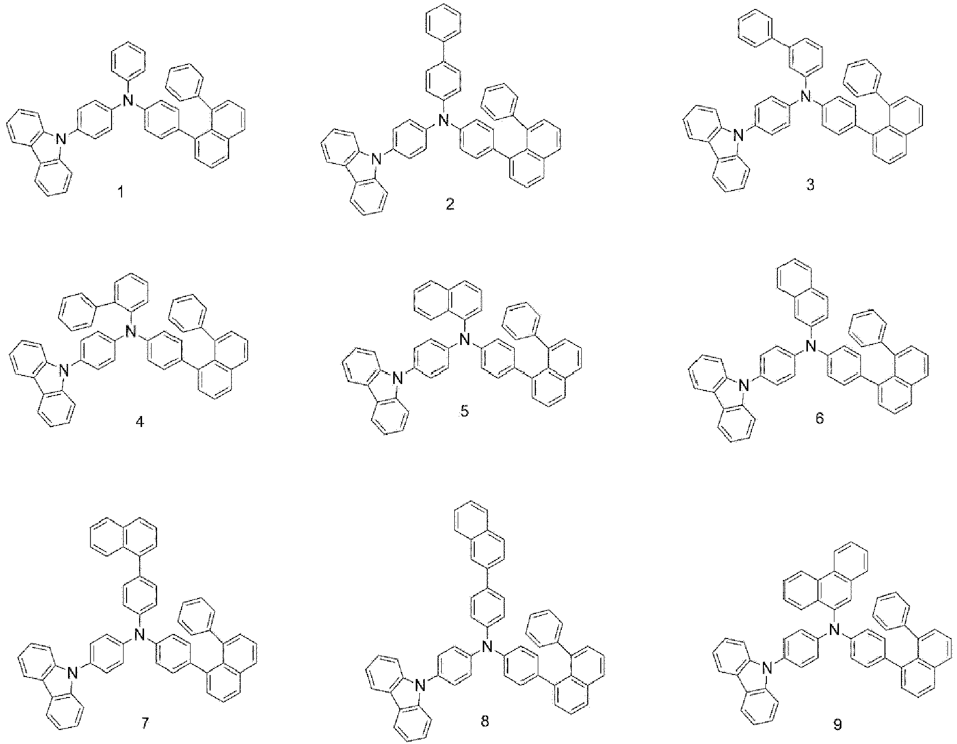 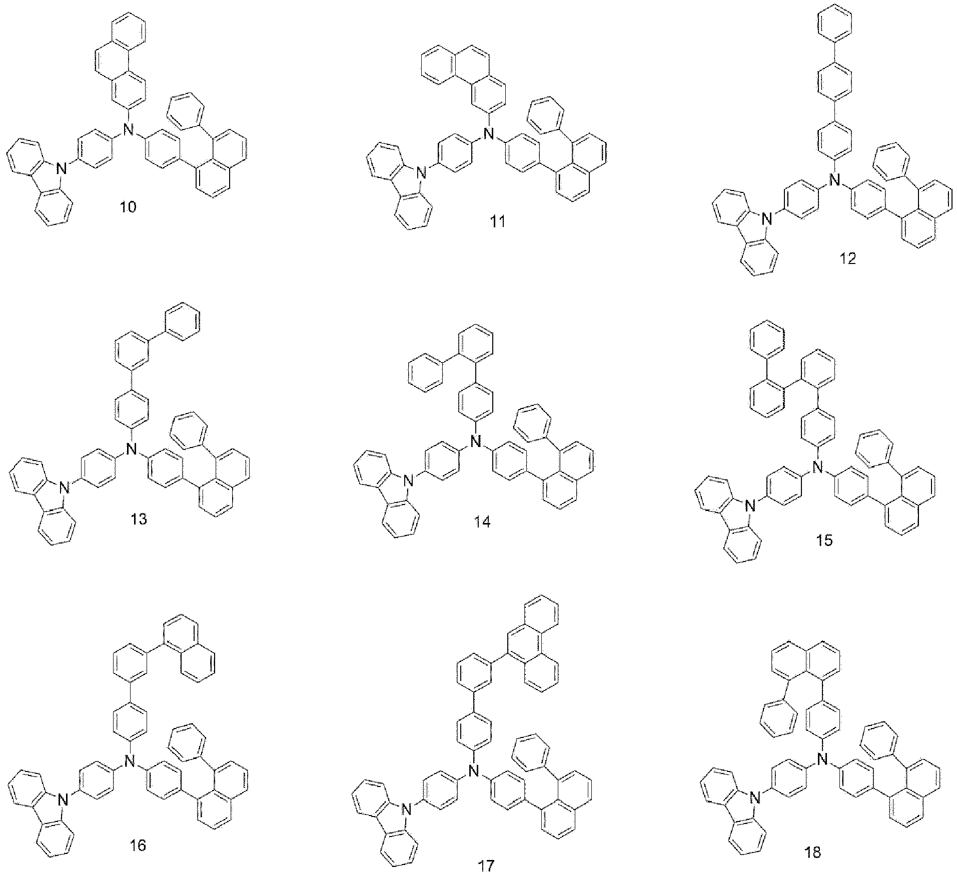 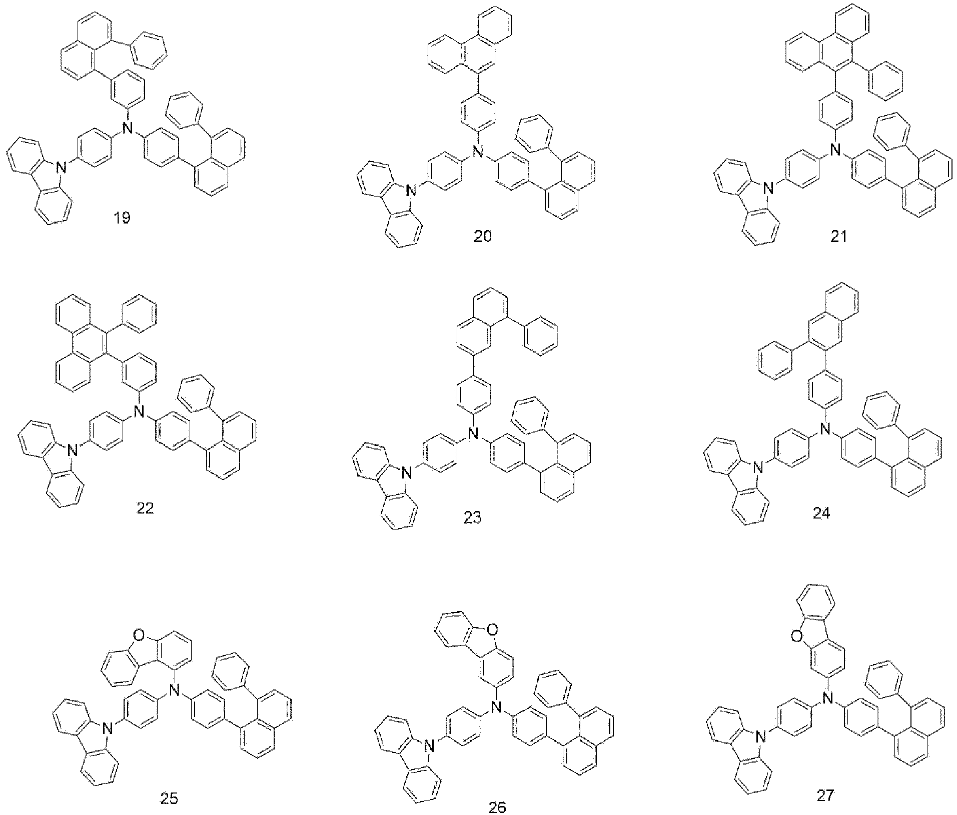 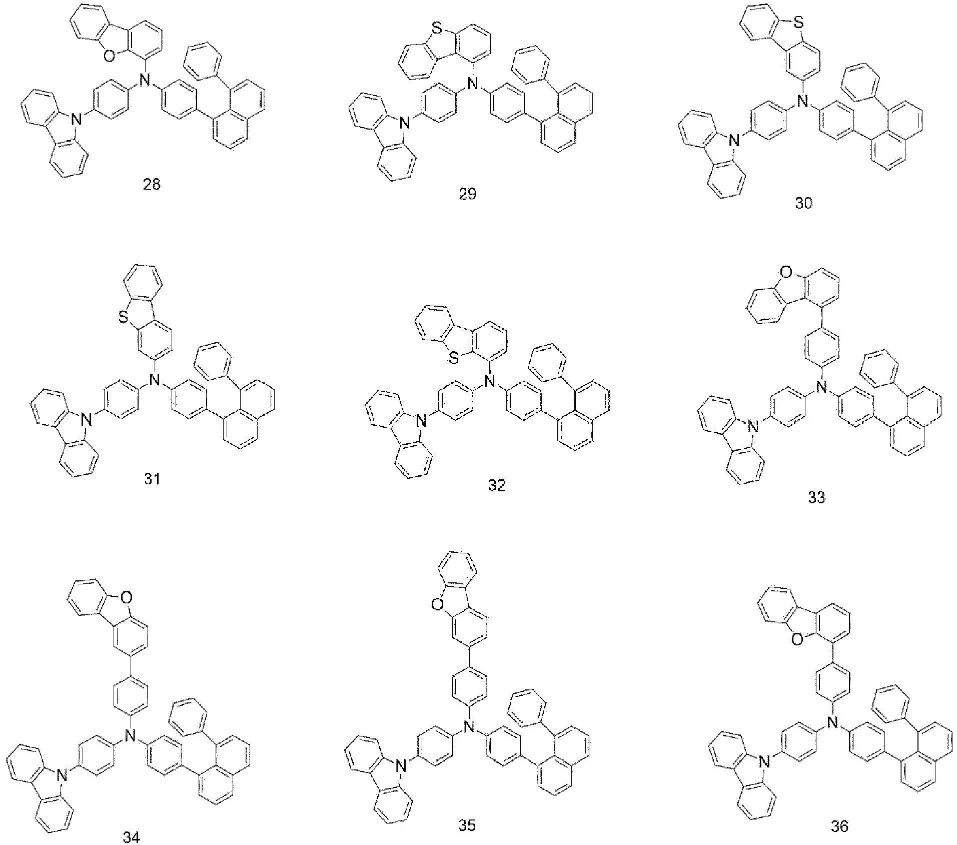 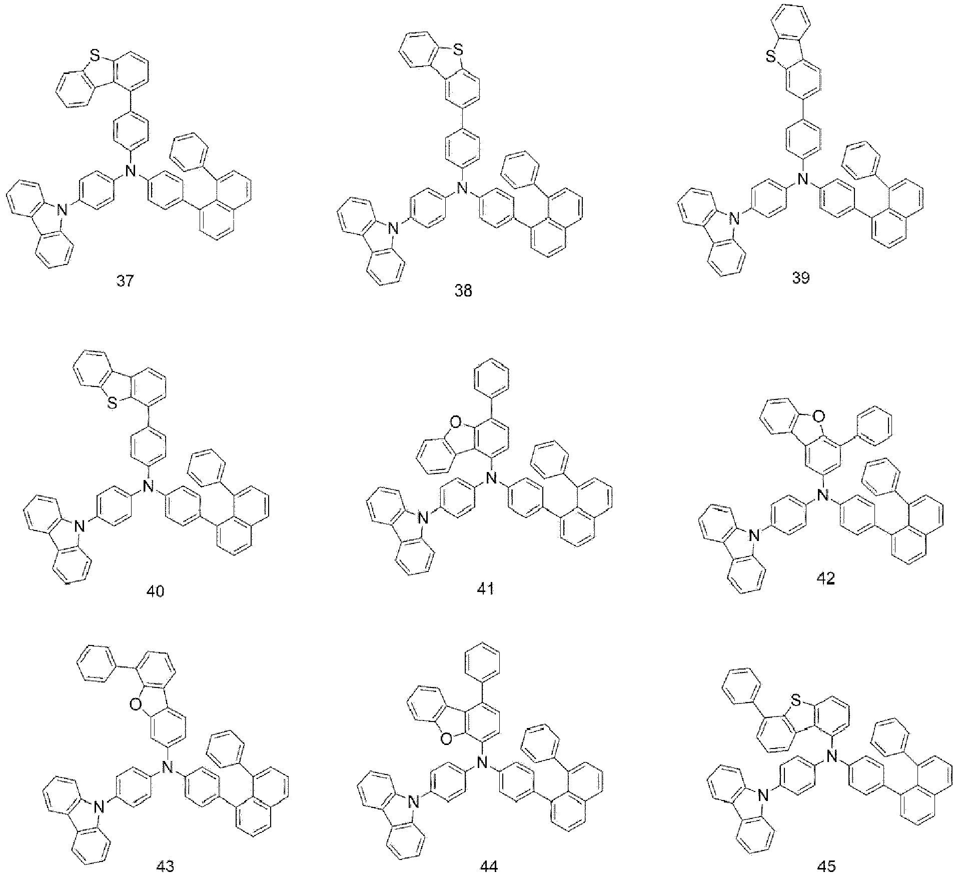 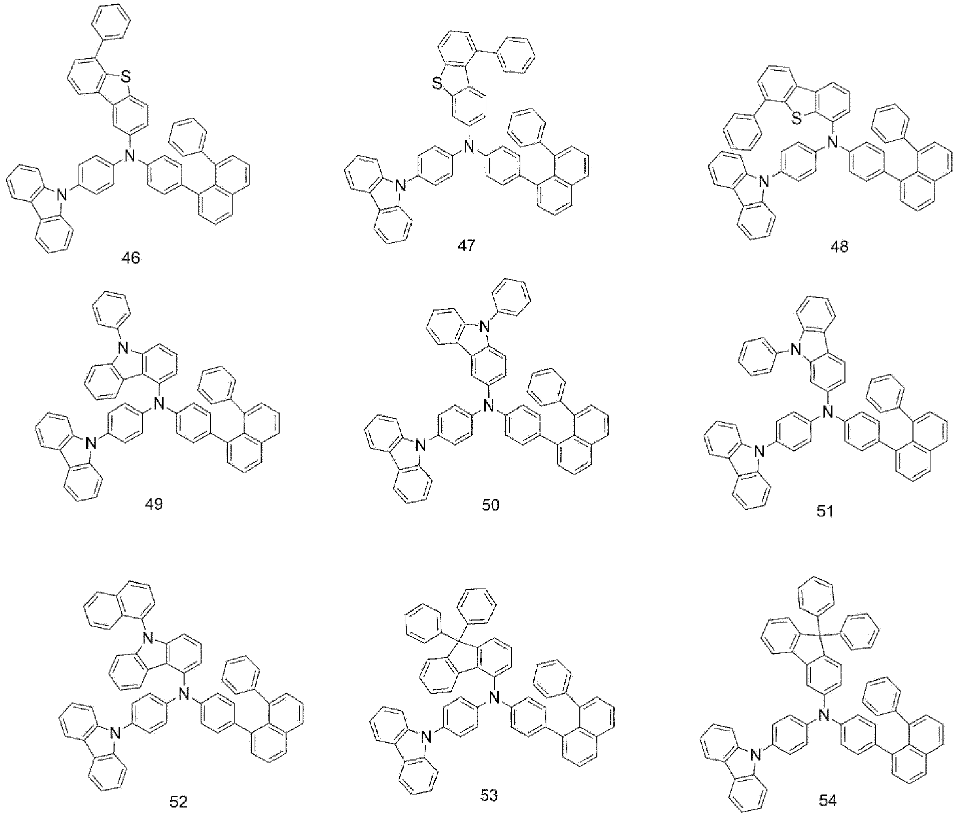 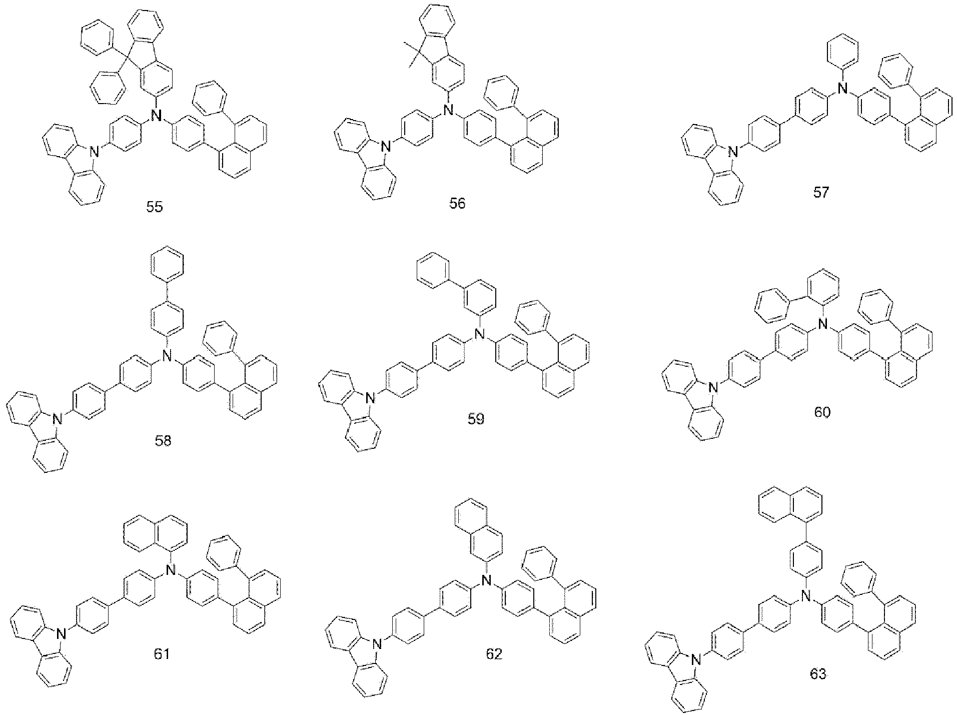 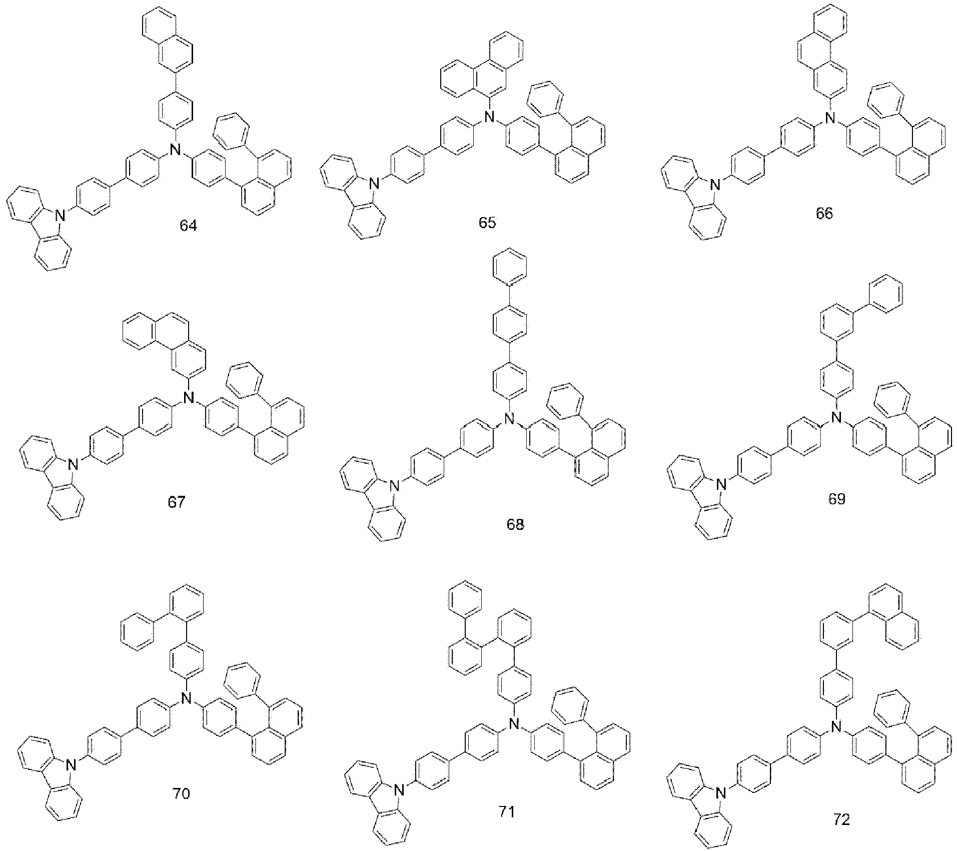 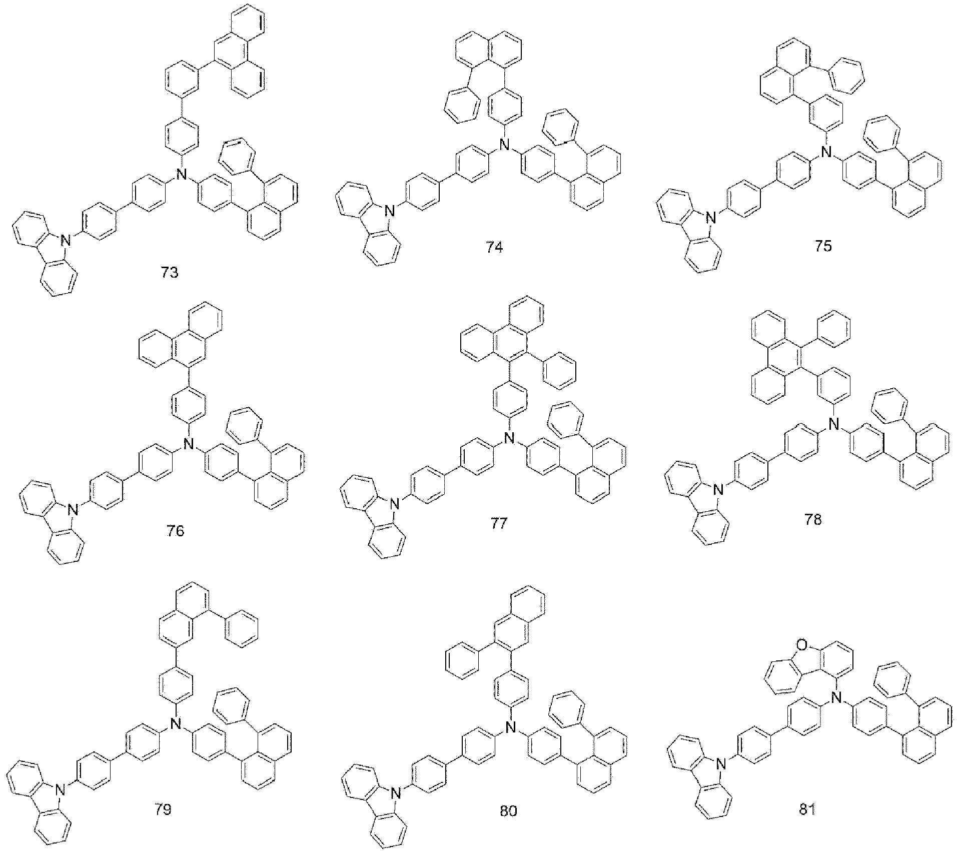 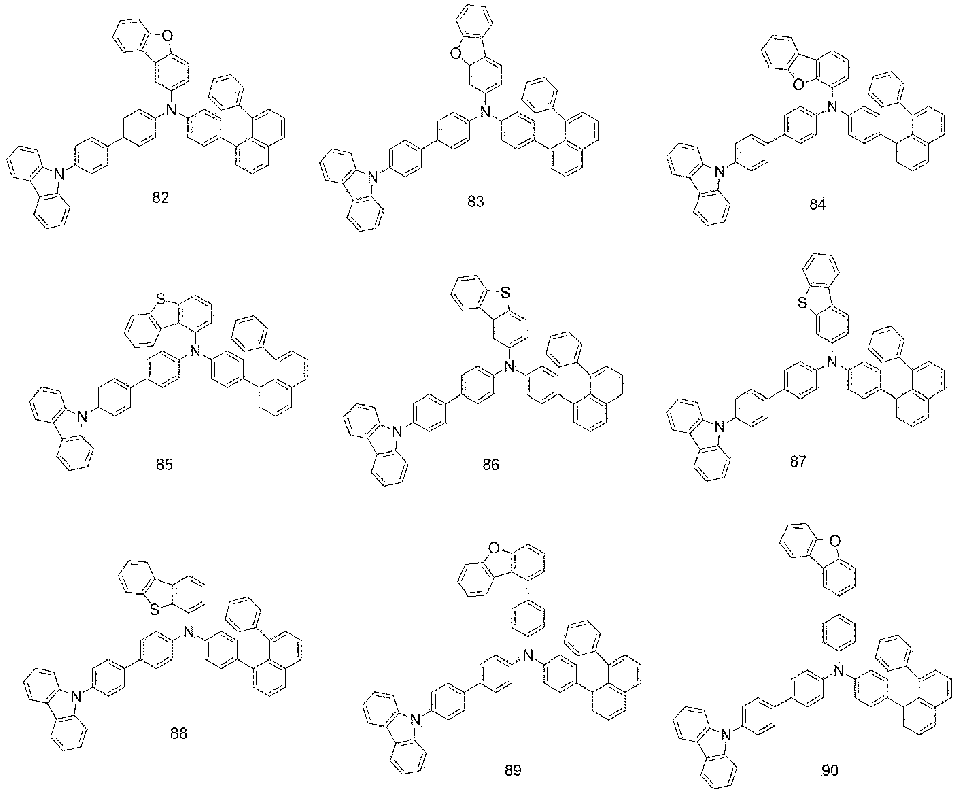 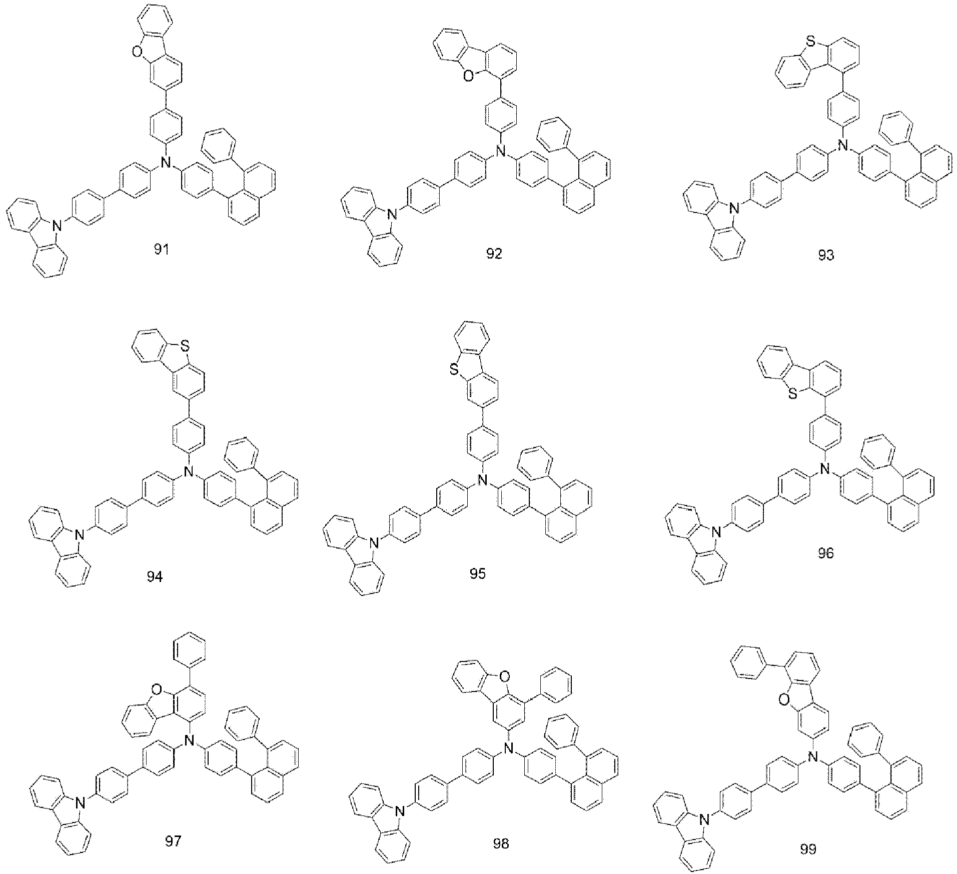 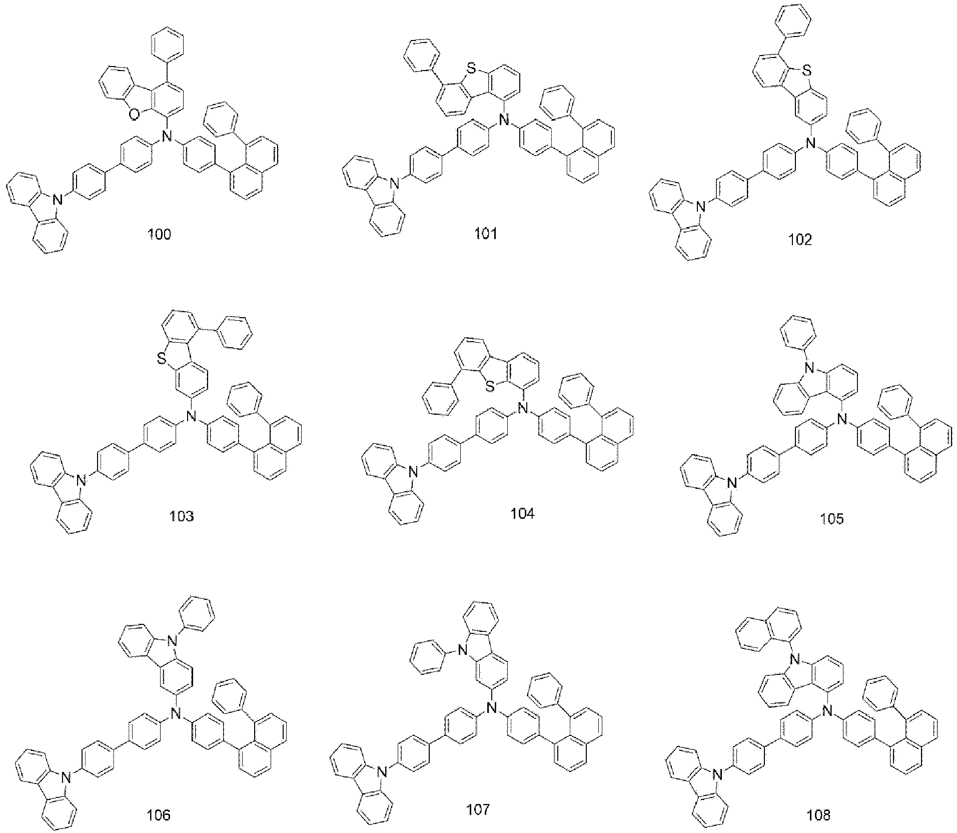 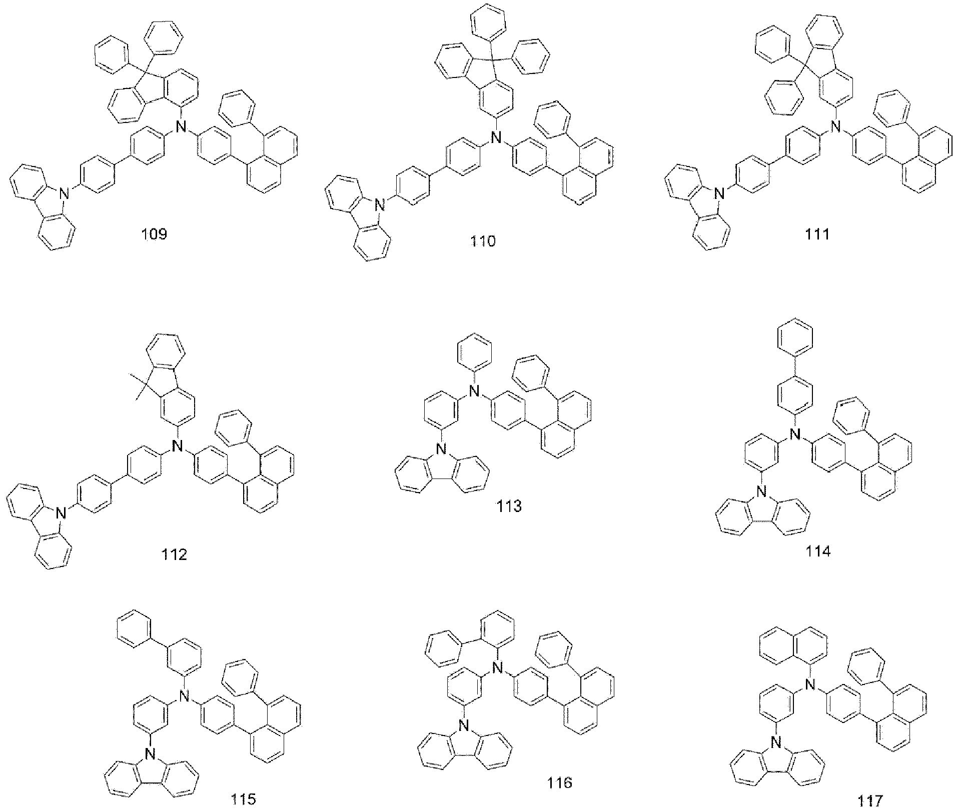 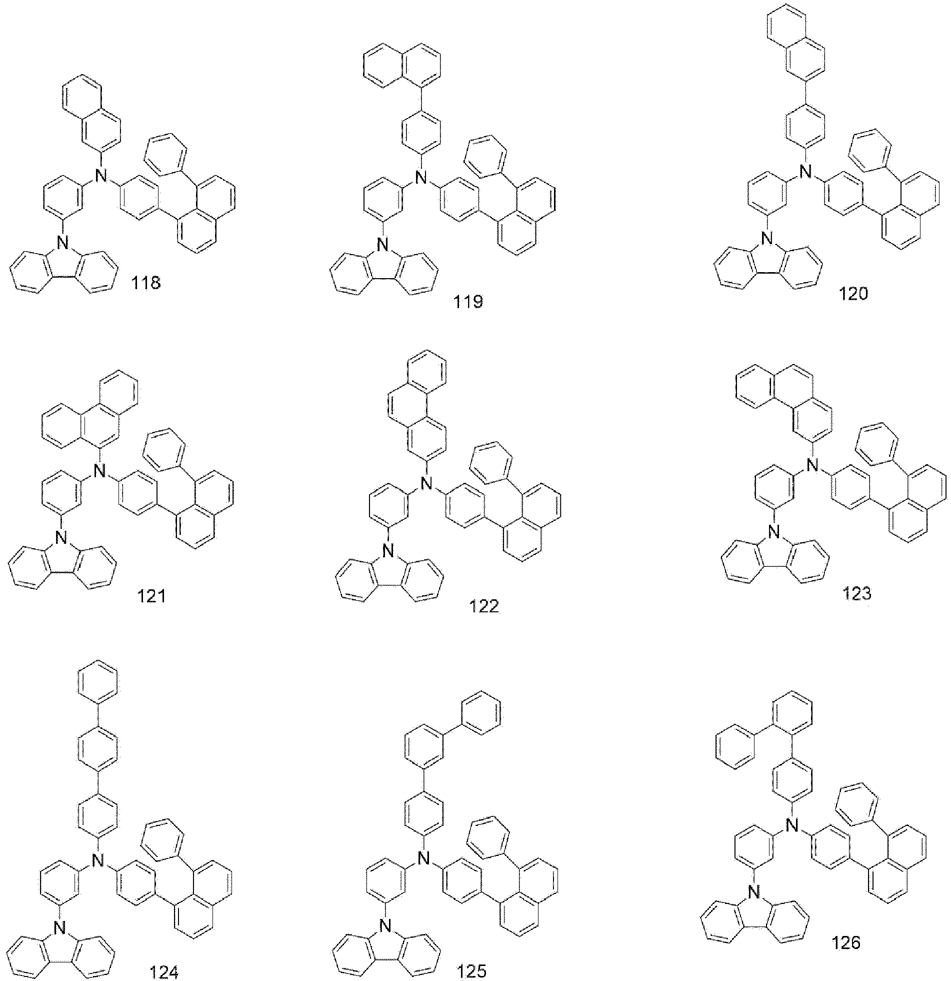 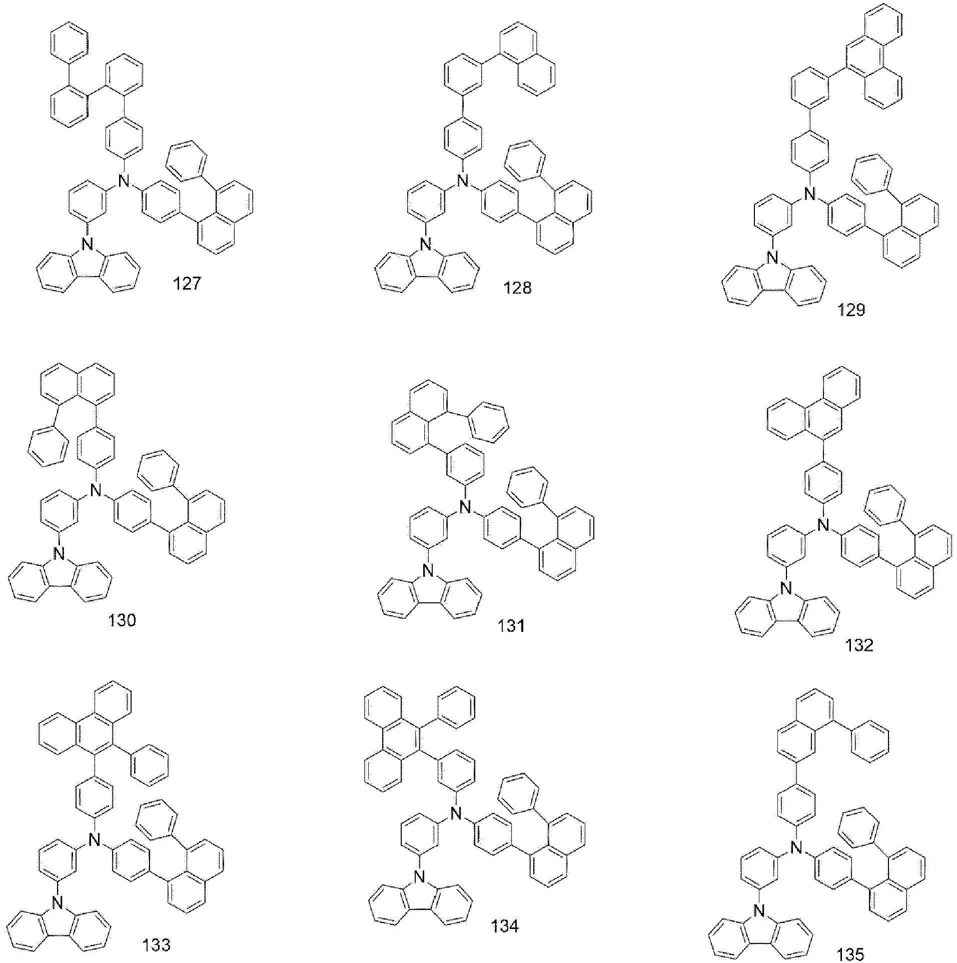 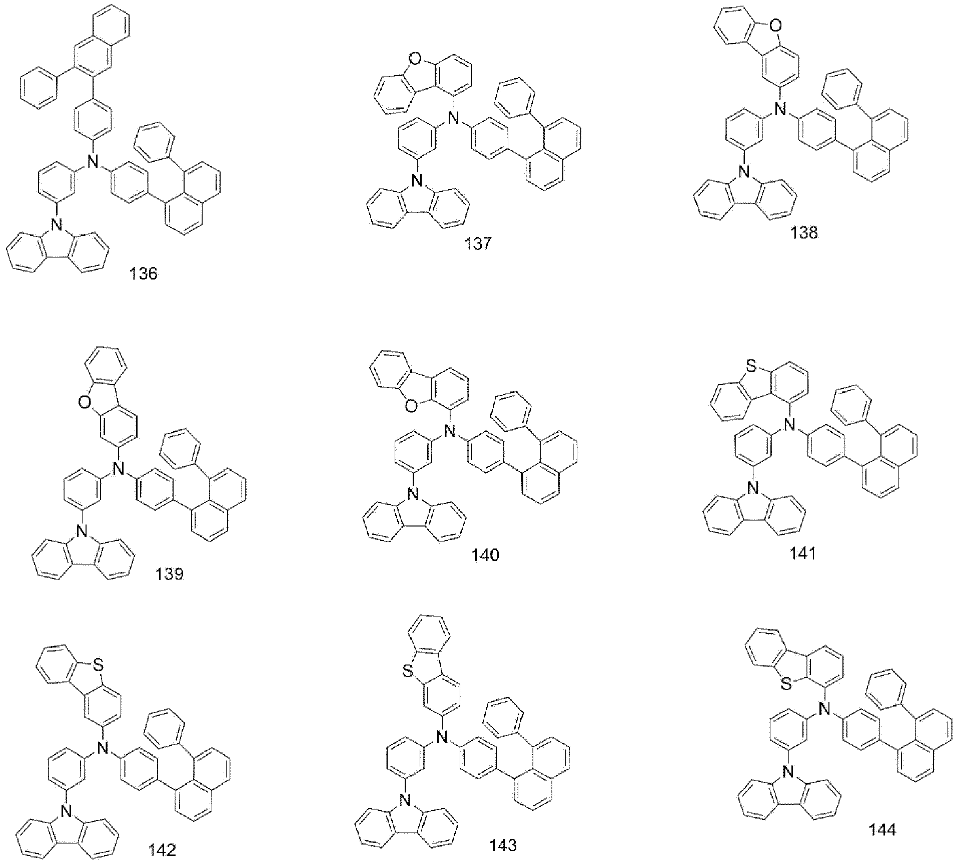 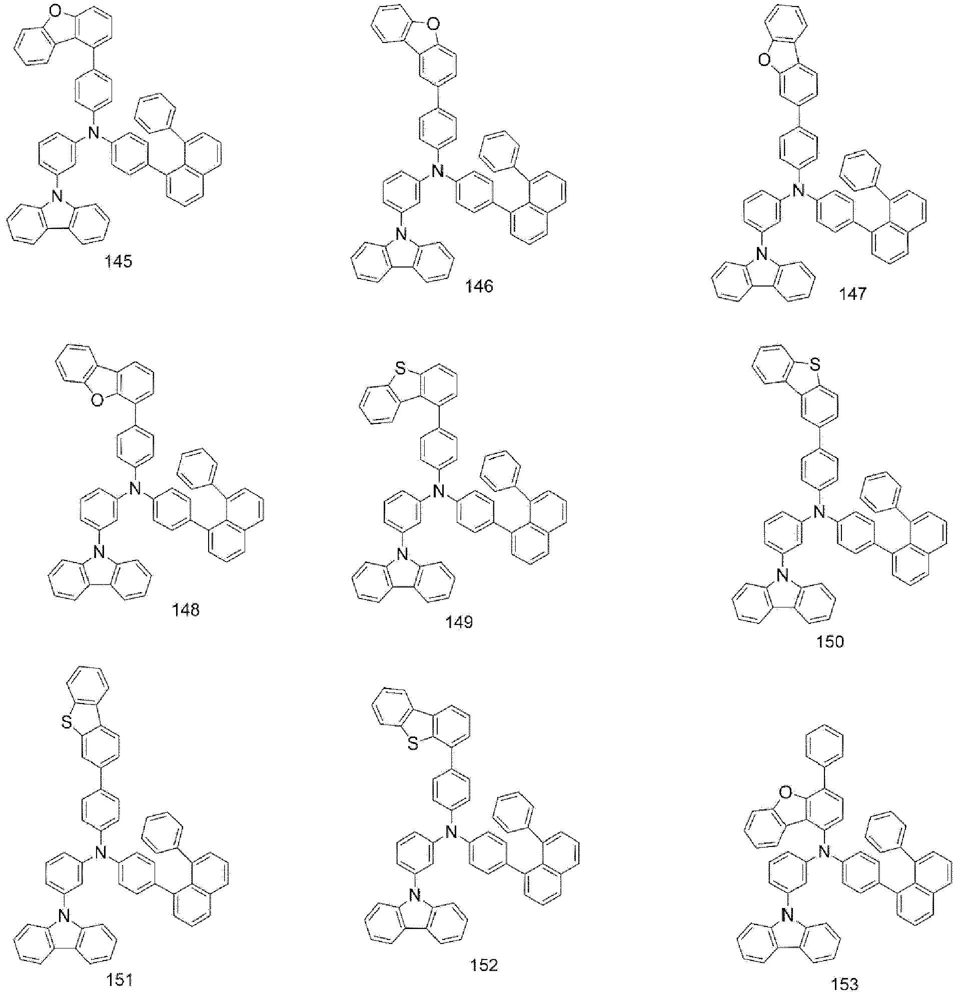 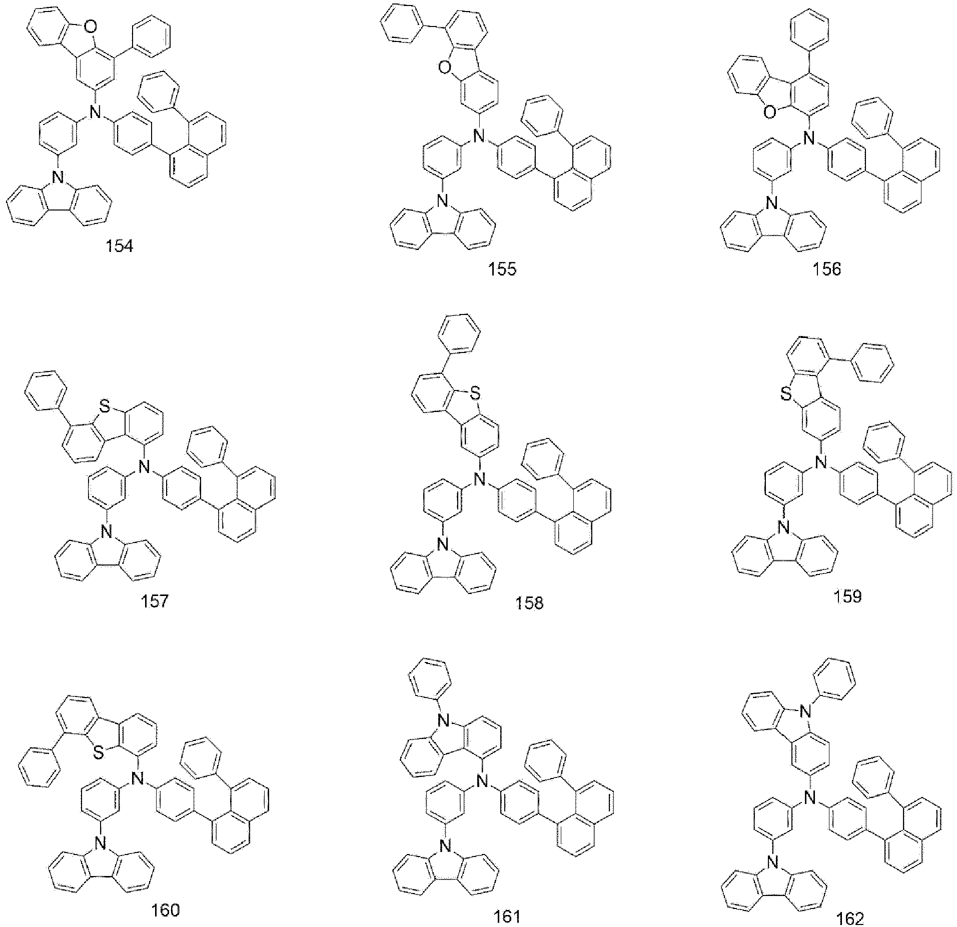 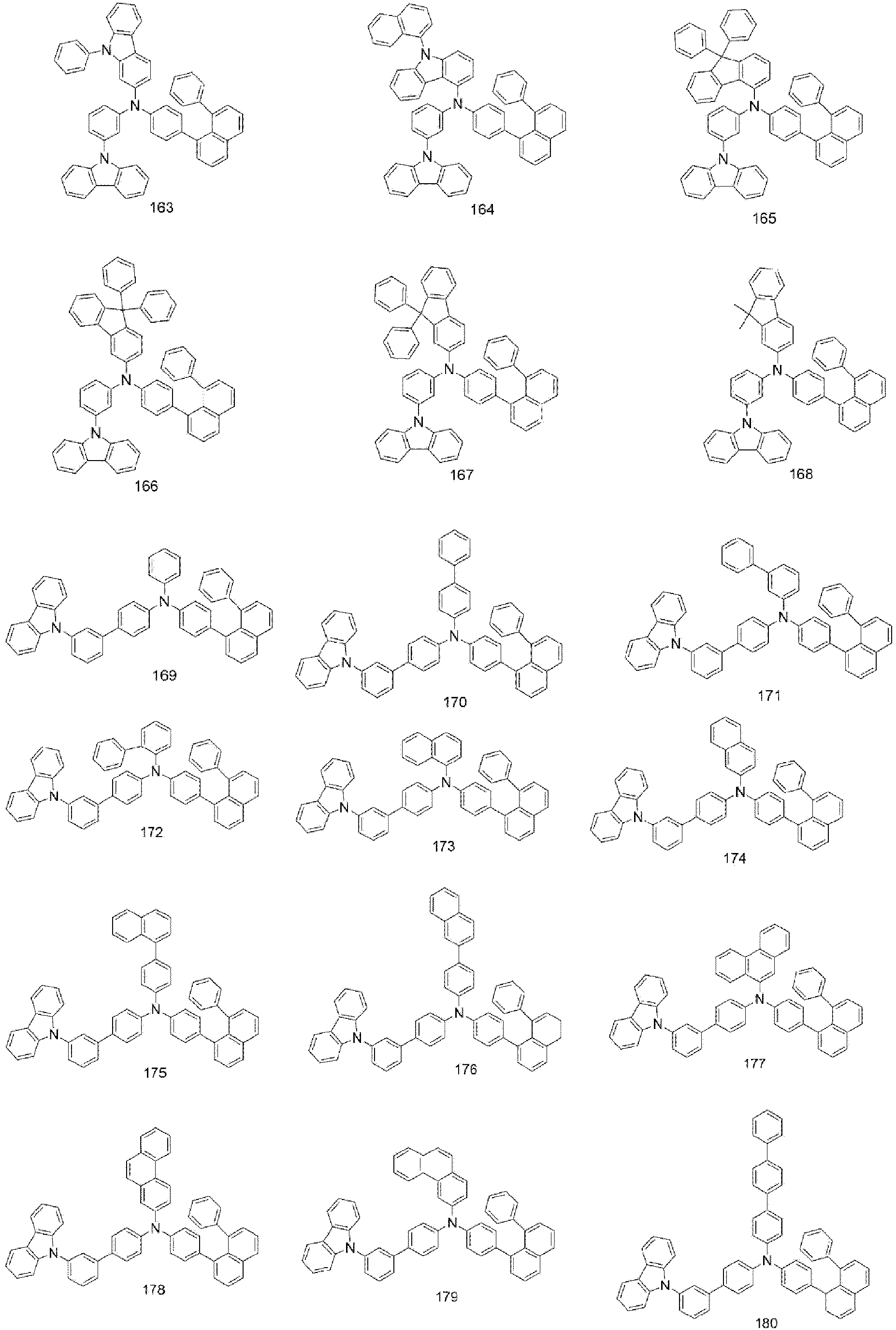 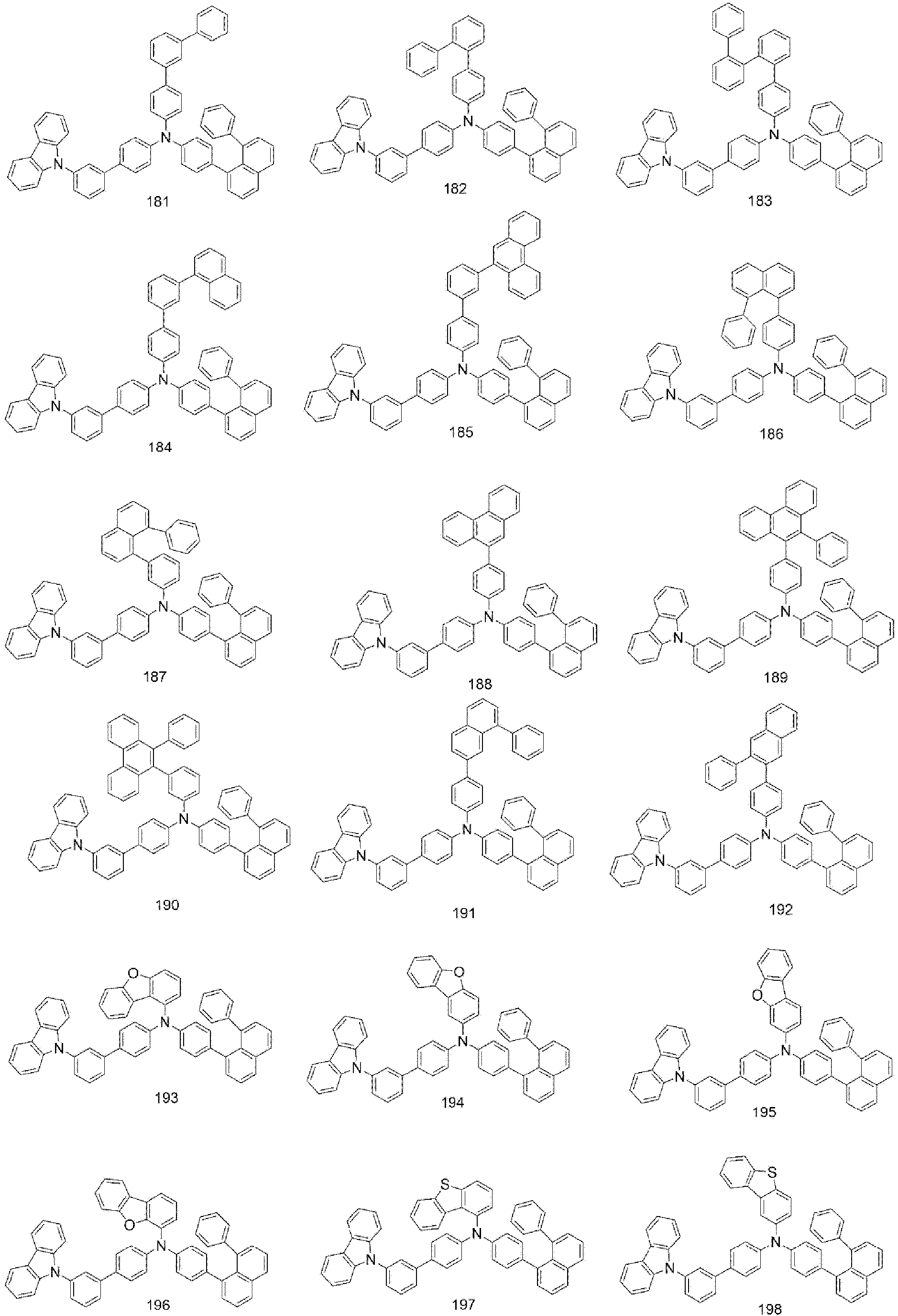 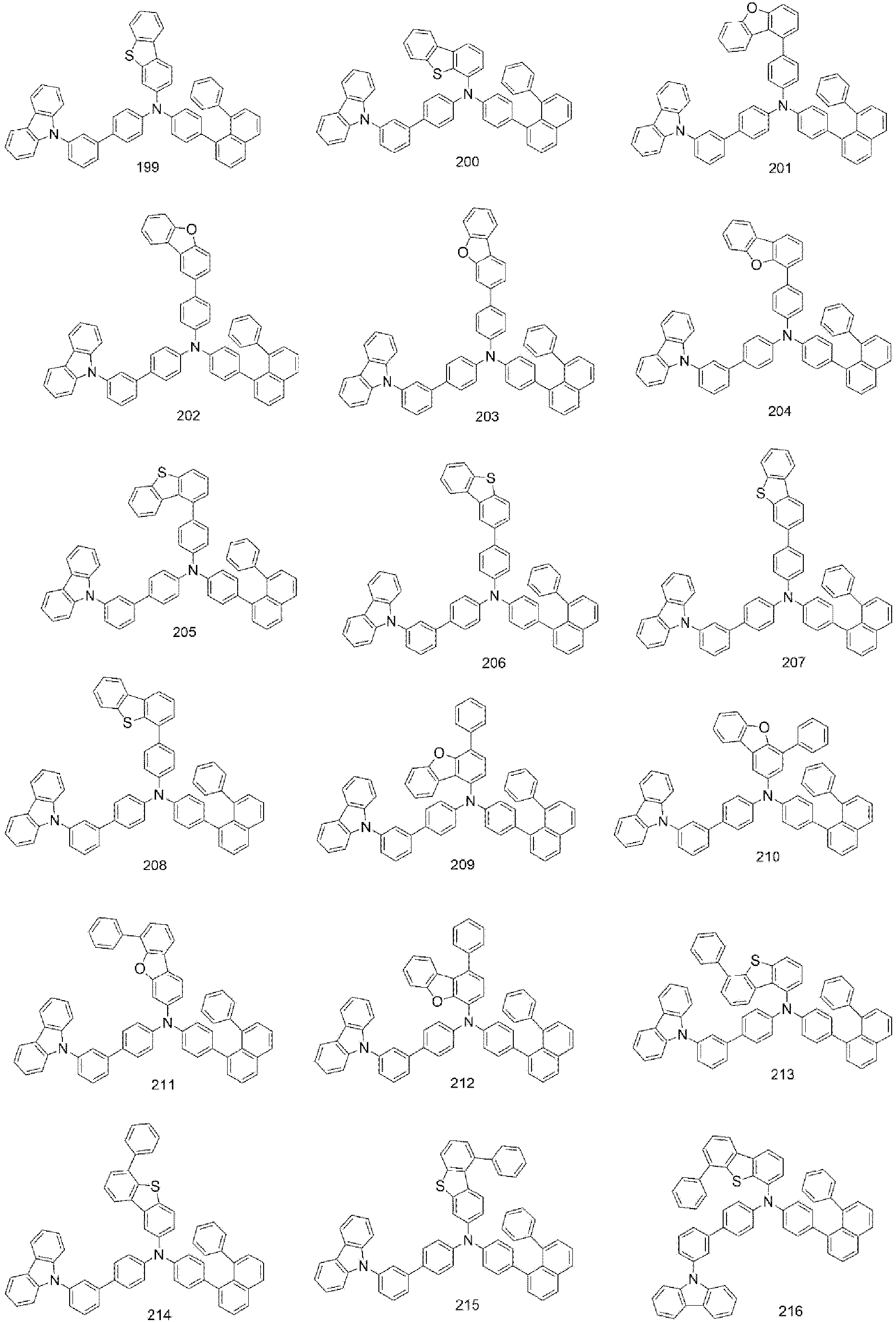 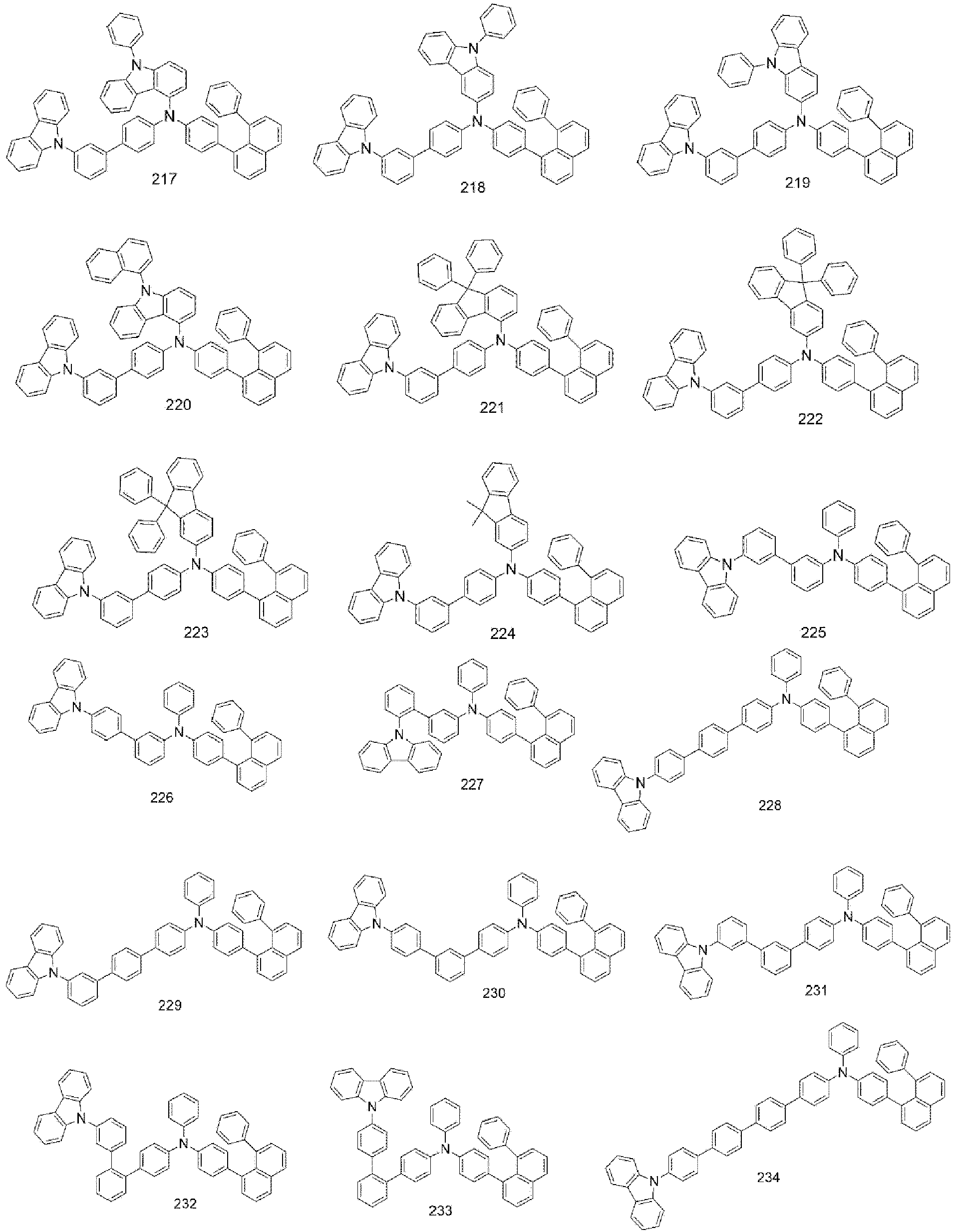 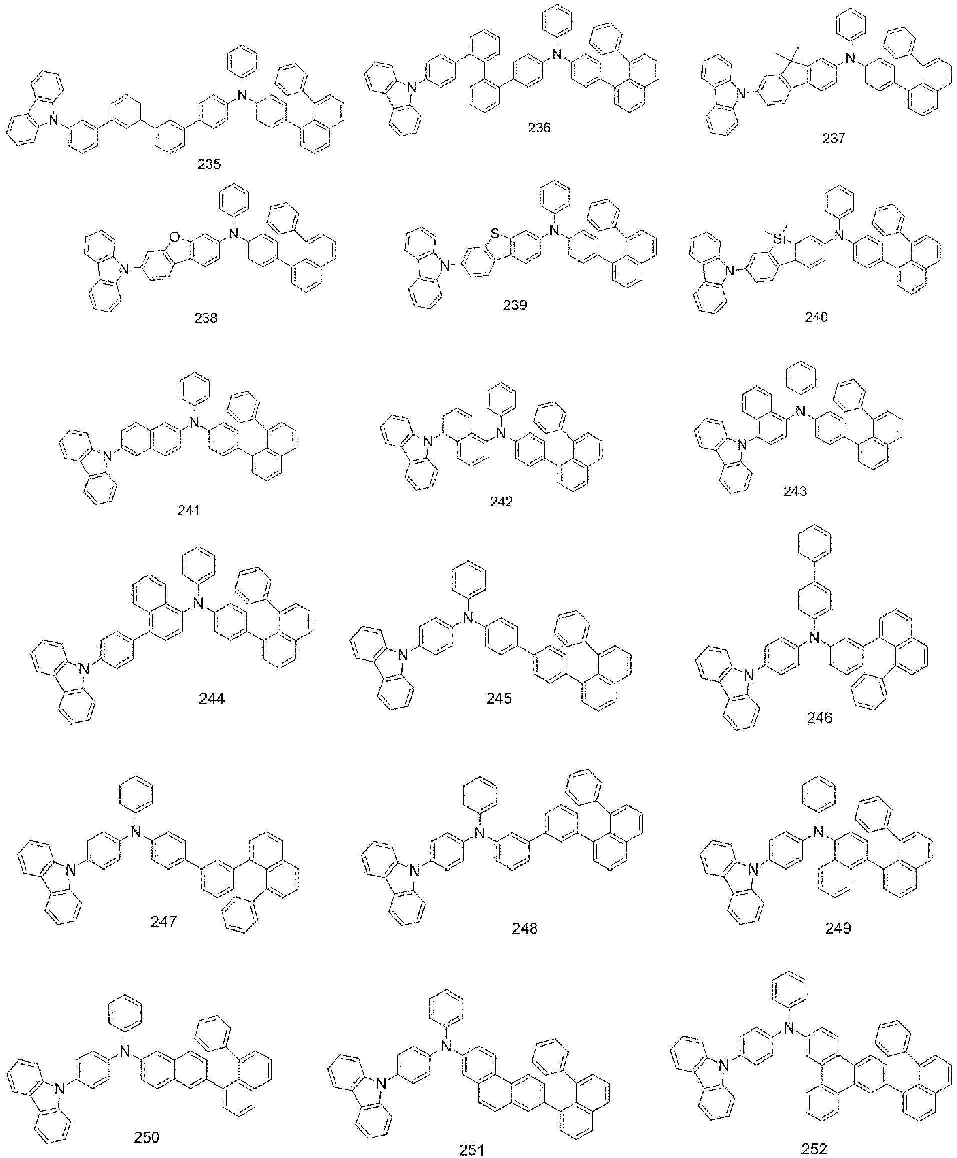 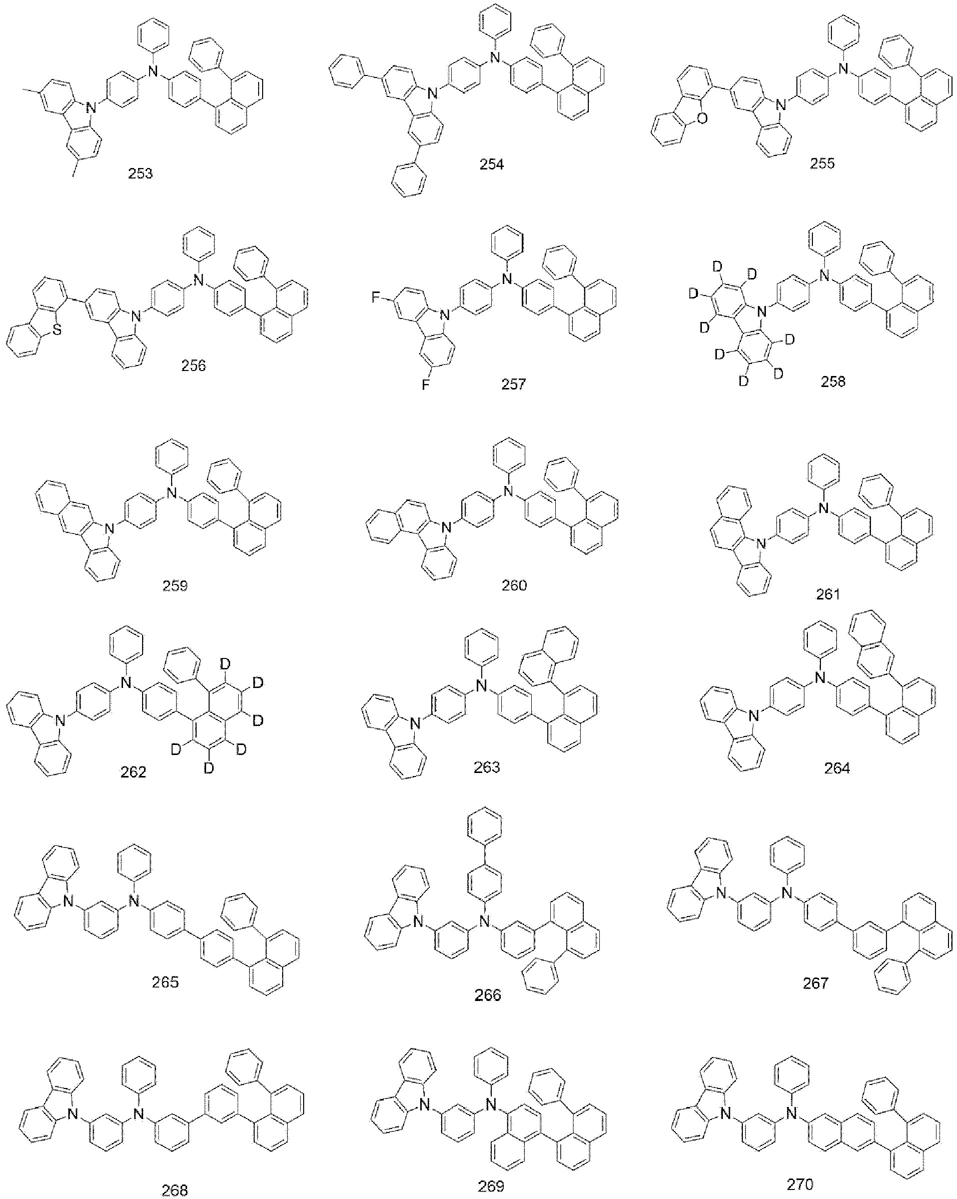 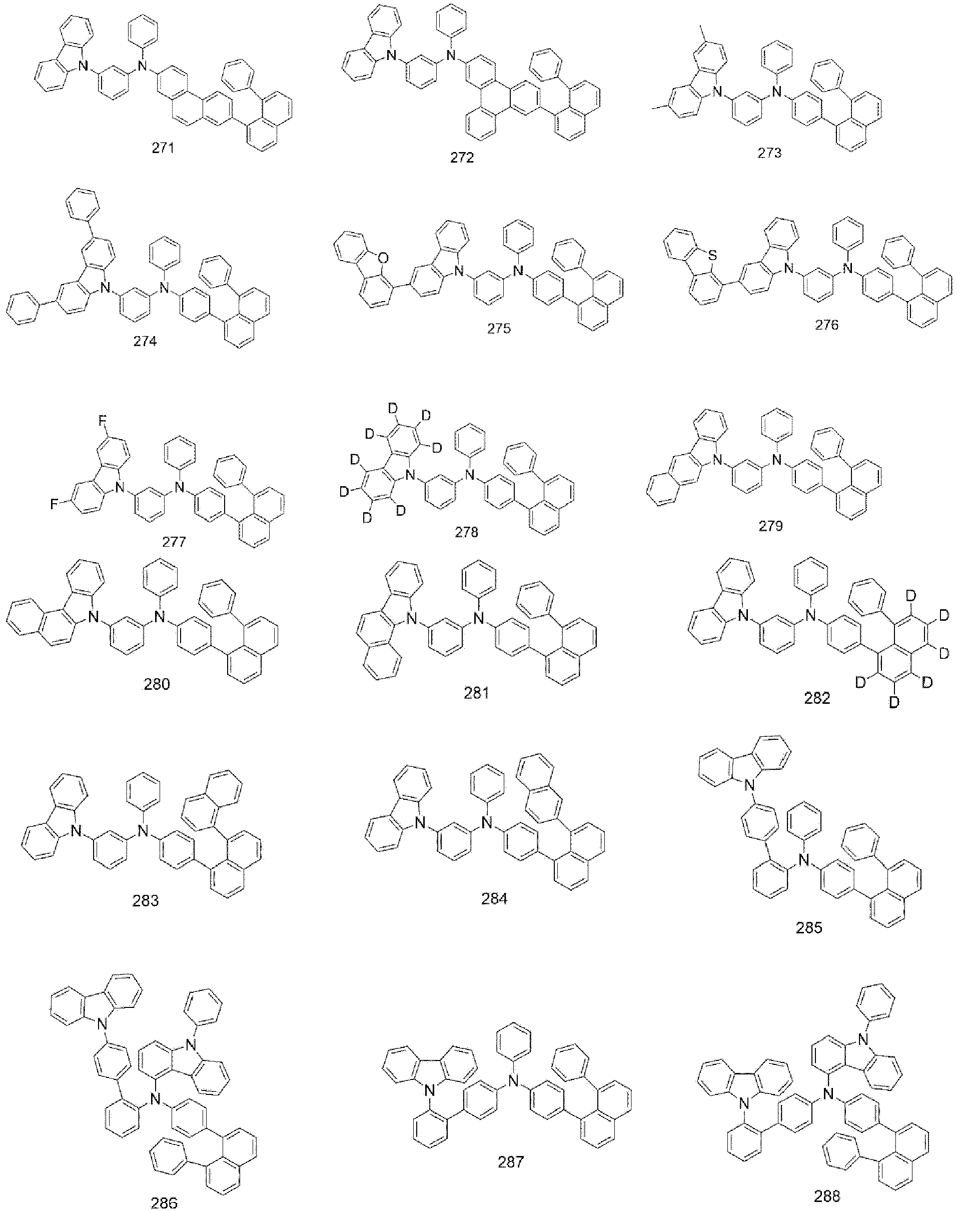 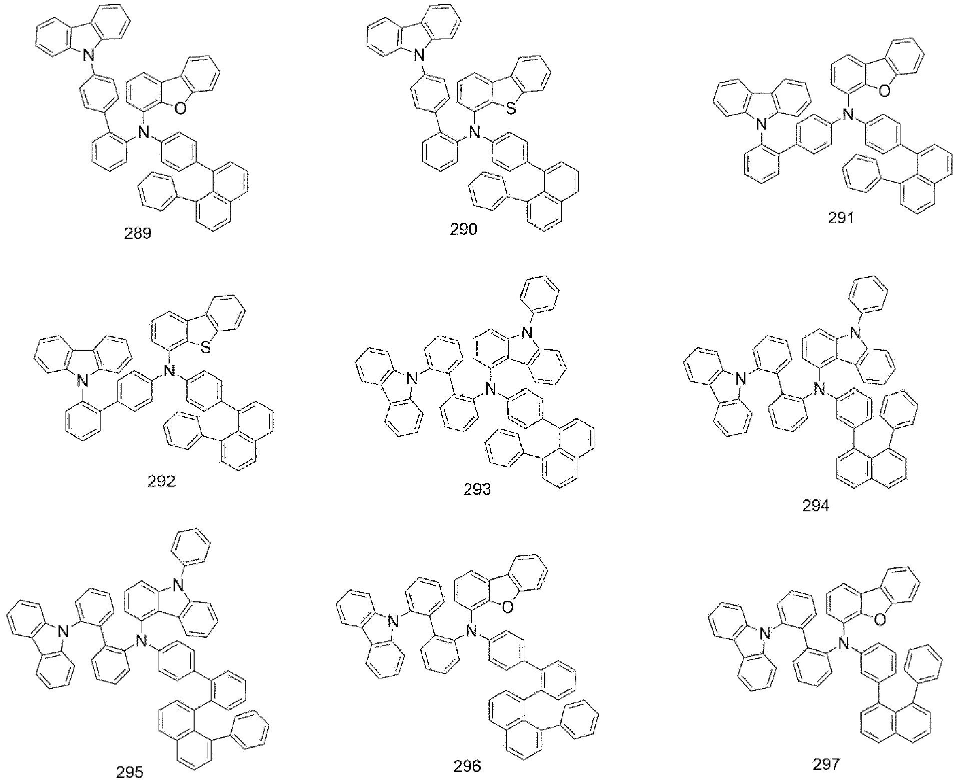 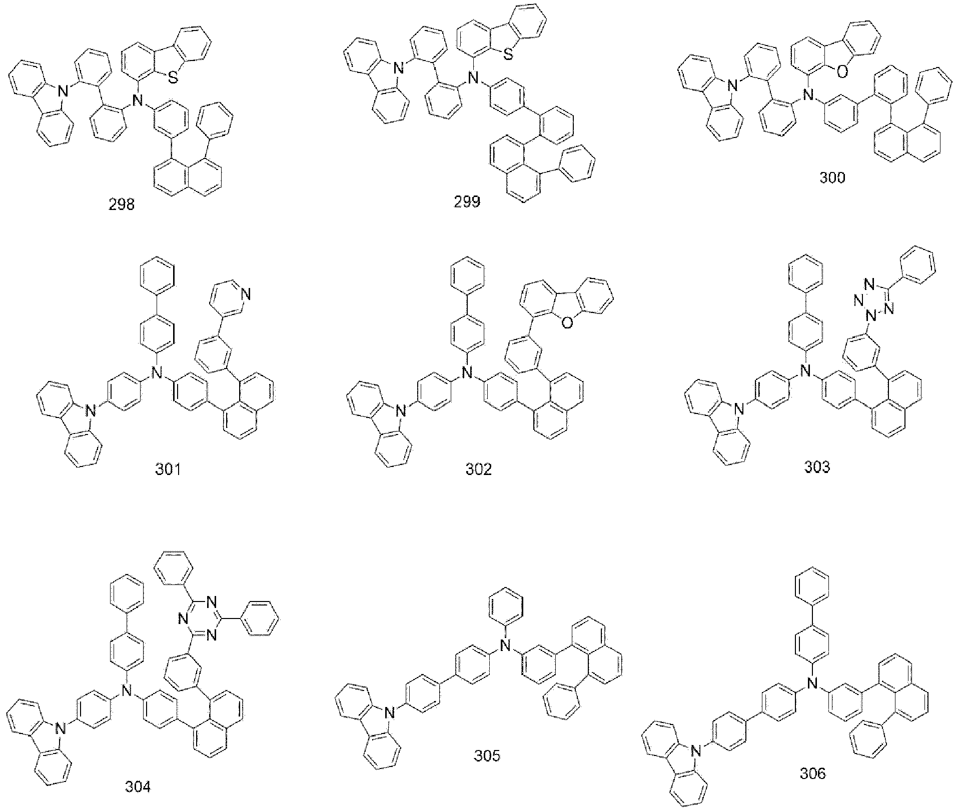 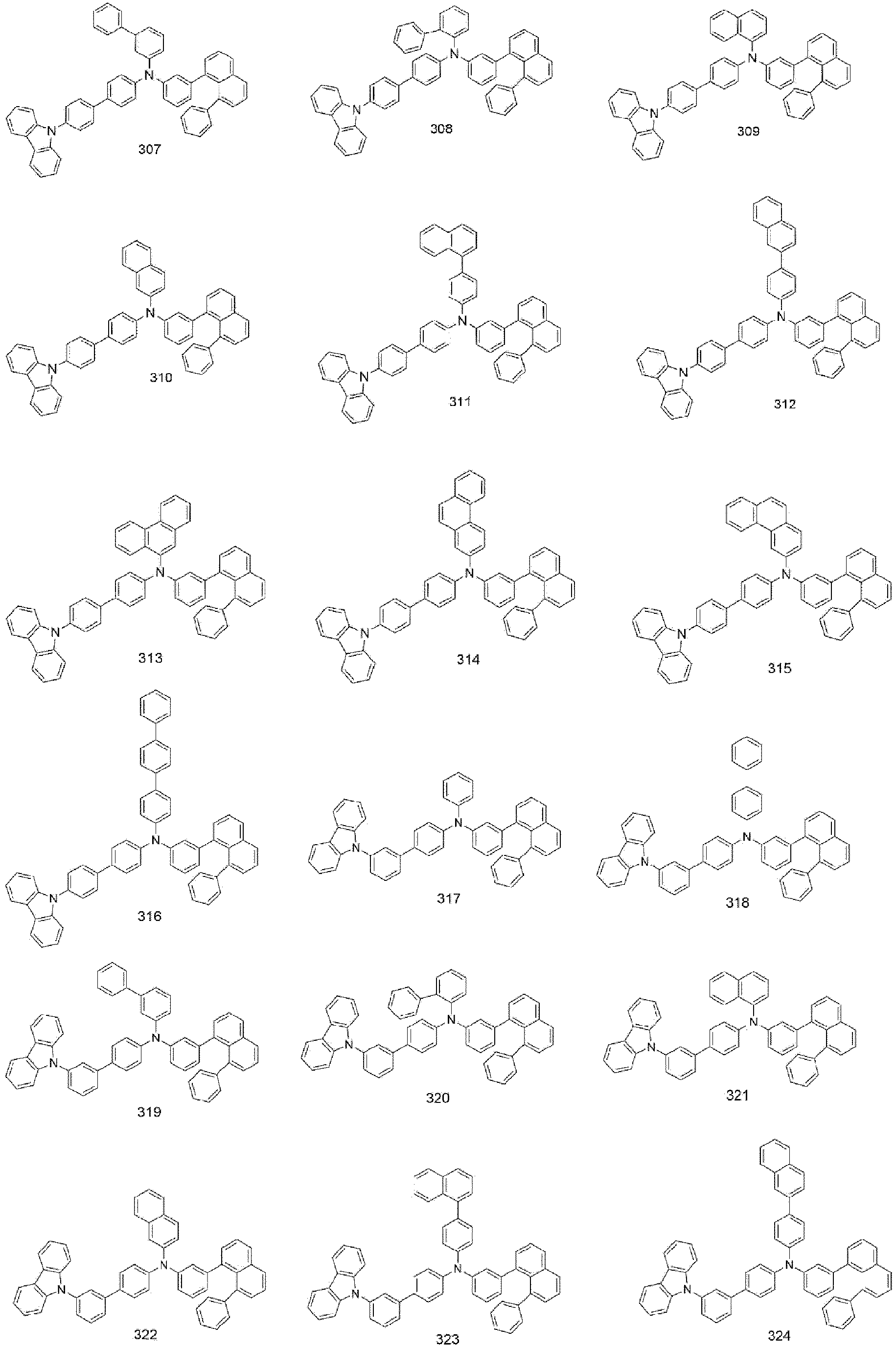 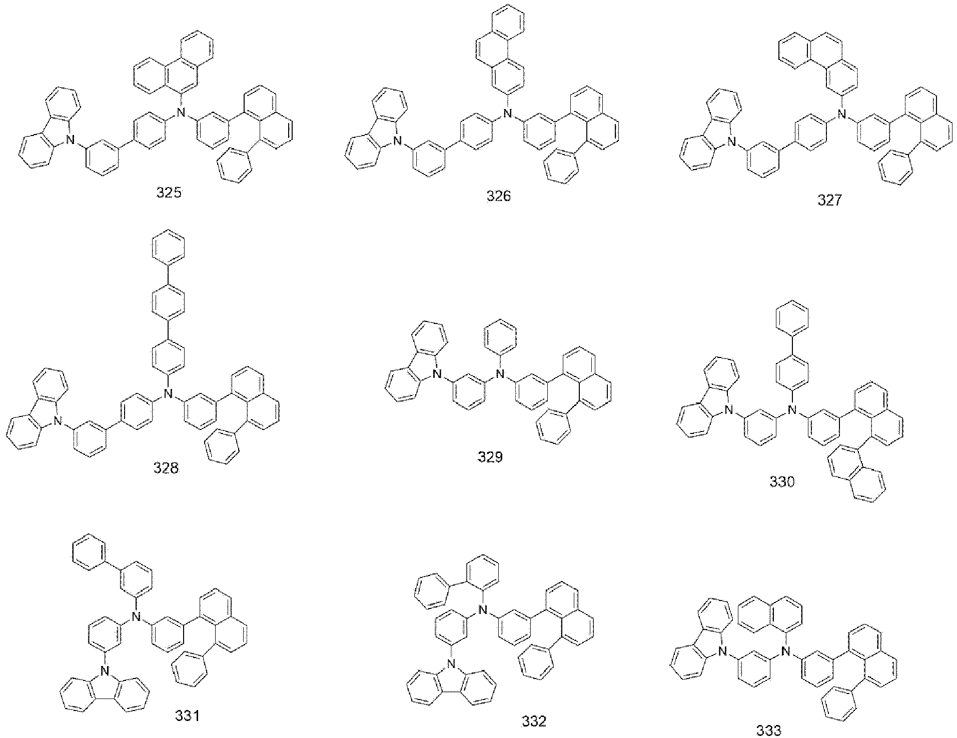 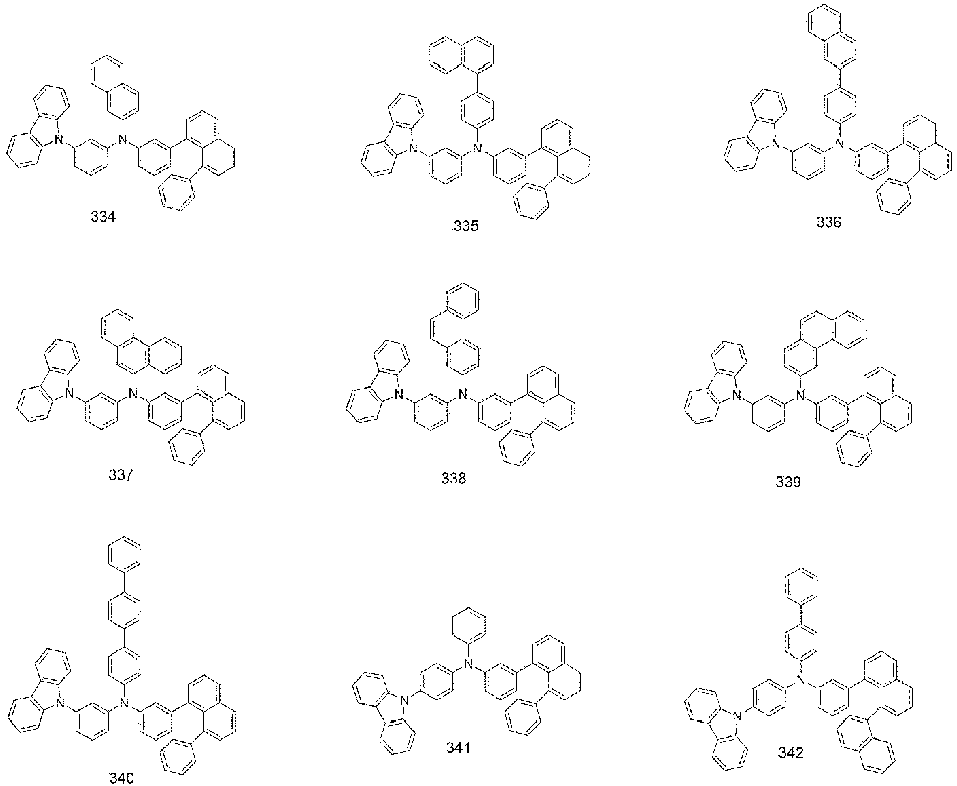 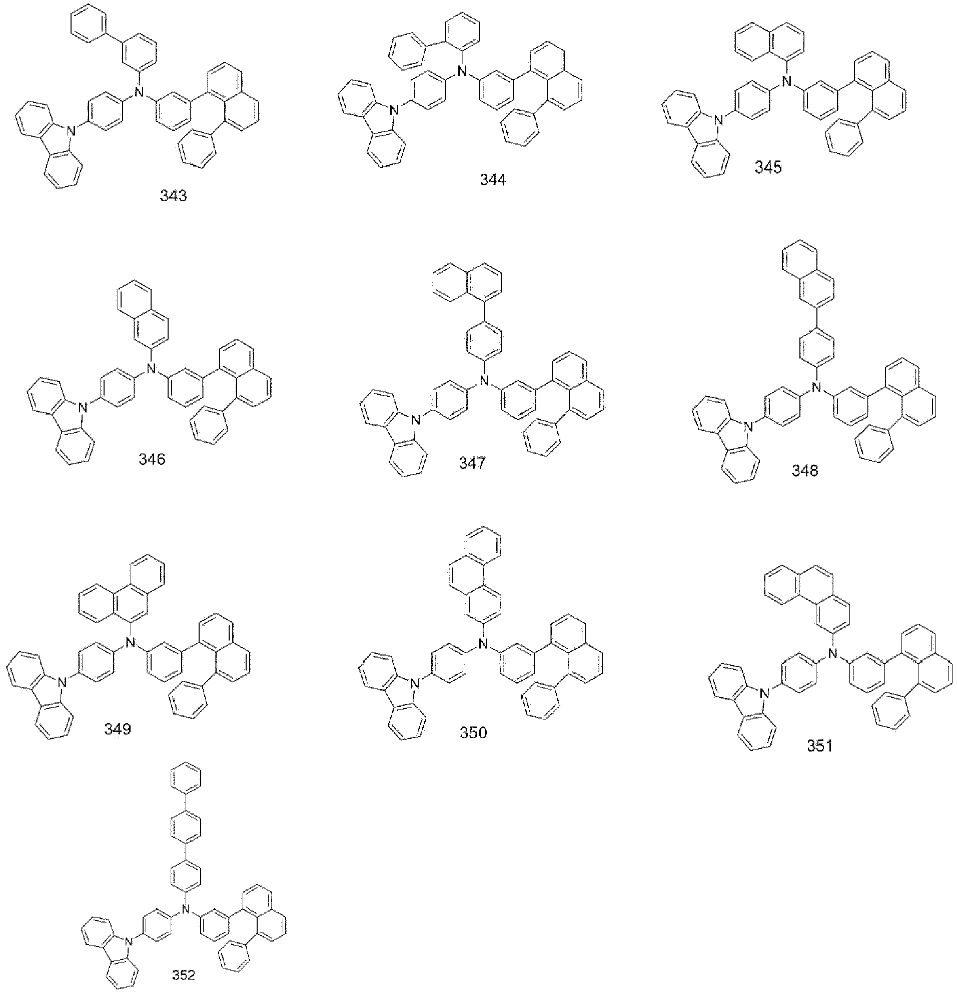 in Compound Group 1, D is a deuterium atom.

13. A light emitting element (ED), comprising:
a first electrode (EL1);
a second electrode (EL2) on the first electrode (EL1); and
at least one functional layer between the first electrode (EL1) and the second electrode (EL2), and comprising an amine compound according to any one of claims 1 to 12.

14. The light emitting element of claim 13, wherein the at least one functional layer comprises an emission layer (EML), a hole transport region (HTR) between the first electrode (EL1) and the emission layer (EML), and an electron transport region (ETR) between the emission layer (EML) and the second electrode (EL2), and
the hole transport region (HTR) comprises the amine compound represented by Formula 1.

15. The light emitting element of claim 14, wherein the hole transport region (HTR) comprises a hole injection layer (HIL) on the first electrode (EL1), and a hole transport layer (HTL) on the hole injection layer (HIL), and
the hole transport layer (HTL) comprises the amine compound represented by Formula 1.
